# EUROPEAN PATENT SPECIFICATION

(11) **EP 3 555 140 B1**
(45) Date of publication and mention of the grant of the patent: **13.07.2022**
(21) Application number: 17837952.5
(22) Date of filing: 18.12.2017
(51) Int. Cl.: C07K 16/46

(54) **ANTIBODY FRAMEWORKS FOR REDUCING AGGREGATION OF MULTISPECIFIC ANTIBODIES**
ANTIKÖRPERGERÜSTE ZUR VERMINDERUNG DER AGGREGATION VON MULTISPEZIFISCHEN ANTIKÖRPERN
STRUCTURES D'ANTICORPS POUR RÉDUIRE L'AGRÉGATION D'ANTICORPS MULTISPÉCIFIQUES

(30) Priority: 19.12.2016 GB 201621591
(43) Date of publication of application: 23.10.2019
(73) Proprietor: UCB Biopharma SRL, 1070 Brussels (BE)
(72) Inventor: BHATTA, Pallavi, Slough Berkshire SL1 3WE (GB); HUMPHREYS, David Paul, Slough Berkshire SL1 3WE (GB)
(74) Representative: UCB Intellectual Property
(86) International application number: PCT/EP2017/083301
(87) International publication number: WO 2018/114795

(56) References cited:
- WO-A1-2014/206561
- WO-A2-2015/189638
- US-B1- 8 399 625
- LEO BORRAS ET AL: "Generic approach for the generation of stable humanized single-chain Fv fragments from rabbit monoclonal antibodies", JOURNAL OF BIOLOGICAL CHEMISTRY, AMERICAN SOCIETY FOR BIOCHEMISTRY AND MOLECULAR BIOLOGY, US, vol. 285, no. 12, 9 March 2010 (2010-03-09), pages 9054-9066, XP002668298, ISSN: 0021-9258, DOI: 10.1074/JBC.M109.072876 [retrieved on 2010-01-07]
- TIMOTHY J. EGAN ET AL: "Novel multispecific heterodimeric antibody format allowing modular assembly of variable domain fragments", MABS, vol. 9, no. 1, 27 October 2016 (2016-10-27), pages 68-84, XP055467772, US ISSN: 1942-0862, DOI: 10.1080/19420862.2016.1248012
- EWERT S ET AL: "Biophysical Properties of Human Antibody Variable Domains", JOURNAL OF MOLECULAR BIO, ACADEMIC PRESS, UNITED KINGDOM, vol. 325, no. 3, 17 January 2003 (2003-01-17), pages 531-553, XP004457555, ISSN: 0022-2836, DOI: 10.1016/S0022-2836(02)01237-8
- WEIDLE U H ET AL: "The intriguing options of multispecific antibody formats for treatment of cancer", CANCER GENOMICS & PROTEOMICS, INTERNATIONAL INSTITUTE OF ANTICANCER RESEARCH, GR, vol. 10, no. 1, 1 January 2013 (2013-01-01), pages 1-18, XP002728008, ISSN: 1109-6535

## Description

The present disclosure relates to an antibody molecule comprising a framework with a reduced propensity for aggregation and a method for generating the same. The disclosure also relates to pharmaceutical compositions comprising an antibody molecule of the present disclosure and use of any one of the same in treatment. The disclosure further extends to antibody molecules obtained from the method.

### BACKGROUND

Through simultaneous binding to two antigens, bispecific antibodies can invoke novel biology and may offer enhanced clinical efficacy via improved drug targeting. Fv and scFv are discrete antigen binding domains that have been fused to Fab or IgG scaffolds to confer multispecificity in a wide variety of formats (reviewed in Spiess et al., 2015). Conversion of Fv to scFv through introduction of a polypeptide linker between the vH and vL was originally carried out to stabilise the relatively weak vH-vL interface, but dynamic domain exchange ('breathing') between proximal scFv monomers can result in variable levels of monomer, dimer and higher-order multimers (Weatherill et al., 2012). High levels of multimer formation during protein expression will thus ultimately result in low overall therapeutic protein yields

Formation of a disulphide (ds) bond in scFv between the vH and vL prevents variable domain 'breathing' within purified scFv, and thus fixes monomer:multimer ratios to enable purification of monomeric products (Weatherill, et al., 2012). The introduction of a vH-vL disulphide bond irreversibly locks all multimeric forms during expression/purification, and therefore facilitates the separation of desirable (monomeric) from undesirable (multimeric) species by purification. Thus the presence of the disulfide bond does not optimise the amount of monomer expressed. WO2014/206561 discloses chimeric human antibody light chain frameworks with the concern of reducing aggregation propensity, in the context of monospecific scFvs.

However, there is a continuing need in the art to provide new means for improving the monomer yield of multispecific antibodies.

### SUMMARY OF THE DISCLOSURE

The present disclosure provides a multispecific antibody molecule comprising at least two binding domains each with a variable heavy region VH and a variable light region VL, wherein each binding domain is specific for an antigen and comprises 6 CDRs, characterised in that at least one binding domain comprises:
a. a variable heavy region, VH_{A}, having a framework comprising SEQ ID NO: 1 for FR1, SEQ ID NO: 3 for FR2, SEQ ID NO: 4 for FR3 and SEQ ID NO: 5 for FR4, with the proviso that CDRH3 in VH_{A} is other than RYYSAMPFAY (SEQ ID NO: 129) and a variable light region, VL_{A}, having a framework comprising SEQ ID NO: 6 for FR1, SEQ ID NO: 7 for FR2, SEQ ID NO: 8 for FR3, and SEQ ID NO: 10 for FR4; or
b. a variable heavy region, VH_{A}, having a framework comprising SEQ ID NO: 16 for FR1, SEQ ID NO: 18 for FR2, SEQ ID NO: 19 for FR3 and SEQ ID NO: 20 for FR4, with the proviso that CDRH3 in VH_{A} is other than RYYSAMPFAY (SEQ ID NO: 129) and a variable light region, VL_{A}, having a framework comprising SEQ ID NO: 11 for FR1, SEQ ID NO: 12 for FR2, SEQ ID NO: 13 for FR3, and SEQ ID NO: 15 for FR4;
and wherein the at least one variable region VH_{A} and the at least one variable region VL_{A} are present in a dsscFv, and wherein the multispecific antibody molecule is a Fab-dsscFv or Fab-(dsscFv)₂.

The disclosure will be summarised in the following paragraphs:
1. A multispecific antibody molecule comprising at least two binding domains each with a variable heavy region VH and a variable light region VL, wherein each binding domain is specific for an antigen and comprises 6 CDRs, characterised in that at least one binding domain comprises:
   a. a variable heavy region, VH_{A}, having a framework comprising SEQ ID NO: 1 for FR1, SEQ ID NO: 3 for FR2, SEQ ID NO: 4 for FR3 and SEQ ID NO: 5 for FR4, with the proviso that CDRH3 in VH_{A} is other than RYYSAMPFAY (SEQ ID NO: 129) and a variable light region, VL_{A}, having a framework comprising SEQ ID NO: 6 for FR1, SEQ ID NO: 7 for FR2, SEQ ID NO: 8 for FR3, and SEQ ID NO: 10 for FR4; or
   b. a variable heavy region, VH_{A}, having a framework comprising SEQ ID NO: 16 for FR1, SEQ ID NO: 18 for FR2, SEQ ID NO: 19 for FR3 and SEQ ID NO: 20 for FR4, with the proviso that CDRH3 in VH_{A} is other than RYYSAMPFAY (SEQ ID NO: 129) and a variable light region, VL_{A}, having a framework comprising SEQ ID NO: 11 for FR1, SEQ ID NO: 12 for FR2, SEQ ID NO: 13 for FR3, and SEQ ID NO: 15 for FR4;
   and wherein the at least one variable region VH_{A} and the at least one variable region VL_{A} are present in a dsscFv, and wherein the multispecific antibody molecule is a Fab-dsscFv or Fab-(dsscFv)₂.
2. A multispecific antibody molecule according to paragraph 1, wherein VL_{A} comprises SEQ ID NO:99 and VH_{A} comprises SEQ ID NO:101.
3. A multispecific antibody molecule according to paragraph 2, wherein one binding domain comprises SEQ ID NO:106.
4. A multispecific antibody molecule according to paragraph 1, wherein VL_{A} comprises SEQ ID NO:103 and VH_{A} comprises SEQ ID NO:105.
5. A multispecific antibody molecule according to paragraph 4, wherein one binding domain comprises SEQ ID NO:111.
6. A multispecific antibody molecule according to any of paragraph 1 to 5, wherein the VH_{A} comprises 3 heavy chain CDRs having the sequence given in SEQ ID NO: 143 for CDRH1, SEQ ID NO: 144 for CDRH2 and SEQ ID NO: 145 for CDRH3.
7. A multispecific antibody molecule according to any of paragraph 1 to 6, wherein the VL_{A} comprises 3 light chain CDRs having the sequence given in SEQ ID NO:146 for CDRL1, SEQ ID NO:147 for CDRL2 and SEQ ID NO:148 for CDRL3.
8. A multispecific antibody molecule according to any preceding paragraph, wherein the CDRs have a non-human origin.
9. A multispecific antibody molecule according to any preceding paragraph, wherein the antibody molecule is bispecific or trispecific, such as bispecific.
10. An acceptor framework for minimising aggregation in a multispecific antibody comprising SEQ ID NO: 22 and 24, wherein XXX in the each of the CDR positions independently represent 1 to 35 amino acids, and wherein SEQ ID NO: 22 and 24 are present in a dsscFv, and wherein the multispecific antibody is a Fab-dsscFv or Fab-(dsscFv)₂.
11. A method of improving the levels of monomer expression of an antibody molecule having at least one binding domain with a variable heavy region and variable light region where each variable region has a framework and 3 CDRs, said method comprising the step of changing a framework of a variable region to a framework selected from the group comprising or consisting of:
   i) a framework (such a VH region framework) comprising SEQ ID NO: 1 for FR1, SEQ ID NO: 3 for FR2, SEQ ID NO: 4 for FR3 and SEQ ID NO: 5 for FR4,
   ii) a framework (such a VH region framework) comprising SEQ ID NO: 16 for FR1, SEQ ID NO: 18 for FR2, SEQ ID NO: 19 for FR3 and SEQ ID NO: 20 for FR4,
   iii) a framework (such a VL region framework) comprising SEQ ID NO: 6 for FR1, SEQ ID NO: 7 for FR2, SEQ ID NO: 8 for FR3, and SEQ ID NO: 10 for FR4.
   iv) a framework (such a VL region framework) comprising VL1, SEQ ID NO: 11 for FR1, SEQ ID NO: 12 for FR2, SEQ ID NO: 13 for FR3, and SEQ ID NO: 15 for FR4,
   v) combinations thereof and,
   wherein the framework i), ii), iii), iv), or combinations thereof are present in a dsscFv, and wherein the antibody molecule is a Fab-dsscFv or Fab-(dsscFv)₂.

### SUMMARY OF THE FIGURES

- **Figure** 1: Is a table setting out the format included in "set 1"

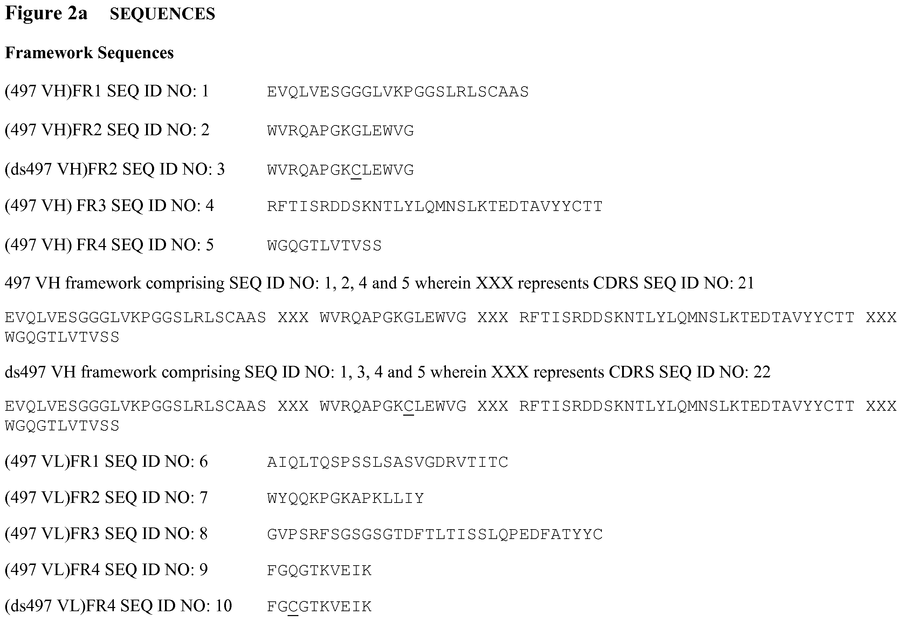

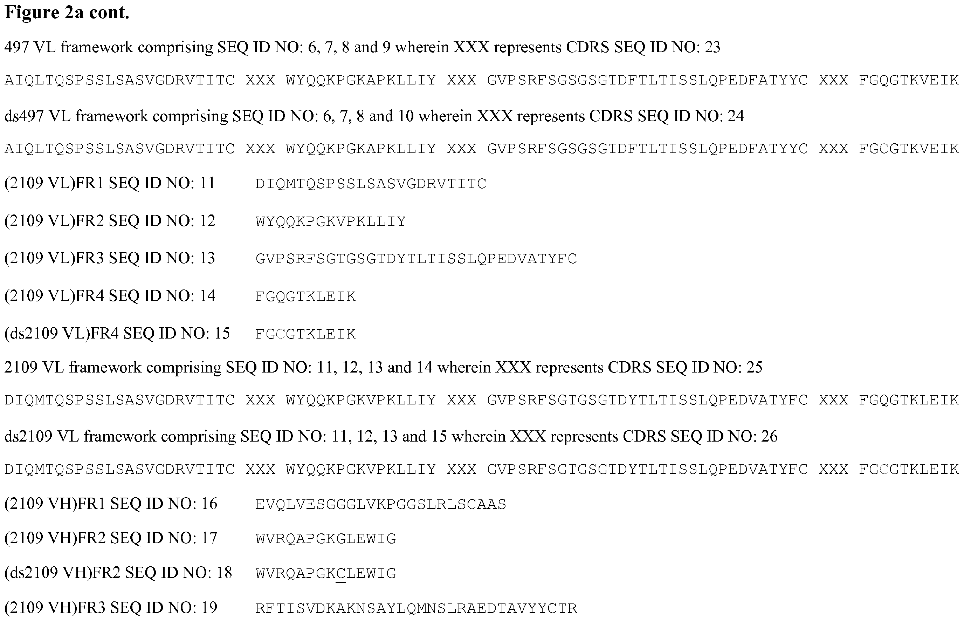

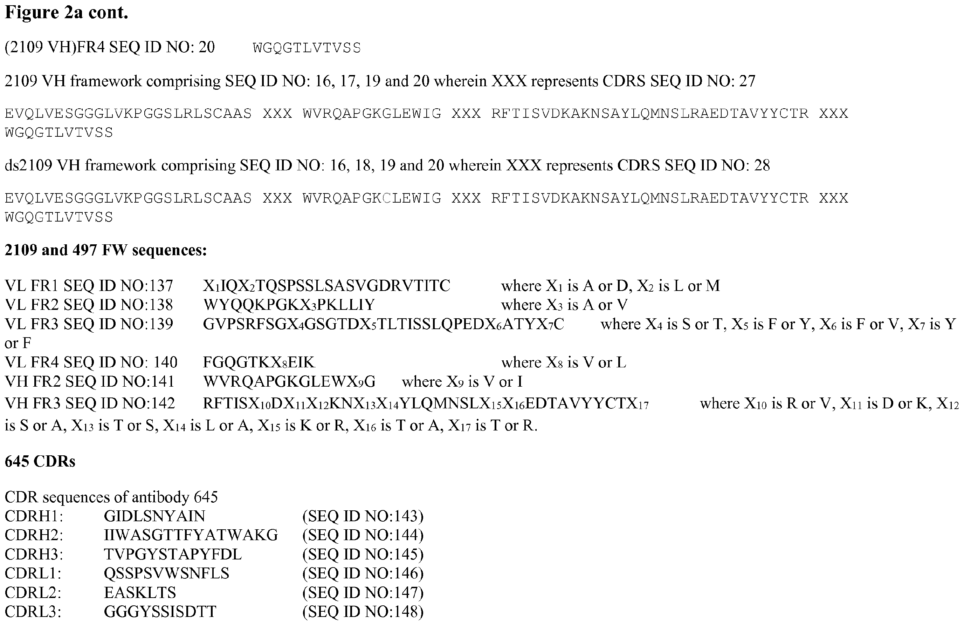

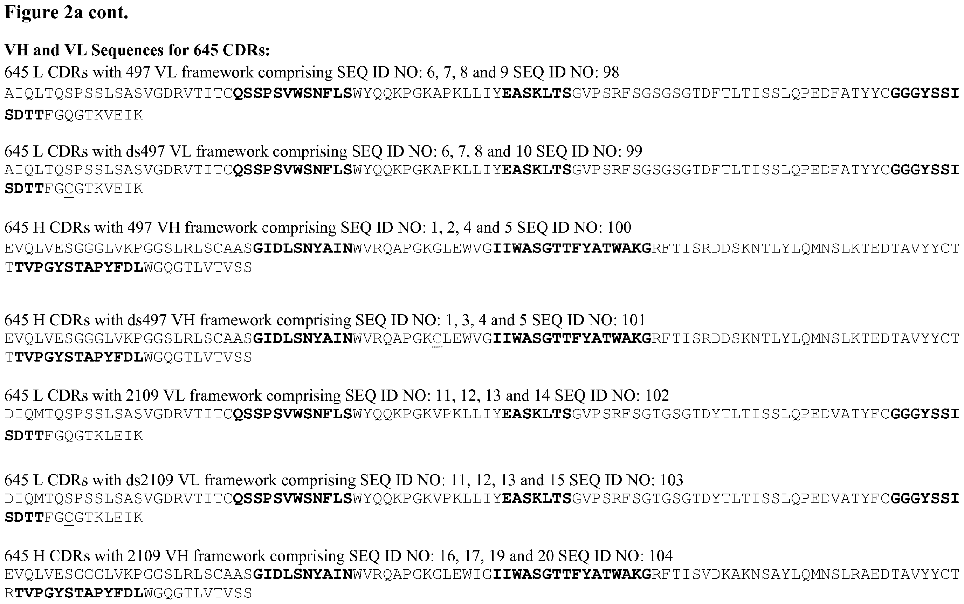

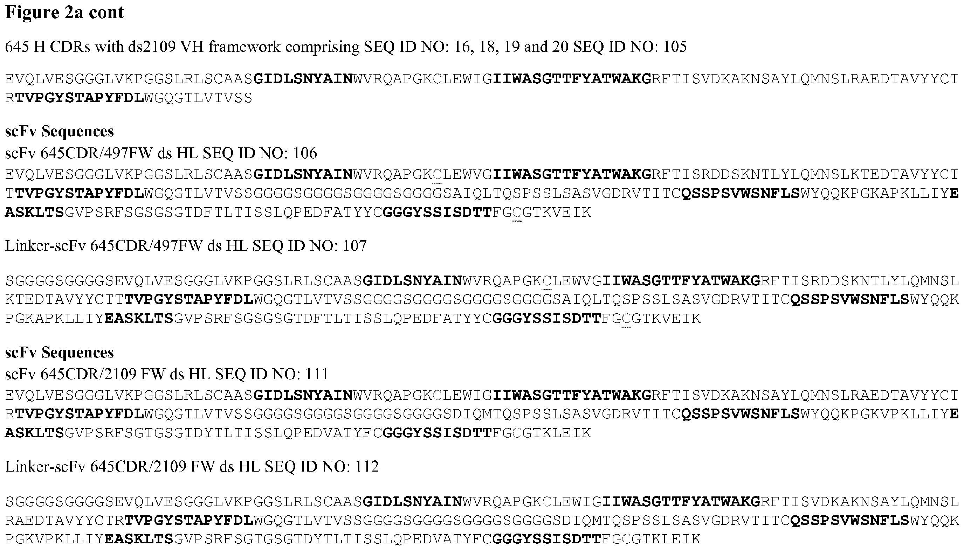

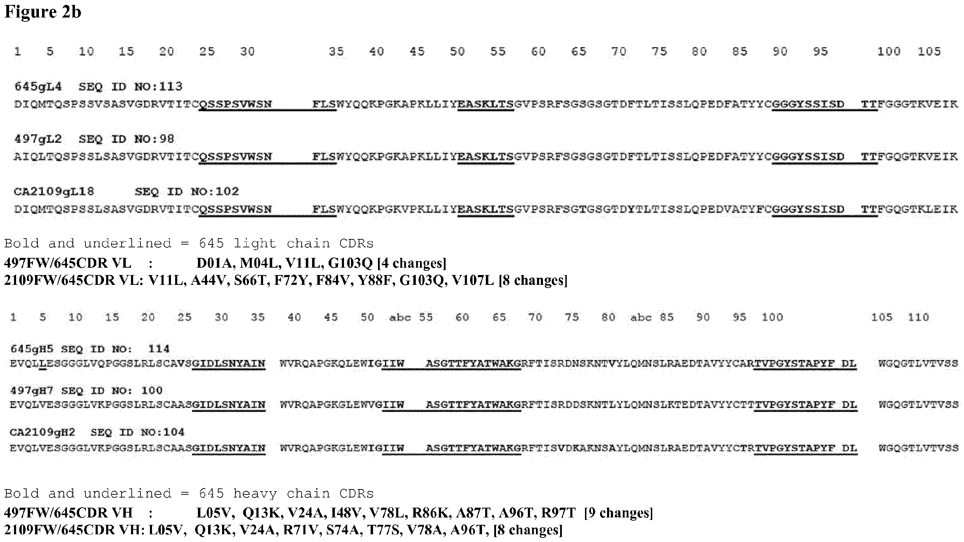

- **Figure 2**: Sets out sequences according to the present invention
- **Figure 3**: Set 1. Expression titres of Fabs and BYbes (cognate frameworks) expressed in ExpiHEK cells, n=2
- **Figure 4**: % monomer of Set 1 Fabs and BYbes (cognate pairs)
- **Figure 5**: SDS-PAGE of Fabs Set 1.Full-length Fab (a) and light and heavy chains (b)
- **Figure 6**: SDS-PAGE of BYbes Set 1
- **Figure 7**: shows the Fab-dsFv and Fab-dsscFv formats and monomer and dimer forms
- **Figure 8**: shows Fab-2x dsscFv formats and Fab-dsscFv-dsFv format

### DETAILED DISCLOSURE

The present inventors have investigated the influence on multimerisation of dsscFv framework (FW) and CDR residues through grafting a range of Fv FW/CDR 'swapped' Fab-dsscFv. Surprisingly they have found that certain frameworks can positively affect the monomer levels achieved.

The present disclosure provides a multispecific antibody molecule comprising at least two binding domains each with a variable heavy region VH and a variable light region VL, wherein each binding domain is specific for an antigen and comprises 6 CDRs, characterised in that at least one binding domain comprises a variable heavy region, VH_{A}, having a framework comprising:
a. SEQ ID NO: 1 for FR1, SEQ ID NO: 3 for FR2, SEQ ID NO: 4 for FR3 and SEQ ID NO: 5 for FR4, with the proviso that CDRH3 in VH_{A} is other than RYYSAMPFAY (SEQ ID NO: 129) and a variable light region, VL_{A}, having a framework comprising SEQ ID NO: 6 for FR1, SEQ ID NO: 7 for FR2, SEQ ID NO: 8 for FR3, and SEQ ID NO: 10 for FR4; or
b. a variable heavy region, VH_{A}, having a framework comprising SEQ ID NO: 16 for FR1, SEQ ID NO: 18 for FR2, SEQ ID NO: 19 for FR3 and SEQ ID NO: 20 for FR4, with the proviso that CDRH3 in VH_{A} is other than RYYSAMPFAY (SEQ ID NO: 129) and a variable light region, VL_{A}, having a framework comprising SEQ ID NO: 11 for FR1, SEQ ID NO: 12 for FR2, SEQ ID NO: 13 for FR3, and SEQ ID NO: 15 for FR4;
and wherein the at least one variable region VH_{A} and the at least one variable region VL_{A} are present in a dsscFv, and wherein the multispecific antibody molecule is a Fab-dsscFv or Fab-(dsscFv)₂.

The multispecific antibody molecules having the VH_{A} and VL_{A} framework residues and sequences as described in the present invention, may comprise any suitable CDR residues. In one embodiment, the multispecific antibody molecule comprises 3 heavy chain CDRs in VH_{A} having the sequence given in SEQ ID NO: 143 for CDRH1, SEQ ID NO: 144 for CDRH2 and SEQ ID NO: 145 for CDRH3. In further embodiment, comprises 3 light chain CDRs in VL_{A} having the sequence given in SEQ ID NO:146 for CDRL1, SEQ ID NO:147 for CDRL2 and SEQ ID NO:148 for CDRL3. In one embodiment, the multispecific antibody molecule comprises 3 heavy chain CDRs in VH_{A} having the sequence given in SEQ ID NO: 143 for CDRH1, SEQ ID NO: 144 for CDRH2 and SEQ ID NO: 145 for CDRH3 and 3 light chain CDRs in VL_{A} having the sequence given in SEQ ID NO:146 for CDRL1, SEQ ID NO:147 for CDRL2 and SEQ ID NO:148 for CDRL3.

In a further embodiment of the present invention the multispecific antibody comprises 497 framework sequences (those of SEQ ID NOs:1, 3-8 and 10).

Accordingly, the present disclosure provides a multispecific antibody molecule comprising at least two binding domains each with a variable heavy region and a variable light region, wherein each binding domain is specific for an antigen and comprises 6 CDRs, characterised in that at least one variable region, VH_{A}, has a framework comprising SEQ ID NO: 1 for FR1, SEQ ID NO: 3 for FR2, SEQ ID NO: 4 for FR3 and SEQ ID NO: 5 for FR4 with the proviso that CDRH3 in VH_{A} is other than RYYSAMPFAY (SEQ ID NO: 129), wherein the VH_{A} is paired with VL_{A}, and wherein VL_{A} has a framework comprising SEQ ID NO: 6 for FR1, SEQ ID NO: 7 for FR2, SEQ ID NO: 8 for FR3, and SEQ ID NO: 10 for FR4, wherein the variable region VH_{A} and the variable region VL_{A} are present in a dsscFv, and wherein the multispecific antibody molecule is a Fab-dsscFv or Fab-(dsscFv)₂.

In one embodiment of the present disclosure there is provided acceptor frameworks for minimising aggregation comprising SEQ ID NO: 22 and SEQ ID NO: 24, wherein XXX in the each of the CDR positions independently represent 1 to 35 amino acids, and wherein SEQ ID NO: 22 and 24 are present in a dsscFv, and wherein the multispecific antibody is a Fab-dsscFv or Fab-(dsscFv)₂.

In one embodiment of the present disclosure there is provided a multispecific antibody molecule comprising at least two binding domains each with a variable heavy region and a variable light region, wherein each binding domain is specific for an antigen and comprises 6 CDRs, characterised in that one variable region, VH_{A}, is paired with variable region VL_{A} having one of the following combinations of VH_{A} and VL_{A} amino acid sequences in Table 3:

**Table 3:**

| **VL_{A}** | **VH_{A}** |
|---|---|
| SEQ ID NO:99 and | SEQ ID NO:101 |

In one aspect the present disclosure provides a dsscFv antibody comprising SEQ ID NO:106 or SEQ ID NO:107 wherein the dsscFv antibody is part of a multispecific antibody BYbe or TrYbe.

In an alternative embodiment of the present invention, at least one binding domain with a variable heavy region VH_{A} and a variable light region VL_{A} comprising 2109 framework sequences as described below (those of SEQ ID NOs:16, 18-20, 11-13 and 15).

In a further embodiment of this aspect of the present invention the multispecific antibody comprises 2109 framework sequences. Accordingly, the present invention provides a multispecific antibody molecule comprising at least two binding domains each with a variable heavy region and a variable light region, wherein each binding domain is specific for an antigen and comprises 6 CDRs, characterised in that at least one variable region, VH_{A}, has a framework comprising SEQ ID NO: 16 for FR1, SEQ ID NO: 18 for FR2, SEQ ID NO: 19 for FR3 and SEQ ID NO: 20 for FR4, with the proviso that CDRH3 in VH_{A} is other than RYYSAMPFAY (SEQ ID NO: 129) or PPQYYEGSIYRLWFAH (SEQ ID NO: 117), wherein the VH_{A} is paired with VL_{A}, and wherein VL_{A} has a framework comprising SEQ ID NO: 11 for FR1, SEQ ID NO: 12 for FR2, SEQ ID NO: 13 for FR3, and SEQ ID NO: 15 for FR4 with the proviso that CDRL3 in VL_{A} is other than QQNYDFPLT (SEQ ID NO: 136) or QQTWSDPWT (SEQ ID NO: 120), wherein the variable region VH_{A} and the variable region VL_{A} are present in a dsscFv, and wherein the multispecific antibody molecule is a Fab-dsscFv or Fab-(dsscFv)₂.

In one embodiment not covered by the claimed invention the present disclosure provides acceptor frameworks for minimising aggregation comprising SEQ ID NO: 26 and 28, wherein XXX in the each of the CDR positions independently represent 1 to 35 amino acids, and wherein SEQ ID NO: 26 and 28 are present in a dsscFv, and wherein the multispecific antibody is a Fab-dsscFv or Fab-(dsscFv)₂.

In an embodiment of the second aspect the present disclosure provides a multispecific antibody molecule comprising at least two binding domains each with a variable heavy region and a variable light region, wherein each binding domain is specific for an antigen and comprises 6 CDRs, characterised in that one variable region, VH_{A}, is paired with variable region VL_{A} having one of the following combinations of VH_{A} and VL_{A} amino acid sequences in

**Table 4:**

| **VL_{A}** | **VH_{A}** |
|---|---|
| SEQ ID NO:103 and | SEQ ID NO:105 |

In one aspect the present disclosure provides a dsscFv antibody comprising SEQ ID NO:111 or SEQ ID NO:112, wherein the dsscFv antibody is part of a multispecific antibody BYbe or a TrYbe.

The multispecific antibody of the present invention may contain the 497 framework residues or sequences as defined above in one or more, such as two, binding domains. The multispecific antibody of the present invention may contain the 2109 framework residues or sequences as defined above in one or more, such as two, binding domains. The multispecific antibody of the present invention may contain the 497 framework residues or sequences as defined above in one binding domain and 2109 framework residues or sequences as defined above in another binding domain.

Accordingly, the present invention provides the following combinations of frameworks in Table 5 as defined in the first and second aspects of the present invention for multispecific antibodies comprising two or more binding domains **Table** 5:

| **Binding Domain 1 - framework residues or sequences of VHA and VLA** | **Binding Domain 2 - framework residues or sequences of VH and VLB** |
|---|---|
| 497 | 497 |
| 2109 | 2109 |
| 497 | 2109 |
| 497 | other |
| 2109 | other |

In any of the above first and second aspects of the present disclosure the variable regions of the present disclosure do not comprise one or more, such as all, the following CDRs of Antibody 1:

| | | |
|---|---|---|
| CDRH1: | GFTFSDYNMA | (SEQ ID NO:115) |
| CDRH2: | TITYEGRNTYYRDSVKG | (SEQ ID NO:116) |
| CDRH3: | PPQYYEGSIYRLWFAH | (SEQ ID NO:117) |
| CDRL1: | RADESVRTLMH | (SEQ ID NO:118) |
| CDRL2: | LVSNSEI | (SEQ ID NO:119) |
| CDRL3: | QQTWSDPWT | (SEQ ID NO:120) |

In any of the above first and second aspects of the present disclosure the variable regions of the present disclosure do not comprise one or more, such as all, the following CDRs of Antibody 2:

| | | |
|---|---|---|
| CDRH1: | GVIFSDYYMA | (SEQ ID NO:121) |
| CDRH2: | SINFNADISYYRESVKG | (SEQ ID NO:122) |
| CDRH3: | DANRQNYDWFAY | (SEQ ID NO:123) |
| CDRL1: | KASESVSSSMYSYMH | (SEQ ID NO:124) |
| CDRL2: | RASNLES | (SEQ ID NO:125) |
| CDRL3: | QQSWTAPRT | (SEQ ID NO:126) |

In any of the above first and second aspects of the present disclosure the variable regions of the present disclosure do not comprise one or more, such as all, the following CDRs of Antibody 3:

| | | |
|---|---|---|
| CDRH1: | GYTFTDNYIH | (SEQ ID NO:127) |
| CDRH2: | YINPSSAYAHYNEKFKT | (SEQ ID NO:128) |
| CDRH3: | RYYSAMPFAY | (SEQ ID NO: 129) |
| CDRL1: | RASEDIYSGLA | (SEQ ID NO:130) or |
| | RASEDIYNGLA | (SEQ ID NO:131) |
| CDRL2: | DSSTLHT | (SEQ ID NO:132), or |
| | NSNTLHT | (SEQ ID NO:133), or |
| | NSSTLHT | (SEQ ID NO:134),or |
| | DSNTLHT | (SEQ ID NO:135) |
| CDRL3: | QQNYDFPLT | (SEQ ID NO:136) |

The variable domain according to the present disclosure does not have a sequence shown in SEQ ID NO: 149.

The variable domain according to the present disclosure does not have a sequence shown in SEQ ID NO: 150.

The variable domain according to the present disclosure does not have a sequence shown in SEQ ID NO: 151.

The variable domain according to the present disclosure does not have a sequence shown in SEQ ID NO: 152.

The variable domain according to the present disclosure does not have a sequence shown in SEQ ID NO: 153.

In the multispecific antibodies of the disclosure, the variable regions do not comprise the CDRs of Antibody 1 defined above, the CDRs of Antibody 2 defined above or the CDRs of Antibody 3 defined above.

In one embodiment at least one binding domain, such as one binding domain in the multispecific antibody molecule does not comprise a framework of the present disclosure.

Advantageously the use of the framework sequences of the present disclosure in variable regions prone to aggregation, for example through the dynamic process referred to as breathing, may provide improved monomer yield and/or a reduced propensity to aggregate.

The present invention also provides a method of improving the levels of monomer expression of an antibody molecule having at least one binding domain with a variable heavy region and variable light region where each variable region has a framework and 3 CDRs, said method comprising the step of changing a framework of a variable region to a framework selected from the group comprising or consisting of:
i) a framework (such a VH region framework) comprising SEQ ID NO: 1 for FR1, SEQ ID NO: 3 for FR2, SEQ ID NO: 4 for FR3 and SEQ ID NO: 5 for FR4,
ii) a framework (such a VH region framework) comprising SEQ ID NO: 16 for FR1, SEQ ID NO: 18 for FR2, SEQ ID NO: 19 for FR3 and SEQ ID NO: 20 for FR4,
iii) a framework (such a VL region framework) comprising SEQ ID NO: 6 for FR1, SEQ ID NO: 7 for FR2, SEQ ID NO: 8 for FR3, and SEQ ID NO: 10 for FR4.
iv) a framework (such a VL region framework) comprising VL1, SEQ ID NO: 11 for FR1, SEQ ID NO: 12 for FR2, SEQ ID NO: 13 for FR3, and SEQ ID NO: 15 for FR4,
v) VH framework and VL framework combinations thereof, and wherein the framework i), ii), iii), iv), or combinations thereof are present in a dsscFv, and wherein the antibody molecule is a Fab-dsscFv or Fab-(dsscFv)₂.

In one embodiment there is provided a method according to the present disclosure wherein the VH framework is changed to a framework comprising SEQ ID NO: 1 for FR1, SEQ ID NO: 3 for FR2, SEQ ID NO: 4 for FR3 and SEQ ID NO: 5 for FR4.

In one embodiment there is provided a method according to the present disclosure wherein the VH framework is changed to a framework comprising SEQ ID NO: 16 for FR1, SEQ ID NO: 18 for FR2, SEQ ID NO: 19 for FR3 and SEQ ID NO: 20 for FR44.

In one embodiment there is provided a method according to present disclosure wherein the VL framework is changed to a framework comprising SEQ ID NO: 6 for FR1, SEQ ID NO: 7 for FR2, SEQ ID NO: 8 for FR3, and SEQ ID NO: 10 for FR4.

In one embodiment there is provided a method according to the present disclosure wherein the VL framework is changed to a framework comprising VL1, SEQ ID NO: 11 for FR1, SEQ ID NO: 12 for FR2, SEQ ID NO: 13 for FR3, and SEQ ID NO: 15 for FR4.

In one embodiment there is provided a method according to the present disclosure wherein the monomer levels of the antibody molecule are analysed before the framework or frameworks is/are changed.

In one embodiment there is provided a method according to the present disclosure wherein the antibody molecule is a multispecific antibody molecule, such as a bispecific or trispecific, such as bispecific.

In one embodiment there provided a method according to the present disclosure wherein the multispecific antibody molecule comprises at least two binding domains and the method comprises the further step of changing the relative position of the binding domains within the antibody molecule.

In one embodiment at least one binding domain of the multispecific antibody does not employ a framework with a reduced propensity to aggregate, as disclosed herein.

In one embodiment the heavy chain germline acceptor sequence employed in the present disclosure is generally the human JH4 VH3 1-U 3-15 (V BASE, http://vbase.mrc-cpe.cam.ac.uk/) and the light chain germline acceptor sequence chosen was the human JK1 or 4 VK1 2-1-(1) L4, L18, O2 or 012 (V BASE, http://vbase.mrc-cpe.cam.ac.uk/).

In one embodiment the CDRs grafted from the donor to the acceptor sequence are as defined by Kabat, with the exception of CDR-H1 where the combined Chothia/Kabat definition is used.

This numbering system is set forth in Kabat *et al.,* 1987, in Sequences of Proteins of Immunological Interest, US Department of Health and Human Services, NIH, USA (hereafter "Kabat *et al. (supra)").* This numbering system is used in the present specification except where otherwise indicated.

The Kabat residue designations do not always correspond directly with the linear numbering of the amino acid residues. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence.

The CDRs of the heavy chain variable domain are located at residues 31-35 (CDR-H1), residues 50-65 (CDR-H2) and residues 95-102 (CDR-H3) according to the Kabat numbering system. However, according to Chothia (Chothia, C. and Lesk, A.M. J. Mol. Biol., 196, 901-917 (1987)), the loop equivalent to CDR-H1 extends from residue 26 to residue 32. Thus 'CDR-H1', as used herein, comprises residues 26 to 35, as described by a combination of the Kabat numbering system and Chothia's topological loop definition.

The CDRs of the light chain variable domain are located at residues 24-34 (CDR-L1), residues 50-56 (CDR-L2) and residues 89-97 (CDR-L3) according to the Kabat numbering system.

"Multi-specific antibody" as employed herein refers to an antibody molecule as described herein which has two or more binding domains, for example two or three binding domains. In one embodiment the multi-specific antibody molecule of the present disclosure has only two antigen binding sites.

In one embodiment the construct is a bi-specific antibody. "Bi-specific molecule" as employed herein refers to a molecule with two antigen binding sites, which may bind the same or different antigens. In one embodiment the bi-specific molecule binds two different antigens. In one embodiment the domains all bind the same antigen, including binding the same epitope on the antigen or binding different epitopes on the same antigen.

"Antigen binding site" as employed herein refers to a portion of the molecule, which comprises a pair of variable regions, in particular a cognate pair, that interact specifically with the target antigen, i.e. the portion of the molecule that specifically recognises the region, shape, area, epitope in another protein/polypeptide.

"Specifically" as employed herein is intended to refer to a binding site that only recognises the antigen to which it is specific or a binding site that has significantly higher binding affinity to the antigen to which it is specific compared to affinity it has for antigens to which it is non-specific, for example 5, 6, 7, 8, 9, 10 times higher binding affinity.

Binding affinity may be measured by standard assays, for example surface plasmon resonance, such as BIAcore.

In one embodiment there is provided a multispecific antibody molecule according to the present disclosure, wherein the antibody molecule is bispecific or trispecific, such as bispecific.

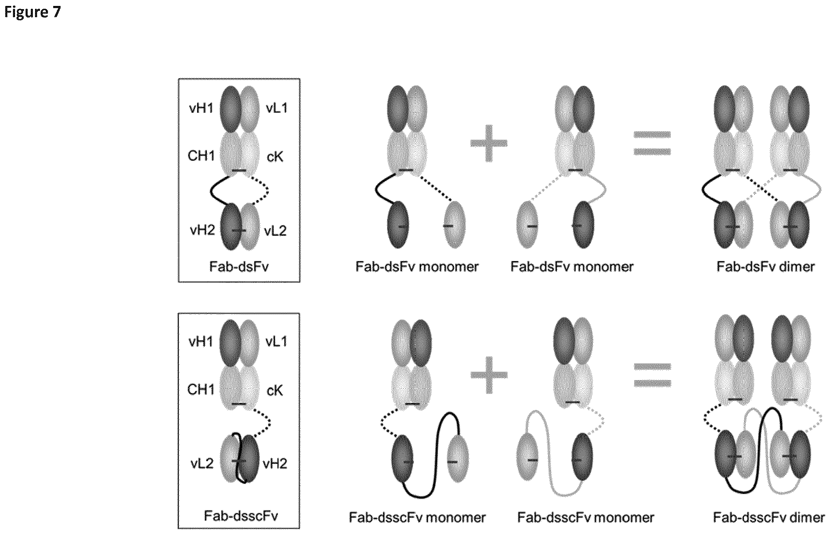

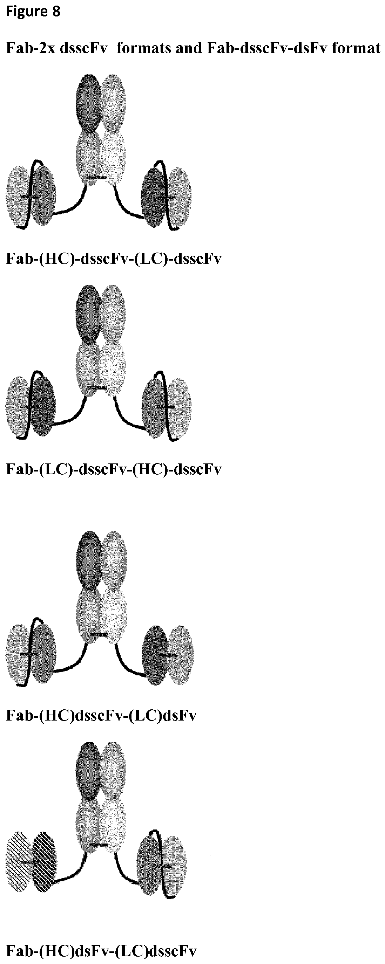

In one embodiment multivalent formats include those known in the art and those described herein (including disclosures in the definition section of the specification), wherein the molecule format is selected from the group comprising or consisting of: Fab-dsscFv (also referred to as BYbe), Fab-(dsscFv)₂, which are discussed in more detail below. Fab-dsscFv (BYbe) is also shown in Figure 7. Fab-(dsscFv)₂ formats are also shown in Figure 8 and also known as TrYbe format.

In one embodiment the VH_{A} and VL_{A} regions having the framework residues or sequences as described in the present invention are present in a dsscFv. The dsscFv is part of a multispecific antibody molecule wherein the dsscFv is part of a BYbe or TrYbe antibody molecule.

In one embodiment, the multi-specific antibody molecule, comprises or consists of:
a) a polypeptide chain of formula (**Ia**):

   **V_{H}-CH₁-X-V₁;**
b) a polypeptide chain of formula (**IIa**):

   **V_{L}-C_{L};**
wherein
- V_{H}: represents a heavy chain variable domain;
- CH₁: represents a domain of a heavy chain constant region, for example domain 1 thereof;
- X: represents a bond or linker;
- V₁: represents a dsscFv;
- V_{L}: represents a variable domain, for example a light chain variable domain;
- C_{L}: represents a domain from a constant region, for example a light chain constant region domain, such as Ckappa.

In one embodiment, there is provided a multi-specific antibody molecule, comprising or consisting of:
a) a polypeptide chain of formula (**Ib**):

   **V_{H}-CH₁;**
b) a polypeptide chain of formula (**IIb**):

   **V_{L}-C_{L}-Y-V₂;**

wherein V_{H}, CH₁, V_{L}, C_{L} are as defined above and;
wherein Y represents a bond or linker and;
wherein V₂ represents a dsscFv.

In one embodiment the multispecific antibody molecule according to the present disclosure, is a BYbe (i.e. an antibody molecule of formula (Ia and IIa) or (Ib and IIb) also as shown in Figure 7.

In the method according to the present invention, in one embodiment the inserted/replaced framework or frameworks are in a dsscFv where the dsscFv is part of a BYbe.

In one embodiment the inserted framework is in the dsscFv component of a BYbe.

In one embodiment, the multi-specific antibody molecule according to the present disclosure is provided as a dimer of a heavy and light chain (together one pair is referred to herein as "monomer") of:
- formula **(I)** and **(II)** respectively, wherein the V_{H}-CH₁ portion together with the V_{L}-C_{L} portion form a functional Fab or Fab' fragment; or
- formula **(Ia)** and **(IIa)** respectively, wherein the V_{H}-CH₁ portion together with the V_{L}-C_{L} portion form a functional Fab or Fab' fragment; or
- formula **(Ib)** and **(IIb)** respectively, wherein the V_{H}-CH₁ portion together with the V_{L}-C_{L} portion form a functional Fab or Fab' fragment.

In one aspect of the present invention, the multi-specific antibody molecule is provided as a dimer comprising or consisting of:
a) a polypeptide chain of formula (I):

   **V_{H1}-CH₁-X-V₁;** and
b) a polypeptide chain of formula (**II**):

   **V_{L1}-C_{L}-Y-V₂;**
wherein:
- V_{H1}: represents a heavy chain variable domain;
- CH₁: represents a domain of a heavy chain constant region, for example domain 1 thereof;
- X: represents a bond or linker;
- Y: represents a bond or linker;
- V₁: represents a dsscFv;
- V_{L1}: represents a light chain variable domain;
- C_{L}: represents a domain from a light chain constant region, such as Ckappa;
- V₂: represents a dsscFv.

V₁ is a dsscFv and V₂ is a dsscFv which may also be referred to as a Fab-(dsscFv)₂. VI and/or V2 may contain the VHA and VLA framework sequences according to the present invention.

V_{H} represents a variable domain. In one embodiment V_{H} represents a heavy chain variable domain. In one embodiment V_{H} is a chimeric variable domain, that is to say it comprises components derived from at least two species, for example a human framework and non-human CDRs. In one embodiment V_{H} is humanised. In one embodiment the V_{H} is fully human.

V_{L} represents a variable domain. In one embodiment V_{L} represents a light chain variable domain. In one embodiment V_{L} is a chimeric variable domain, that is to say it comprises components derived from at least two species, for example a human framework and non-human CDRs. In one embodiment V_{L} is humanised. In one embodiment the V_{L} is fully human.

Generally, V_{H} and V_{L} together form an antigen binding domain. In one embodiment V_{H} and V_{L} form a cognate pair.

"Cognate pair" as employed herein refers to a pair of variable domains from a single antibody, which was generated *in vivo,* i.e. the naturally occurring pairing of the variable domains isolated from a host. A cognate pair is therefore a V_{H} and V_{L} pair. In one example the cognate pair bind the antigen co-operatively. Cognate pairs are often advantageous because affinity maturation for the antigen occurs in the host and may be highly optimised and very specific for said antigen.

"Variable region" as employed herein refers to the region in an antibody chain comprising the 3 CDRs and a suitable framework. Variable regions for use in the present disclosure will generally be derived from an antibody (such as an antibody isolated from a host), which may be generated by any method known in the art.

"Derived from" as employed herein refers to the fact that the sequence employed or a sequence highly similar to the sequence employed was obtained from the original genetic material, such as the light or heavy chain of an antibody.

"Highly similar" as employed herein is intended to refer to an amino acid sequence which over its full length is 95% similar or more, such as 96, 97, 98 or 99% similar.

In one embodiment the binding domain formed by V_{H} and V_{L} is specific to a first antigen.

In one embodiment the CH₁ domain is a naturally occurring domain 1 from a heavy chain or a derivative thereof, for example the IgG1 CH1 sequence provided in SEQ ID NO:108 or the IgG4 CH1 sequence provided in SEQ ID NO: 109.

In one embodiment the C_{L} fragment, in the light chain, is a constant kappa sequence or a constant lambda sequence or a derivative of either of the same, for example the kappa sequence provided in SEQ ID NO: 110.

A derivative of a naturally occurring domain as employed herein is intended to refer to where one, two, three, four or five amino acids in a naturally occurring sequence have been replaced or deleted, for example to optimize the properties of the domain such as eliminating undesirable properties but wherein the characterizing feature(s) of the domain is/are retained.

In one embodiment one or more natural or engineered inter chain (i.e. inter light and heavy chain) disulphide bonds are present in the functional Fab or Fab' fragment.

In one embodiment a "natural" disulfide bond is present between a CH₁ and C_{L} in the polypeptide chains of the constructs of the present disclosure, for example formula (I) and **(II);** or formula **(Ia)** and **(IIa);** or formula **(Ib)** and **(IIb).**

When the C_{L} domain is derived from either Kappa or Lambda the natural position for a bond forming cysteine is 214 in human cKappa and cLambda (Kabat numbering 4^{th} edition 1987).

The exact location of the disulfide bond forming cysteine in CH₁ depends on the particular domain actually employed. Thus, for example in human gamma-1 the natural position of the disulfide bond is located at position 233 (Kabat numbering 4^{th} edition 1987). The position of the bond forming cysteine for other human isotypes such as gamma 2, 3, 4, IgM and IgD are known, for example position 127 for human IgM, IgE, IgG2, IgG3, IgG4 and 128 of the heavy chain of human IgD and IgA2B.

A disulfide bond or bond(s) in the constant region of the molecule may be in addition to the disulfide bond between the variable domain pair V_{H} and V_{L}.

In one embodiment the multi-specific antibody according to the disclosure has a disulfide bond in a position equivalent or corresponding to that naturally occurring between CH₁ and C_{L}.

In one embodiment a constant region comprising CH₁ and a constant region such as C_{L} has a disulfide bond which is in a non-naturally occurring position. This may be engineered into the molecule by introducing cysteine(s) into the amino acid chain at the position or positions required. This non-natural disulfide bond is in addition to or as an alternative to the natural disulfide bond present between CH₁ and C_{L}.

Introduction of engineered cysteines can be performed using any method known in the art. These methods include, but are not limited to, PCR extension overlap mutagenesis, site-directed mutagenesis or cassette mutagenesis (see, generally, Sambrook et al., Molecular Cloning, A Laboratory Manual, Cold Spring Harbour Laboratory Press, Cold Spring Harbour, NY, 1989; Ausbel et al., Current Protocols in Molecular Biology, Greene Publishing & Wiley-Interscience, NY, 1993). Site-directed mutagenesis kits are commercially available, e.g. QuikChange^{®} Site-Directed Mutagenesis kit (Stratagene, La Jolla, CA). Cassette mutagenesis can be performed based on Wells et al., 1985, Gene, 34:315-323. Alternatively, mutants can be made by total gene synthesis by annealing, ligation and PCR amplification and cloning of overlapping oligonucleotides.

"Single chain variable fragment" or "scFv" as employed herein refers to a single chain variable fragment which is stabilised by a peptide linker between the V_{H} and V_{L} variable domains.

"Disulphide-stabilised single chain variable fragment" or "dsscFv" as employed herein refers to a single chain variable fragment which is stabilised by a peptide linker between the V_{H} and V_{L} variable domain and also includes an inter-domain disulphide bond between V_{H} and V_{L}.

In one embodiment, disulfide bond between the variable domains V_{H} and V_{L} of V₁ or V₂ is between two of the residues listed below (unless the context indicates otherwise Kabat numbering is employed in the list below). Wherever reference is made to Kabat numbering the relevant reference is Kabat et al., 1987, in Sequences of Proteins of Immunological Interest, US Department of Health and Human Services, NIH, USA.

In one embodiment the disulfide bond is in a position selected from the group comprising:
- V_{H}37 + V_{L}95C see for example Protein Science 6, 781-788 Zhu et al (1997);
- V_{H}44 + V_{L}100 see for example; Biochemistry 33 5451-5459 Reiter et al (1994); or Journal of Biological Chemistry Vol. 269 No. 28 pp. 18327-18331 Reiter et al (1994); or Protein Engineering, vol.10 no.12 pp.1453-1459 Rajagopal et al (1997);
- V_{H}44 + V_{L}105 see for example J Biochem. 118, 825-831 Luo et al (1995);
- V_{H}45 + V_{L}87 see for example Protein Science 6, 781-788 Zhu et al (1997);
- V_{H}55 + V_{L}101 see for example FEBS Letters 377 135-139 Young et al (1995);
- V_{H}100 + V_{L}50 see for example Biochemistry 29 1362-1367 Glockshuber et al (1990);
- V_{H}100b + V_{L}49;
- V_{H}98 + V_{L} 46 see for example Protein Science 6, 781-788 Zhu et al (1997);
- V_{H}101 + V_{L}46;
- V_{H}105 + V_{L}43 see for example; Proc. Natl. Acad. Sci. USA Vol. 90 pp.7538-7542 Brinkmann et al (1993); or Proteins 19, 35-47 Jung et al (1994),
- V_{H}106 + V_{L}57 see for example FEBS Letters 377 135-139 Young et al (1995)
and a position or positions corresponding thereto in variable region pair located in the molecule.

In one embodiment, the disulphide bond is formed between positions V_{H}44 and V_{L}100.

The amino acid pairs listed above are in the positions conducive to replacement by cysteines such that disulfide bonds can be formed. Cysteines can be engineered into these desired positions by known techniques. In one embodiment therefore an engineered cysteine according to the present disclosure refers to where the naturally occurring residue at a given amino acid position has been replaced with a cysteine residue.

Introduction of engineered cysteines can be performed using any method known in the art. These methods include, but are not limited to, PCR extension overlap mutagenesis, site-directed mutagenesis or cassette mutagenesis (see, generally, Sambrook et al., Molecular Cloning, A Laboratory Manual, Cold Spring Harbour Laboratory Press, Cold Spring Harbour, NY, 1989; Ausbel et al., Current Protocols in Molecular Biology, Greene Publishing & Wiley-Interscience, NY, 1993). Site-directed mutagenesis kits are commercially available, e.g. QuikChange^{®} Site-Directed Mutagenesis kit (Stratagen, La Jolla, CA). Cassette mutagenesis can be performed based on Wells et al., 1985, Gene, 34:315-323. Alternatively, mutants can be made by total gene synthesis by annealing, ligation and PCR amplification and cloning of overlapping oligonucleotides.

Accordingly, in one embodiment the variable domains V_{H} and V_{L} of V₁ or V₂ may be linked by a disulfide bond between two cysteine residues, wherein the position of the pair of cysteine residues is selected from the group comprising or consisting of: V_{H}37 and V_{L}95, V_{H}44 and V_{L}100, V_{H}44 and V_{L}105, V_{H}45 and V_{L}87, V_{H}100 and V_{L}50, V_{H}100b and V_{L}49, V_{H}98 and V_{L}46, V_{H}101 and V_{L}46, V_{H}105 and V_{L}43 and V_{H}106 and V_{L}57.

In one embodiment, the variable domains V_{H} and V_{L} of V₁ or V₂ may be linked by a disulfide bond between two cysteine residues, one in V_{H} and one in V_{L}, which are outside of the CDRs, for example wherein the position of the pair of cysteine residues is selected from the group consisting of V_{H}37 and V_{L}95, V_{H}44 and V_{L}100, V_{H}44 and V_{L}105, V_{H}45 and V_{L}87, V_{H}100 and V_{L}50, V_{H}98 and V_{L}46, V_{H}105 and V_{L}43 and V_{H}106 and V_{L}57.

In one embodiment, the variable domains V_{H} and V_{L} of V₁ are linked by a disulphide bond between two engineered cysteine residues, one at position V_{H}44 and the other at V_{L}100.

In one embodiment, the variable domains V_{H} and V_{L} of V₂ are linked by a disulphide bond between two engineered cysteine residues, one at position V_{H}44 and the other at V_{L}100.

In one embodiment, the heavy chain variable domain of the dsscFv V₁ is attached to X or the heavy chain variable domain of the dsscFv V₂ is attached to Y.

In one embodiment, the light chain variable domain of the dsscFvVi is attached to X or the light chain variable domain of the dsscFv V₂ is attached to Y.

In one embodiment, the V_{H} domain of V₁ is attached to X, for example is attached to CH₁. In one embodiment, the V_{L} domain of V₁ is attached to X, for example is attached to CH₁. In one embodiment, the V_{H} domain of V₂ is attached to Y, for example is attached to CKappa. In one embodiment, the V_{L} domain of V₂ is attached to Y, for example is attached to CKappa. In one embodiment X is a bond. In one embodiment Y is a bond. In one embodiment both X and Y are bonds.

In one embodiment X is a linker, for example a suitable peptide for connecting the portions CH₁ and V₁. In one embodiment Y is a linker, for example a suitable peptide for connecting the portions C_{L} and V₂. In one embodiment both X and Y are linkers.

In one embodiment the peptide linker is 50 amino acids in length or less, for example 20 amino acids or less, such as 19, 10, 17, 16, 15, 14, 13, 12, 11, 10, 9, 8, 7, 6, 5, 4, 3, 2 or 1 amino acids.

In one embodiment the linker is based on repeating units of G4S (i.e. GGGGS SEQ ID NO: 42), for example 1, 2, 3, 4 or 5 units thereof, such as SGGGGSGGGGSGGGGSGGGGS (SEQ ID NO:45). In embodiment a linker employed in a construct of the present disclosure has the sequence shown in SEQ ID NO:46, for example the sequence may be employed in when X or Y is a linker.

Of course, there are at least three possible locations for linkers in the constructs of the present the disclosure, X, Y and in the scFv component.

In one embodiment the linker is selected from a sequence shown herein, for example sequence 29 to 97. In one embodiment the linker is selected from a sequence shown in SEQ ID NO: 29 or SEQ ID NO: 30. In one embodiment X has the sequence shown in SEQ ID NO: 29. In one embodiment Y has the sequence shown in SEQ ID NO: 30. Hinge sequences are shown in SEQ ID NO: 31 to 39. Flexible linker sequences are shown in SEQ ID NO: 40 to 80, where (S) is optional in sequences 42 to 47. **Table 6:**

| | |
|---|---|
| 42 | (S)GGGGS |
| 43 | (S)GGGGSGGGGS |
| 44 | (S)GGGGSGGGGSGGGGS |
| 45 | (S)GGGGSGGGGSGGGGSGGGGS |
| 47 | (S)GGGGSGGGGSGGGGSGGGGSGGGGS |

Examples of rigid linkers include the peptide sequences shown in SEQ ID NO: 81, SEQ ID NO: 82 and PPP.

In one embodiment the peptide linker is an albumin binding peptide.

Examples of albumin binding peptides are provided in WO2007/106120 and include the linkers in SEQ ID NO: 83 to 96.

Advantageously, use of albumin binding peptides as a linker may increase the half-life of the bi-specific antibody molecule.

In one embodiment there is a linker (for example a suitable peptide linker) connecting the variable domains V_{H} and V_{L} of V₁ (i.e. between V_{H} and V_{L}). In one embodiment there is a linker (for example a suitable peptide linker) connecting the variable domains V_{H} and V_{L} of V₂ (i.e. between V_{H} and V_{L}).

In one embodiment, the linker connecting the variable domains V_{H} and V_{L} of V₁ has the sequence GGGGSGGGGSGGGGSGGGGS (SEQ ID NO: 97).

In one embodiment, the linker connecting the variable domains V_{H} and V_{L} of V₂ has the sequence GGGGSGGGGSGGGGSGGGGS (SEQ ID NO: 97).

The term "Fab fragment" as used herein refers to an antibody fragment comprising a light chain fragment comprising a VL (variable light) domain and a constant domain of a light chain (CL), and a VH (variable heavy) domain and a first constant domain (CH1) of a heavy chain.

The Fv refers two variable domains, for example co-operative variable domains, such as a cognate pair or affinity matured variable domains, i.e. a VH and VL pair.

Co-operative variable domains as employed herein are variable domains that complement each other and/or both contribute to antigen binding to render the Fv specific for the antigen in question.

The term "single-chain Fv" or abbreviated as "scFv", as used herein refers to an antibody fragment that comprises the VH and VL antibody domains linked (for example by a peptide linker) to form a single polypeptide chain. The constant regions of the heavy and light chain are omitted in this format.

The term "single domain antibody" as used herein refers to an antibody fragment consisting of a single monomeric variable antibody domain. Examples of single domain antibodies include V_{H} or V_{L} or V_{H}H. These terms are used interchangeably herein.

Diabody as employed herein refers to two Fv pairs VH/VL and a further VH/VL pair which have two inter-Fv linkers, such that the VH of a first Fv is linked to the VL of the second Fv and the VL of the first Fv is linked to the VH of the second Fv.

Triabody as employed herein refers to a format similar to the diabody comprising three Fvs and three inter-Fv linkers.

Tetrabody as employed herein refers to a format similar to the diabody comprising fours Fvs and four inter-Fv linkers.

Tandem scFv as employed herein refers to two scFvs linked via a single linker such that there is a single inter-Fv linker.

Tandem scFv-Fc as employed herein refers to two tandem scFvs, wherein each one is appended to the N-terminus of a CH₂ domain, for example via a hinge, of constant region fragment -CH₂CH₃.

FabFv as employed herein refers to a Fv fragment with a variable region appended to the C-terminal of each of the following, the CH1 of the heavy chain and CL of the light chain. The format may be provided as a PEGylated version thereof.

Fab'Fv as employed herein is similar to FabFv, wherein the Fab portion is replaced by a Fab'. The format may be provided as a PEGylated version thereof.

FabdsFv as employed herein refers to a FabFv wherein an intra-Fv disulfide bond stabilises the appended C-terminal variable regions. The format may be provided as a PEGylated version thereof.

Fab-scFv as employed herein is a Fab molecule with a scFv appended on the C-terminal of the light or heavy chain.

Fab'-scFv as employed herein is a Fab' molecule with a scFv appended on the C-terminal of the light or heavy chain.

DiFab as employed herein refers to two Fab molecules linked via their C-terminus of the heavy chains.

DiFab' as employed herein refers to two Fab' molecules linked via one or more disulfide bonds in the hinge region thereof.

As employed herein scdiabody is a diabody comprising an intra-Fv linker, such that the molecule comprises three linkers and forms a normal scFv whose VH and VL terminals are each linked to a one of the variable regions of a further Fv pair.

Scdiabody-Fc as employed herein is two scdiabodies, wherein each one is appended to the N-terminus of a CH₂ domain, for example via a hinge, of constant region fragment - CH₂CH₃.

ScFv-Fc-scFv as employed herein refers to four scFvs, wherein one of each is appended to the N-terminus and the C-terminus of both the heavy and light chain of a -CH₂CH₃ fragment.

Scdiabody-CH₃ as employed herein refers to two scdiabody molecules each linked, for example via a hinge to a CH₃ domain.

IgG-scFv as employed herein is a full-length antibody with a scFv on the C-terminal of each of the heavy chains or each of the light chains.

scFv-IgG as employed herein is a full-length antibody with a scFv on the N-terminal of each of the heavy chains or each of the light chains.

V-IgG as employed herein is a full-length antibody with a variable domain on the N-terminal of each of the heavy chains or each of the light chains.

IgG-V as employed herein is a full-length antibody with a variable domain on the C-terminal of each of the heavy chains or each of the light chains

DVD-Ig (also known as dual V domain IgG) is a full-length antibody with 4 additional variable domains, one on the N-terminus of each heavy and each light chain.

The present disclosure also provides sequences which are 80%, 90%, 91%, 92%, 93% 94%, 95% 96%, 97%, 98% or 99% similar to a sequence disclosed herein.

"Identity", as used herein, indicates that at any particular position in the aligned sequences, the amino acid residue is identical between the sequences.

"Similarity", as used herein, indicates that, at any particular position in the aligned sequences, the amino acid residue is of a similar type between the sequences. For example, leucine may be substituted for isoleucine or valine. Other amino acids which can often be substituted for one another include but are not limited to:
- phenylalanine, tyrosine and tryptophan (amino acids having aromatic side chains);
- lysine, arginine and histidine (amino acids having basic side chains);
- aspartate and glutamate (amino acids having acidic side chains);
- asparagine and glutamine (amino acids having amide side chains); and
- cysteine and methionine (amino acids having sulphur-containing side chains).

Degrees of identity and similarity can be readily calculated (Computational Molecular Biology, Lesk, A.M., ed., Oxford University Press, New York, 1988; Biocomputing. Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987, Sequence Analysis Primer, Gribskov, M. and Devereux, J., eds., M Stockton Press, New York, 1991, the BLAST^{™} software available from NCBI (Altschul, S.F. et al., 1990, J. Mol. Biol. 215:403-410; Gish, W. & States, D.J. 1993, Nature Genet. 3:266-272. Madden, T.L. et al., 1996, Meth. Enzymol. 266:131-141; Altschul, S.F. et al., 1997, Nucleic Acids Res. 25:3389-3402; Zhang, J. & Madden, T.L. 1997, Genome Res. 7:649-656,).

In one embodiment there is provided a multispecific antibody molecule according to the present disclosure, wherein the CDRs have a non-human origin, in particular mammalian, for example from a rat, mouse, rabbit, camelid, sheep, goat or the like. In one embodiment the CDRs are human.

Antibodies generated against an antigen polypeptide may be obtained, where immunisation of an animal is necessary, by administering the polypeptides to an animal, preferably a non-human animal, using well-known and routine protocols, see for example Handbook of Experimental Immunology, D. M. Weir (ed.), Vol 4, Blackwell Scientific Publishers, Oxford, England, 1986). Many warm-blooded animals, such as rabbits, mice, rats, sheep, cows, camels or pigs may be immunized. However, mice, rabbits, pigs and rats are generally most suitable.

Methods are known for isolating human antibody repertoires from humans, for example isolation from a peripheral blood sample, wherein the sample is from a person previously exposed to a relevant antigen (such as a pathogen).

Monoclonal antibodies may be prepared by any method known in the art such as the hybridoma technique (Kohler & Milstein, 1975, Nature, 256:495-497), the trioma technique, the human B-cell hybridoma technique (Kozbor et al., 1983, Immunology Today, 4:72) and the EBV-hybridoma technique (Cole et al., Monoclonal Antibodies and Cancer Therapy, pp77-96, Alan R Liss, Inc., 1985).

Antibodies may also be generated using single lymphocyte antibody methods by cloning and expressing immunoglobulin variable region cDNAs generated from single lymphocytes selected for the production of specific antibodies by, for example, the methods described by Babcook, J. et al., 1996, Proc. Natl. Acad. Sci. USA 93(15):7843-78481; WO92/02551; WO2004/051268 and WO2004/106377.

The antibodies for use in the present disclosure can also be generated using various phage display methods known in the art and include those disclosed by Brinkman et al. (in J. Immunol. Methods, 1995, 182: 41-50), Ames et al. (J. Immunol. Methods, 1995, 184:177-186), Kettleborough et al. (Eur. J. Immunol. 1994, 24:952-958), Persic et al. (Gene, 1997 187 9-18), Burton et al. (Advances in Immunology, 1994, 57:191-280) and WO90/02809; WO91/10737; WO92/01047; WO92/18619; WO93/11236; WO95/15982; WO95/20401; and US5,698,426; US5,223,409; US5,403,484; US5,580,717; US5,427,908; US5,750,753; US5,821,047; US5,571,698; US5,427,908; US5,516,637; US5,780,225; US5,658,727; US5,733,743; US5,969,108, and WO20011/30305.

In one embodiment the multi-specific molecules according to the disclosure are humanised (one, two or all binding domains are humanised).

Humanised (which include CDR-grafted antibodies) as employed herein refers to molecules having one or more complementarity determining regions (CDRs) from a non-human species and a framework region from a human immunoglobulin molecule (see, e.g. US5,585,089; WO91/09967). It will be appreciated that it may only be necessary to transfer the specificity determining residues of the CDRs rather than the entire CDR (see for example, Kashmiri et al., 2005, Methods, 36, 25-34). Humanised antibodies may optionally further comprise one or more framework residues derived from the non-human species from which the CDRs were derived.

As used herein, the term "humanised antibody molecule" refers to an antibody molecule wherein the heavy and/or light chain contains one or more CDRs (including, if desired, one or more modified CDRs) from a donor antibody (e.g. a murine monoclonal antibody) grafted into a heavy and/or light chain variable region framework of an acceptor antibody (e.g. a human antibody) see, e.g. US 5,585,089; WO91/09967. For a review, see Vaughan et al, Nature Biotechnology, 16, 535-539, 1998. In one embodiment rather than the entire CDR being transferred, only one or more of the specificity determining residues from any one of the CDRs described herein above are transferred to the human antibody framework (see for example, Kashmiri et al., 2005, Methods, 36, 25-34). In one embodiment only the specificity determining residues from one or more of the CDRs described herein above are transferred to the human antibody framework. In another embodiment only the specificity determining residues from each of the CDRs described herein above are transferred to the human antibody framework.

In one embodiment the multi-specific molecules according to the disclosure are humanised

When the CDRs or specificity determining residues are grafted, any appropriate acceptor variable region framework sequence may be used having regard to the class/type of the donor antibody from which the CDRs are derived, including mouse, primate and human framework regions. Suitably, the humanised antibody according to the present invention has a variable domain comprising human acceptor framework regions as well as one or more of the CDRs provided from the donor antibody or disclosed herein.

In a humanised antibody of the present disclosure, the framework regions need not have exactly the same sequence as those of the acceptor antibody. For instance, unusual residues may be changed to more frequently-occurring residues for that acceptor chain class or type. Alternatively, selected residues in the acceptor framework regions may be changed so that they correspond to the residue found at the same position in the donor antibody (see Reichmann et al., 1998, Nature, 332, 323-324). Such changes should be kept to the minimum necessary to recover the affinity of the donor antibody. A protocol for selecting residues in the acceptor framework regions which may need to be changed is set forth in WO91/09967.

Donor antibody as employed herein refers to the original antibody isolated from a host or identified in a library.

A donor residue as employed herein is a residue found in the antibody that donated the CDRs. Generally, the donor antibody will be non-human and thus a donor residue will be from a non-human antibody. In one embodiment the donor residue is a residue in the same or corresponding position in the donor antibody to the position of the residue being replaced in the humanised antibody.

Examples of human frameworks which can be used in the present disclosure are KOL, NEWM, REI, EU, TUR, TEI, LAY and POM (Kabat et al., supra). For example, KOL and NEWM can be used for the heavy chain, REI can be used for the light chain and EU, LAY and POM can be used for both the heavy chain and the light chain. Alternatively, human germline sequences may be used; these are available at: http://www2.mrc-lmb.cam.ac.uk/vbase/list2.php.

In a humanised antibody molecule of the present disclosure, the acceptor heavy and light chains do not necessarily need to be derived from the same antibody and may, if desired, comprise composite chains having framework regions derived from different chains.

The framework regions need not have exactly the same sequence as those of the acceptor antibody. For instance, unusual residues may be changed to more frequently-occurring residues for that acceptor chain class or type. Alternatively, selected residues in the acceptor framework regions may be changed so that they correspond to the residue found at the same position in the donor antibody (see Reichmann et al., 1998, Nature, 332, 323-324). Such changes should be kept to the minimum necessary to recover the affinity of the donor antibody. A protocol for selecting residues in the acceptor framework regions which may need to be changed is set forth in WO91/09967.

Donor residues are residues from the donor antibody, i.e. the antibody from which the CDRs were originally derived. Donor residues may be replaced by a suitable residue derived from a human receptor framework (acceptor residues), for example mouse, rat or rabbit residues.

In one embodiment the multi-specific antibodies of the present disclosure are fully human, in particular one or more of the variable domains are fully human.

Fully human molecules are those in which the variable regions and the constant regions (where present) of both the heavy and the light chains are all of human origin, or substantially identical to sequences of human origin, not necessarily from the same antibody. Examples of fully human antibodies may include antibodies produced, for example by the phage display methods described above and antibodies produced by mice in which the murine immunoglobulin variable and optionally the constant region genes have been replaced by their human counterparts eg. as described in general terms in EP0546073 ; US 5,545,806; US5,569,825; US5,625,126; US5,633,425; US5,661,016; US5,770,429; EP 0438474 and EP0463151.

In one embodiment the multi-specific antibody molecules of the disclosure are capable of selectively binding two, three or more different antigens of interest.

In one embodiment, antigens of interest bound by the antigen binding domain formed by VH/VL, or V₁ or V₂ are independently selected from a cell-associated protein, for example a cell surface protein on cells such as bacterial cells, yeast cells, T-cells, endothelial cells or tumour cells, and a soluble protein.

Antigens of interest may also be any medically relevant protein such as those proteins upregulated during disease or infection, for example receptors and/or their corresponding ligands. Particular examples of cell surface proteins include adhesion molecules, for example integrins such as β1 integrins e.g. VLA-4, E-selectin, P selectin or L-selectin, CD2, CD3, CD4, CD5, CD7, CD8, CD11a, CD11b, CD18, CD19, CD20, CD23, CD25, CD33, CD38, CD40, CD45, CDW52, CD69, CD134 (OX40), ICOS, BCMP7, CD137, CD27L, CDCP1, DPCR1, DPCR1, dudulin2, FLJ20584, FLJ40787, HEK2, KIAA0634, KIAA0659, KIAA1246, KIAA1455, LTBP2, LTK, MAL2, MRP2, nectin-like2, NKCC1, PTK7, RAIG1, TCAM1, SC6, BCMP101, BCMP84, BCMP11, DTD, carcinoembryonic antigen (CEA), human milk fat globulin (HMFG1 and 2), MHC Class I and MHC Class II antigens, and VEGF, and where appropriate, receptors thereof.

Soluble antigens include interleukins such as IL-1, IL-2, IL-3, IL-4, IL-5, IL-6, IL-8, IL-12, IL-16, IL-23, viral antigens, for example respiratory syncytial virus or cytomegalovirus antigens, immunoglobulins, such as IgE, interferons such as interferon α, interferon β or interferon γ, colony stimulating factors such as G-CSF or GM-CSF, and platelet derived growth factors such as PDGF-α, and PDGF-β and where appropriate receptors thereof. Other antigens include bacterial cell surface antigens, bacterial toxins, viruses such as influenza, EBV, HepA, B and C, bioterrorism agents, radionuclides and heavy metals, and snake and spider venoms and toxins.

In one embodiment the antibody molecule of the disclosure may be used to functionally alter the activity of the antigen of interest. For example, the antibody molecule may neutralize, antagonize or agonise the activity of said antigen, directly or indirectly.

In one embodiment V₁ is specific for human serum albumin.

In one embodiment V₂ is specific for human serum albumin.

In one embodiment V₁ or V₂ and VH/VL are specific for two different antigens. In one embodiment V₁ or V₂ and VH/VL are specific for the same antigen, for example binding the same or a different epitope therein.

In one embodiment, an antigen of interest bound by VH/VL and/or V₁ or V₂ provides the ability to recruit effector functions, such as complement pathway activation and/or effector cell recruitment.

The recruitment of effector function may be direct in that effector function is associated with a cell, said cell bearing a recruitment molecule on its surface. Indirect recruitment may occur when binding of an antigen to an antigen binding domain (such as V₁ or V₂) in the molecule according to present disclosure to a recruitment polypeptide causes release of, for example, a factor which in turn may directly or indirectly recruit effector function, or may be via activation of a signalling pathway. Examples include IL2, IL6, IL8, IFNγ, histamine, C1q, opsonin and other members of the classical and alternative complement activation cascades, such as C2, C4, C3-convertase, and C5 to C9.

As used herein, "a recruitment polypeptide" includes a FcγR such as FcγRI, FcγRII and FcγRIII, a complement pathway protein such as, but without limitation, C1q and C3, a CD marker protein (Cluster of Differentiation marker) such as, but without limitation, CD68, CD115, CD16, CD80, CD83, CD86, CD56, CD64, CD3, CD4, CD8, CD28, CD45, CD19, CD20 and CD22. Further recruitment polypeptides which are CD marker proteins include CD1, CD1d, CD2, CD5, CD8, CD9, CD10, CD11, CD11a, CD11b, CD11c, CD13, CD14, CD15, CD16, CD18, CD19, CD20, CD21, CD22, CD23, CD24, CD25, CD26, CD27, CD28, CD29, CD30, CD31, CD32, CD33, CD34, CD35, CD36, CD37, CD38, CD40, CD43, CD44, CD45, CD46, CD49, CD49a, CD49b, CD49c, CD49d, CD52, CD53, CD54, CD55, CD56, CD58, CD59, CD61, CD62, D62E, CD62L, CD62P, CD63, CD64, CD66e, CD68, CD70, CD71, CD72, CD79, CD80, CD81, CD82, CD83, CD84, CD85, CD86, CD88, CD89, CD90, CD94, CD95, CD98, CD106, CD114, CD116, CD117, CD118, CD120, CD122, CD130, CD131, CD132, CD133, CD134, CD135, CD137, CD138, CD141, CD142, CD143, CD146, CD147, CD151, CD152, CD153, CD154, CD155, CD162, CD164, CD169, CD184, CD206, CD209, CD257, CD278, CD281, CD282, CD283 and CD304, or a fragment of any thereof which retains the ability to recruit cell-mediated effector function either directly or indirectly. A recruitment polypeptide also includes immunoglobulin molecules such as IgG1, IgG2, IgG3, IgG4, IgE and IgA which possess effector function.

In one embodiment an antigen binding domain (such as V₁ or V₂) in the multi-specific antibody molecule according to the present disclosure has specificity for a complement pathway protein, for example C1q.

Further, multi-specific antibody molecules of the present disclosure may be used to chelate radionuclides by virtue of an antibody or fragment which binds to a nuclide chelator protein. Such fusion proteins are of use in imaging or radionuclide targeting approaches to therapy. See for example, Altai et al Bioconjug Chem 2013 June 19: 24 (6) 1102-9 incorporated herein by reference.

In one embodiment an antigen binding domain in a molecule according to the disclosure (such as V₁ or V₂) has specificity for a CD marker protein, selected from the group comprising CD68, CD80, CD86, CD64, CD3, CD4, CD8 CD45, CD16 and CD35.

In one embodiment an antigen binding domain within a molecule according to the disclosure (such as V₁ or V₂) has specificity for a serum carrier protein, a circulating immunoglobulin molecule, or CD35/CR1, for example for providing an extended half-life to the antibody fragment with specificity for said antigen of interest by binding to said serum carrier protein, circulating immunoglobulin molecule or CD35/CR1.

As used herein, "serum carrier proteins" include thyroxine-binding protein, transthyretin, α1-acid glycoprotein, transferrin, fibrinogen and albumin, or a fragment of any thereof.

As used herein, a "circulating immunoglobulin molecule" includes IgG1, IgG2, IgG3, IgG4, sIgA, IgM and IgD, or a fragment of any thereof.

CD35/CR1 is a protein present on red blood cells which have a half-life of 36 days (normal range of 28 to 47 days; Lanaro et al., 1971, Cancer, 28(3):658-661).

In one embodiment, the antigen of interest for which VH/VL has specificity is a serum carrier protein, such as a human serum carrier, such as albumin, in particular human serum albumin.

In one embodiment, the antigen of interest for which V₁ has specificity is a serum carrier protein, such as a human serum carrier, such as albumin, in particular human serum albumin.

In one embodiment, the antigen of interest for which V₂ has specificity is a serum carrier protein, such as a human serum carrier, such as albumin, in particular human serum albumin.

Generally V₁ or V₂ and VH/VL will not both have specificity for a serum carrier protein.

In one embodiment the multi-specific antibody molecules of the present disclosure are processed to provide improved affinity for a target antigen or antigens. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of E. coli (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996) and sexual PCR (Crameri et al., Nature, 391, 288-291, 1998). Vaughan et al. (supra) discusses these methods of affinity maturation.

Improved affinity as employed herein in this context refers to an improvement over the starting molecule.

If desired an antibody molecule for use in the present disclosure may be conjugated to one or more effector molecule(s). It will be appreciated that the effector molecule may comprise a single effector molecule or two or more such molecules so linked as to form a single moiety that can be attached to the antibodies of the present invention. Where it is desired to obtain an antibody fragment linked to an effector molecule, this may be prepared by standard chemical or recombinant DNA procedures in which the antibody fragment is linked either directly or via a coupling agent to the effector molecule. Techniques for conjugating such effector molecules to antibodies are well known in the art (see, Hellstrom et al., Controlled Drug Delivery, 2nd Ed., Robinson et al., eds., 1987, pp. 623-53; Thorpe et al., 1982, Immunol. Rev., 62:119-58 and Dubowchik et al., 1999, Pharmacology and Therapeutics, 83, 67-123). Particular chemical procedures include, for example, those described in WO93/06231, WO92/22583, WO89/00195, WO89/01476 and WO03/031581. Alternatively, where the effector molecule is a protein or polypeptide the linkage may be achieved using recombinant DNA procedures, for example as described in WO86/01533 and EP0392745.

The term "effector molecule" as used herein includes, for example, biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy.

Other effector molecules may include chelated radionuclides, such as 111In and 90Y, Lu177, Bismuth213, Californium252, Iridium192 and Tungsten188/Rhenium188; or drugs such as but not limited to, alkylphosphocholines, topoisomerase I inhibitors, taxoids and suramin.

Other effector molecules include proteins, peptides and enzymes. Enzymes of interest include, but are not limited to, proteolytic enzymes, hydrolases, lyases, isomerases, transferases. Proteins, polypeptides and peptides of interest include, but are not limited to, immunoglobulins, toxins such as abrin, ricin A, pseudomonas exotoxin, or diphtheria toxin, a protein such as insulin, α-interferon, β-interferon, nerve growth factor, platelet derived growth factor or tissue plasminogen activator, a thrombotic agent or an anti-angiogenic agent, e.g. angiostatin or endostatin, or, a biological response modifier such as a lymphokine, interleukin-1 (IL-1), interleukin-2 (IL-2), granulocyte macrophage colony stimulating factor (GM-CSF), granulocyte colony stimulating factor (G-CSF), nerve growth factor (NGF) or other growth factor and immunoglobulins.

Other effector molecules may include detectable substances useful, for example in diagnosis. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally US 4,741,900 for metal ions which can be conjugated to antibodies for use as diagnostics. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include 1251, 1311, 111In and 99Tc.

In another embodiment the effector molecule may increase the half-life of the antibody *in vivo,* and/or reduce immunogenicity of the antibody and/or enhance the delivery of an antibody across an epithelial barrier to the immune system. Examples of suitable effector molecules of this type include polymers, albumin, albumin binding proteins or albumin binding compounds such as those described in WO05/117984.

Where the effector molecule is a polymer it may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide.

Specific optional substituents which may be present on the above-mentioned synthetic polymers include one or more hydroxy, methyl or methoxy groups.

Specific examples of synthetic polymers include optionally substituted straight or branched chain poly(ethyleneglycol), poly(propyleneglycol) poly(vinylalcohol) or derivatives thereof, especially optionally substituted poly(ethyleneglycol) such as methoxypoly(ethyleneglycol) or derivatives thereof.

Specific naturally occurring polymers include lactose, amylose, dextran, glycogen or derivatives thereof.

"Derivatives" as used herein is intended to include reactive derivatives, for example thiol-selective reactive groups such as maleimides and the like. The reactive group may be linked directly or through a linker segment to the polymer. It will be appreciated that the residue of such a group will in some instances form part of the product as the linking group between the antibody fragment and the polymer.

The size of the polymer may be varied as desired, but will generally be in an average molecular weight range from 500Da to 50000Da, for example from 5000 to 40000Da such as from 20000 to 40000Da. The polymer size may in particular be selected on the basis of the intended use of the product for example ability to localize to certain tissues such as tumors or extend circulating half-life (for review see Chapman, 2002, Advanced Drug Delivery Reviews, 54, 531-545). Thus, for example, where the product is intended to leave the circulation and penetrate tissue, for example for use in the treatment of a tumour, it may be advantageous to use a small molecular weight polymer, for example with a molecular weight of around 5000Da. For applications where the product remains in the circulation, it may be advantageous to use a higher molecular weight polymer, for example having a molecular weight in the range from 20000Da to 40000Da.

Suitable polymers include a polyalkylene polymer, such as a poly(ethyleneglycol) or, especially, a methoxypoly(ethyleneglycol) or a derivative thereof, and especially with a molecular weight in the range from about 15000Da to about 40000Da.

In one embodiment antibodies for use in the present disclosure are attached to poly(ethyleneglycol) (PEG) moieties. In one particular example the antibody is an antibody fragment and the PEG molecules may be attached through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods (see for example US5,219,996; US5,667,425; WO98/25971, WO2008/038024). In one embodiment the antibody molecule of the present invention is a modified Fab fragment wherein the modification is the addition to the C-terminal end of its heavy chain one or more amino acids to allow the attachment of an effector molecule. Suitably, the additional amino acids form a modified hinge region containing one or more cysteine residues to which the effector molecule may be attached. Multiple sites can be used to attach two or more PEG molecules.

Suitably PEG molecules are covalently linked through a thiol group of at least one cysteine residue located in the antibody fragment. Each polymer molecule attached to the modified antibody fragment may be covalently linked to the sulphur atom of a cysteine residue located in the fragment. The covalent linkage will generally be a disulphide bond or, in particular, a sulphur-carbon bond. Where a thiol group is used as the point of attachment appropriately activated effector molecules, for example thiol selective derivatives such as maleimides and cysteine derivatives may be used. An activated polymer may be used as the starting material in the preparation of polymer-modified antibody fragments as described above. The activated polymer may be any polymer containing a thiol reactive group such as an α-halocarboxylic acid or ester, e.g. iodoacetamide, an imide, e.g. maleimide, a vinyl sulphone or a disulphide. Such starting materials may be obtained commercially (for example from Nektar, formerly Shearwater Polymers Inc., Huntsville, AL, USA) or may be prepared from commercially available starting materials using conventional chemical procedures. Particular PEG molecules include 20K methoxy-PEG-amine (obtainable from Nektar, formerly Shearwater; Rapp Polymere; and SunBio) and M-PEG-SPA (obtainable from Nektar, formerly Shearwater).

In one embodiment, a Fab or Fab' in the constructs of the present disclosure is PEGylated, i.e. has PEG (poly(ethyleneglycol)) covalently attached thereto, e.g. according to the method disclosed in EP 0948544 or EP1090037 [see also "Poly(ethyleneglycol) Chemistry, Biotechnical and Biomedical Applications", 1992, J. Milton Harris (ed), Plenum Press, New York, "Poly(ethyleneglycol) Chemistry and Biological Applications", 1997, J. Milton Harris and S. Zalipsky (eds), American Chemical Society, Washington DC and "Bioconjugation Protein Coupling Techniques for the Biomedical Sciences", 1998, M. Aslam and A. Dent, Grove Publishers, New York; Chapman, A. 2002, Advanced Drug Delivery Reviews 2002, 54:531-545]. In one embodiment PEG is attached to a cysteine in the hinge region. In one example, a PEG modified Fab fragment has a maleimide group covalently linked to a single thiol group in a modified hinge region. A lysine residue may be covalently linked to the maleimide group and to each of the amine groups on the lysine residue may be attached a methoxypoly(ethyleneglycol) polymer having a molecular weight of approximately 20,000Da. The total molecular weight of the PEG attached to the Fab fragment may therefore be approximately 40,000Da.

Particular PEG molecules include 2-[3-(N-maleimido)propionamido]ethyl amide of N,N'-bis(methoxypoly(ethylene glycol) MW 20,000) modified lysine, also known as PEG2MAL40K (obtainable from Nektar, formerly Shearwater).

Alternative sources of PEG linkers include NOF who supply GL2-400MA2 (wherein m in the structure below is 5) and GL2-400MA (where m is 2) and n is approximately 450:

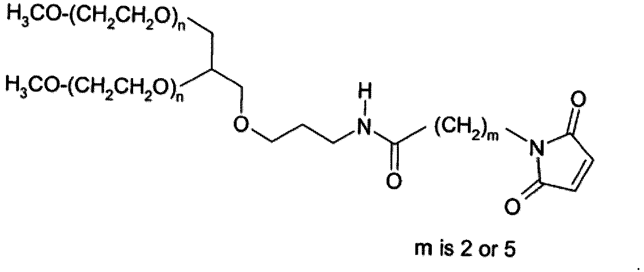

That is to say each PEG is about 20,000Da.

Further alternative PEG effector molecules of the following type: 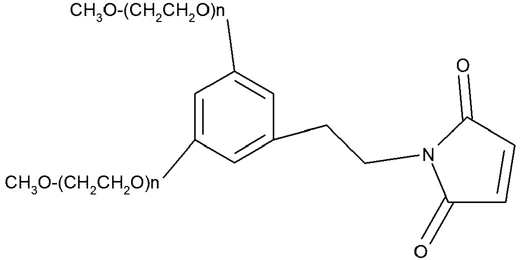 are available from Dr Reddy, NOF and Jenkem.

In one embodiment there is provided an antibody molecule of the present disclosure which is PEGylated (for example with a PEG described herein), attached through a cysteine amino acid residue at or about amino acid 226 in the chain, for example amino acid 226 of the heavy chain (by sequential numbering).

In one embodiment there is provided a polynucleotide sequence encoding a molecule of the present disclosure, such as a DNA sequence. In one embodiment there is provided a DNA sequence encoding a polypeptide chain of formula (I), (Ia) or (Ib). In one embodiment there is a DNA sequence encoding a polypeptide chain of formula (II), (IIa) or (IIb). In one embodiment there is provided a polynucleotide sequence encoding one or more, such as two or more polypeptide components of a molecule of the present disclosure, for example there is provided a DNA sequence encoding a polypeptide of formula (I) and (II), or (Ia) and (IIa) or (Ib) and (IIb).

In one embodiment the polynucleotide, such as the DNA is comprised in a vector.

In one embodiment the vector comprises a DNA sequence encoding a polypeptide of formula (I), (Ia) or (Ib), for example the DNA is not directly joined or connected to a DNA sequence encoding a polypeptide of formula (I), (IIa) and/or (IIb).

In one embodiment the vector comprises a DNA sequence encoding a polypeptide of formula (II), (IIa) or (IIb), for example and the DNA is not directly joined or connected to a DNA sequence encoding a polypeptide of formula (I), (Ia) and/or (Ib).

In one embodiment the vector comprises DNA encoding a heavy and light chain sequence of an antibody construct according to present disclosure, for example on the same or different DNA strand (i.e. connected DNA or separate DNA), such as where the DNA encoding the heavy and light chains are provide on one plasmid or vector where the heavy chain is provided on one plasmid/vector and the light chain is provided on a different plasmid/vector.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

Also provided is a host cell comprising one or more cloning or expression vectors comprising one or more DNA sequences, described herein, encoding an antibody of the present disclosure. Any suitable host cell/vector system may be used for expression of the DNA sequences encoding the antibody molecule of the present invention. Bacterial, for example *E*. *coli,* and other microbial systems may be used or eukaryotic, for example mammalian, host cell expression systems may also be used. Suitable mammalian host cells include CHO, myeloma or hybridoma cells.

The present disclosure also provides a process for the production of an antibody molecule according to the present disclosure comprising culturing a host cell containing a vector of the present invention under conditions suitable for leading to expression of protein from DNA encoding the antibody molecule of the present invention, and isolating the antibody molecule.

For production of products comprising both heavy and light chains, the cell line may be transfected with two vectors, a first vector encoding a light chain polypeptide and a second vector encoding a heavy chain polypeptide. Alternatively, a single vector may be used, the vector including sequences encoding light chain and heavy chain polypeptides, for example as described herein. In one example the cell line may be transfected with three vectors, each encoding a polypeptide chain of an antibody molecule of the present invention.

In one example the cell is transfected with two vectors each one encoding a different polypeptide of the present disclosure, for example provided on the same vector or on different vectors. In one example the cell (cell line) comprises DNA encoding a polypeptide of formula (Ia) and a polypeptide of formula (IIa), for example where DNA encoding each polypeptide are on the same or different vectors. In one example the cell (cell line) comprises DNA encoding a polypeptide of formula (Ib) and a polypeptide of formula (IIb), for example on where DNA encoding each polypeptide are on the same or different vectors.

It will be appreciated that the ratio of each vector transfected into the host cell may be varied in order to optimise expression of the multi-specific antibody product. In one example the ratio of vectors is 1:1. It will be appreciated that skilled person is able to find an optimal ratio by routine testing of protein expression levels following transfection.

It will also be appreciated that where two or more of the polypeptide components are encoded by a polynucleotide in a single vector the relative expression of each polypeptide component can be varied by utilising different promoters for each polynucleotide encoding a polypeptide component of the present disclosure.

The antibodies and fragments according to the present disclosure are expressed at good levels from host cells. Thus the properties of the antibodies and/or fragments appear to be conducive to commercial processing.

The antibodies of the present disclosure are useful in the treatment and/or prophylaxis, for example of a pathological condition.

The present disclosure also provides a pharmaceutical or diagnostic composition comprising an antibody molecule of the present disclosure in combination with one or more of a pharmaceutically acceptable excipient, diluent or carrier. Accordingly, provided is the use of an antibody of the present disclosure or a composition comprising the same for use in treatment and for the manufacture of a medicament.

The composition will usually be supplied as part of a sterile, pharmaceutical composition that will normally include a pharmaceutically acceptable carrier.

The present disclosure also provides a process for preparation of a pharmaceutical or diagnostic composition comprising adding and mixing the antibody molecule of the present disclosure together with one or more of a pharmaceutically acceptable excipient, diluent or carrier.

The antibody molecule may be the sole active ingredient in the pharmaceutical or diagnostic composition or may be accompanied by other active ingredients including other antibody ingredients, for example anti- IL-1β, anti-T cell, anti-IFNy or anti-LPS antibodies, or non-antibody ingredients such as xanthines. Other suitable active ingredients include antibodies capable of inducing tolerance, for example, anti-CD3 or anti-CD4 antibodies.

In a further embodiment the antibody molecule or composition according to the disclosure is employed in combination with a further pharmaceutically active agent, for example a corticosteroid (such as fluticasonoe propionate) and/or a beta-2-agonist (such as salbutamol, salmeterol or formoterol) or inhibitors of cell growth and proliferation (such as rapamycin, cyclophosphmide, methotrexate) or alternatively a CD28 and /or CD40 inhibitor. In one embodiment the inhibitor is a small molecule. In another embodiment the inhibitor is a biological therapeutic or diagnostic, for example an antibody specific to the target.

The pharmaceutical compositions suitably comprise a therapeutically effective amount of the antibody of the invention. The term "therapeutically effective amount" as used herein refers to an amount of a therapeutic agent needed to treat, ameliorate or prevent a targeted disease or condition, or to exhibit a detectable therapeutic or preventative effect. For any antibody, the therapeutically effective amount can be estimated initially either in cell culture assays or in animal models, usually in rodents, rabbits, dogs, pigs or primates. The animal model may also be used to determine the appropriate concentration range and route of administration. Such information can then be used to determine useful doses and routes for administration in humans.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgement of the clinician. Generally, a therapeutically effective amount will be from 0.01 mg/kg to 50 mg/kg, for example 0.1 mg/kg to 20 mg/kg. Alternatively, the dose may be 1 to 500mg per day such as 10 to 100, 200, 300 or 400mg per day. Pharmaceutical compositions may be conveniently presented in unit dose forms containing a predetermined amount of an active agent of the invention.

Compositions may be administered individually to a patient or may be administered in combination (e.g. simultaneously, sequentially or separately) with other agents, drugs or hormones.

In one embodiment the antibodies or compositions of the present disclosure are employed in a combination therapy, for example a combination therapy to treat cancer, in particular where the therapy comprises administering a chemotherapeutic agent and/or administering radiotherapy.

The dose at which the antibody molecule of the present disclosure is administered depends on the nature of the condition to be treated, the extent of the inflammation present and on whether the antibody molecule is being used prophylactically or to treat an existing condition.

The frequency of dose will depend on the half-life of the antibody molecule and the duration of its effect. If the antibody molecule has a short half-life (e.g. 2 to 10 hours) it may be necessary to give one or more doses per day. Alternatively, if the antibody molecule has a long half-life (e.g. 2 to 15 days) it may only be necessary to give a dosage once per day, once per week or even once every 1 or 2 months.

The pharmaceutically acceptable carrier should not itself induce the production of antibodies harmful to the individual receiving the composition and should not be toxic. Suitable carriers may be large, slowly metabolised macromolecules such as proteins, polypeptides, liposomes, polysaccharides, polylactic acids, polyglycolic acids, polymeric amino acids, amino acid copolymers and inactive virus particles.

Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in therapeutic compositions may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the patient.

Suitable forms for administration include forms suitable for parenteral administration, e.g. by injection or infusion, for example by bolus injection or continuous infusion. Where the product is for injection or infusion, it may take the form of a suspension, solution or emulsion in an oily or aqueous vehicle and it may contain formulatory agents, such as suspending, preservative, stabilising and/or dispersing agents. Alternatively, the antibody molecule may be in dry form, for reconstitution before use with an appropriate sterile liquid.

Once formulated, the compositions of the invention can be administered directly to the subject. The subjects to be treated can be animals. However, in one or more embodiments the compositions are adapted for administration to human subjects.

In one embodiment, in formulations according to the present disclosure, the pH of the final formulation is not similar to the value of the isoelectric point of the antibody or fragment, for if the pH of the formulation is 7 then a pI of from 8-9 or above may be appropriate. Whilst not wishing to be bound by theory it is thought that this may ultimately provide a final formulation with improved stability, for example the antibody or fragment remains in solution.

The pharmaceutical compositions of this disclosure may be administered by any number of routes including, but not limited to, oral, intravenous, intramuscular, intra-arterial, intramedullary, intrathecal, intraventricular, transdermal, transcutaneous (for example, see WO98/20734), subcutaneous, intraperitoneal, intranasal, enteral, topical, sublingual, intravaginal or rectal routes. Hyposprays may also be used to administer the pharmaceutical compositions of the invention. Typically, the therapeutic compositions may be prepared as injectables, either as liquid solutions or suspensions. Solid forms suitable for solution in, or suspension in, liquid vehicles prior to injection may also be prepared. In one embodiment the antibody molecules of the present invention are administered subcutaneously.

In one embodiment an antibody or composition according to the present disclosure is provided pre-filled into a syringe, for example in a format suitable for self-administration.

Direct delivery of the compositions will generally be accomplished by injection, subcutaneously, intraperitoneally, intravenously or intramuscularly, or delivered to the interstitial space of a tissue. The compositions can also be administered into a specific tissue of interest. Dosage treatment may be a single dose schedule or a multiple dose schedule.

It will be appreciated that the active ingredient in the composition will be an antibody molecule of the present disclosure. As such, it will be susceptible to degradation in the gastrointestinal tract. Thus, if the composition is to be administered by a route using the gastrointestinal tract, the composition will need to contain agents which protect the antibody from degradation but which release the antibody once it has been absorbed from the gastrointestinal tract.

A thorough discussion of pharmaceutically acceptable carriers is available in Remington's Pharmaceutical Sciences (Mack Publishing Company, N.J. 1991).

In one embodiment the formulation is provided as a formulation for topical administrations including inhalation.

Suitable inhalable preparations include inhalable powders, metering aerosols containing propellant gases or inhalable solutions free from propellant gases (such as nebulisable solutions or suspensions). Inhalable powders according to the disclosure containing the active substance may consist solely of the abovementioned active substances or of a mixture of the above mentioned active substances with physiologically acceptable excipient.

These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

Particles for deposition in the lung require a particle size less than 10 microns, such as 1-9 microns for example from 0.1 to 5 µm, in particular from 1 to 5 µm. The particle size of the active agent (such as the antibody or antibody fragment) is of primary importance.

The propellent gases which can be used to prepare the inhalable aerosols are known in the art. Suitable propellent gases are selected from among hydrocarbons such as n-propane, n-butane or isobutane and halohydrocarbons such as chlorinated and/or fluorinated derivatives of methane, ethane, propane, butane, cyclopropane or cyclobutane. The abovementioned propellent gases may be used on their own or in mixtures thereof.

Particularly suitable propellent gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable.

The propellent-gas-containing inhalable aerosols may also contain other ingredients such as cosolvents, stabilisers, surface-active agents (surfactants), antioxidants, lubricants and means for adjusting the pH. All these ingredients are known in the art.

The propellant-gas-containing inhalable aerosols according to the invention may contain up to 5 % by weight of active substance. Aerosols according to the invention contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active.

Alternatively, topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

In one embodiment the formulation is provided as discrete ampoules containing a unit dose for delivery by nebulisation.

In one embodiment the antibody is supplied in lyophilised form, for reconstitutions or alternatively as a suspension formulation.

The antibody of the present disclosure can be delivered dispersed in a solvent, e.g., in the form of a solution or a suspension. It can be suspended in an appropriate physiological solution, e.g., physiological saline, a pharmacologically acceptable solvent or a buffered solution. Buffered solutions known in the art may contain 0.05 mg to 0.15 mg disodium edetate, 8.0 mg to 9.0 mg NaCl, 0.15 mg to 0.25 mg polysorbate, 0.25 mg to 0.30 mg anhydrous citric acid, and 0.45 mg to 0.55 mg sodium citrate per 1 ml of water so as to achieve a pH of about 4.0 to 5.0. As mentioned supra a suspension can made, for example, from lyophilised antibody.

The therapeutic suspensions or solution formulations can also contain one or more excipients. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. The formulation will generally be provided in a substantially sterile form employing sterile manufacture processes.

This may include production and sterilization by filtration of the buffered solvent solution used for the formulation, aseptic suspension of the antibody in the sterile buffered solvent solution, and dispensing of the formulation into sterile receptacles by methods familiar to those of ordinary skill in the art.

Nebulisable formulation according to the present disclosure may be provided, for example, as single dose units (e.g., sealed plastic containers or vials) packed in foil envelopes. Each vial contains a unit dose in a volume, e.g., 2 ml, of solvent/solution buffer.

The antibodies of the present disclosure are thought to be suitable for delivery via nebulisation.

Also provided is a pharmaceutical composition comprising a multispecific antibody molecule according to the present disclosure and an excipient, diluent or carrier.

The disclosure also extends to a multispecific antibody molecule or a pharmaceutical composition according to the present disclosure for use in treatment, in particular, for use in the treatment of a condition or disorder selected from the group consisting of infections (viral, bacterial, fungal and parasitic), endotoxic shock associated with infection, arthritis such as rheumatoid arthritis, asthma such as severe asthma, chronic obstructive pulmonary disease (COPD), pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, meningoencephalitis, autoimmune uveitis, immune mediated inflammatory disorders of the central and peripheral nervous system such as multiple sclerosis, lupus (such as systemic lupus erythematosus) and Guillain-Barr syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart disease including ischaemic diseases such as myocardial infarction as well as atherosclerosis, intravascular coagulation, bone resorption, osteoporosis, osteoarthritis, periodontitis, hypochlorhydia and cancer, for example an epithelial cancer.

As used herein, the term "comprising" in context of the present specification should be interpreted as "including".

Embodiments and preferences may be combined as technically appropriate.

The disclosure herein describes embodiments comprising certain integers. The disclosure also extends to the same embodiments consisting or consisting essentially of said integers.

### EXAMPLES

### Example 1 Analyses of 645 variants where 645 CDRs are grafted onto 497 or 2109 frameworks in a Fab and Fab-dsscFv

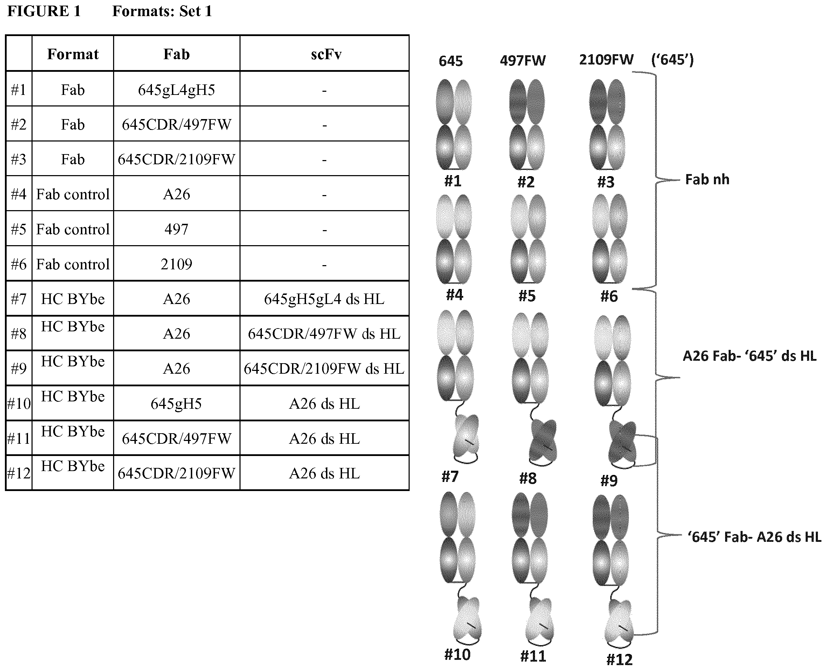

Fabs were designed as Fab no hinge (Fab) formats and all Fab-dsscFv antibodies (also referred to as BYbes) were constructed as HC BYbes where the Fab heavy chain (HC) is linked to a di-sulphide stabilised scFv (ds HL i.e. VH-linker-VL, linker = GGGGSGGGGSGGGGSGGGGS SEQ ID NO: 97) via a G4S -based linker (SGGGGSGGGGS SEQ ID NO: 43). HC BYbes were constructed using the parent 645 graft (gL4gH5) and its 645 variants where 645 CDRs were grafted onto 497 or 2109 frameworks. The 645 variants were either used in the scFv position as a ds HL, or in the Fab position with A26 as the di-sulphide stabilised scFv. The antibodies shown in Figure 1 were constructed wherein FW means framework.

### Methods

### Transient expiHEK Expression

Light chain and heavy chain plasmids in a ratio of 1:1, were transfected into ExpiHEK cells by the expifectamine method at 50 ml scale in duplicate, as recommended by the manufacturer (Life Technologies). Transfections were incubated at 37°C, with shaking at 140 rpm and at 8 % CO₂. After 7 days, the supernatants were harvested, filtered through a 0.22 µm filter and expression yields were determined by Protein G HPLC using a proprietary Fab as a standard

### Protein Purification

Supernatants were concentrated 40-fold using Centricon concentrators with a MWCO 10 kDa and subsequently loaded on a HiTrap Protein G column (GE Healthcare). The column was washed with PBS pH 7.4 followed by elution of bound material with 0.1 M glycine pH 2.7. The eluate was neutralized with 2 M Tris-HCl (pH 8.5) prior to buffer exchange into PBS pH 7.4. The eluted proteins were quantified by absorbance at 280 nm and diluted to 1 mg/ml in PBS pH 7.4 and stored at 4 °C for at least 16 h before further analyses.

### Size exclusion (SE HPLC)

For G3000 SE HPLC, purified protein samples (~20 µg) were loaded on to a TSKgel G3000SW, 10 µm, 7.5 mm ID × 300 mm column (Tosoh) and developed with an isocratic gradient of 0.2 M phosphate pH 7 at 1 mL/min. Continuous detection -by absorbance at 280 nm.

### SDS-PAGE

Purified protein (2 µg) samples were prepared for non-reducing and reducing Novex 4-20 % acrylamide Tris/glycine SDS-polyacrylamide gel separation according to the manufacturers recommendations (Life Technologies). For non-reducing gels, 100 mM of N-ethyl maleimide was also added to the samples. Samples were loaded onto gels and separated at a constant voltage of 125 V for ~ 200 min in Tris/glycine running buffer. SeeBlue2 (Life Technologies) protein ladders were used as the standard. The gels were stained with Instant Blue (Expedeon) and destained with several changes of distilled water.

### Thermal stability

A thermofluor assay was used to monitor the thermal stability of purified proteins. The reaction mix contained 5 µl of 30× SYPRO^{®} Orange dye, diluted with water from 5000x stock solution and 45 µl of the purified protein sample at 0.11 mg/mL in PBS pH 7.4. Aliquots (10 µl) of the mixture were dispensed in quadruplicate into a 384 PCR optical well plate and were run on a 7900HT Fast Real-Time PCR System (Agilent). The peltier-based thermal cycling system was set at 20°C to 99°C with a ramp rate of 1.1°C/min. A charge-coupled device (CCD) monitored the fluorescence changes in the wells. The intensity increases in fluorescence were plotted and the inflection point of the slope(s) was used to generate the thermostability transition midpoint (Tₘ).

### Surface plasmon Resonance/ dual affinity assay

The binding affinities and kinetic parameters for the interactions of antibodies were determined by surface plasmon resonance (SPR) conducted on a Biacore T100 or a Biacore 3000 using CM5 sensor chips (GE Healthcare Bio-Sciences AB) and HBS-EP (10mM HEPES (pH7.4), 150 mM NaCl, 3 mM EDTA, 0.05 % v/v surfactant P20) running buffer. All experiments were performed at 25°C. The antibody samples were captured to the sensor chip surface using either a human F(ab')₂-specific goat Fab (Jackson ImmunoResearch) or an in-house generated anti human CH1 monoclonal antibody. Covalent immobilisation of the capture antibody was achieved by standard amine coupling chemistry to a level of 6000-7000 response units (RU).

Human albumin, ChromPure (Jackson ImmunoResearch) was titrated over the captured antibody from 50 nM. Each assay cycle consisted of firstly capturing the antibody sample using a 1 min injection, before an association phase consisting of a 3 min injection of antigen (Human albumin), after which dissociation was monitored for 10 min. After each cycle, the capture surface was regenerated. The flow rates used were 10 µl/min for capture, 30 µl/min for association and dissociation phases, and 10 µl/min for regeneration. The potential for the bispecific antibody to bind simultaneously to both human serum albumin and the T-cell receptor ligand (ligand antigen) was assessed by capturing the ligand (bispecific antibody) to the sensor chip surface, before performing either separate 3 min injections of 5 µM human serum albumin: 50 nM ligand antigen or a mixed solution of both 5 µM human serum albumin and 50 nM ligand antigen.

For kinetic assays, a titration of antigen (for human serum albumin typically 62.5 nM-2 µM) was performed, a blank flow-cell was used for reference subtraction and buffer-blank injections were included to subtract instrument noise and drift.

Kinetic parameters were determined by simultaneous global-fitting of the resulting sensorgrams to a standard 1:1 binding model using Biacore T100 evaluation software v2.0.1 or Biacore 3000 Biaevaluation v3.2.

### Results

### Transient Expression of Fabs and BYbes

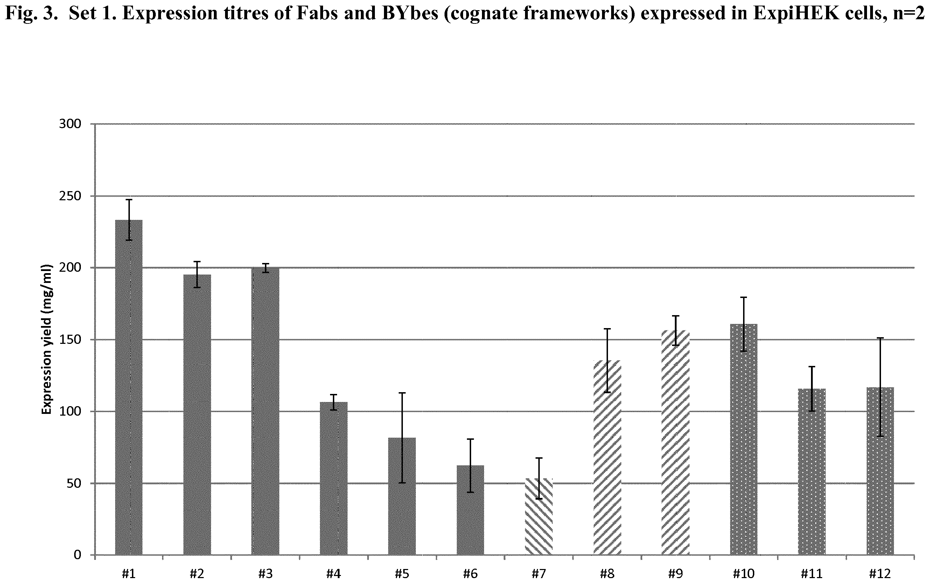

Figure 3 Expression yields of Fabs with 497 or 2109 frameworks (Fig.3, #2- 3) were comparable to the parent 645 Fab (Fig. 1, #1). As HC BYbes with 645 as a di-sulphide linked HL scFv, a two-fold increase in expression was observed when frameworks of the parent 645 graft were substituted for 497 or 2109 frameworks (Fig. 1, #7-9). When 645 was placed in the Fab position in a HC BYbe format, no significant change in expression was observed between the parent 645 and the variant BYbes with 497 or 2109 frameworks (Fig. 3, #10-12).

### Biophysical Analysis of Fabs and BYbes

### Size Exclusion HPLC

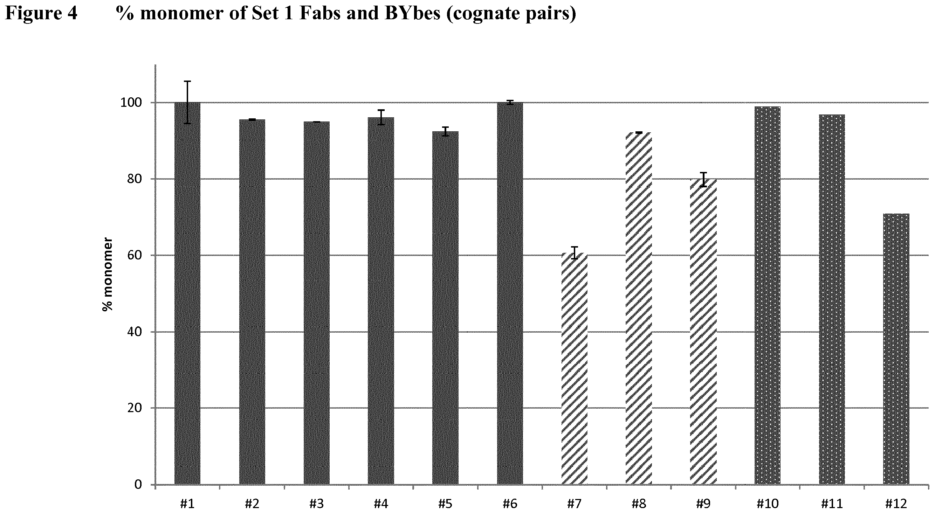

Figure 4 All Fabs were highly monomeric, irrespective of framework isotype or CDRs (Fig. 4. #1-6). As HC BYbes with 645 in the scFv (ds HL) position, the parent 645 BYbe (Fig.4, #7), was 59 % monomeric, whereas where 645 CDRs were grafted onto 2109 frameworks (Fig. 4., #9) or 497 frameworks (Fig. 4, # 8), % monomer increased to 80 % and 93 %, respectively confirming that framework residues impact multimerisation significantly. On the other hand, with 645 in the Fab position of the HC BYbe format, 497 frameworks had no effect and both parent 645 and this variant were highly monomeric (Fig. 4, #10-11). With 2019 frameworks (Fig. 4, #12), framework residues appear to impact multimerisation even though as a Fab, this combination of CDRs and frameworks, and the parent 2109 (Fig. 4, #3, #6), are both highly monomeric.

### SDS-PAGE

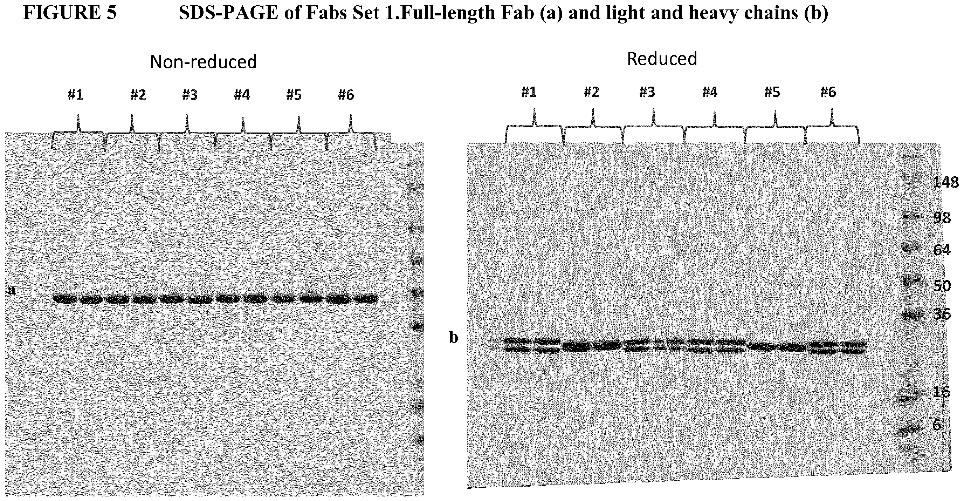

Figure 5 On non-reducing and reducing SDS-PAGE, full-length Fabs (Fig. 5, a) and light and heavy chains (Fig. 5, b) migrated to their predicted Mr of ~49 kDa and ~24- 26 kDa, respectively (Fig. 5, #1-6).

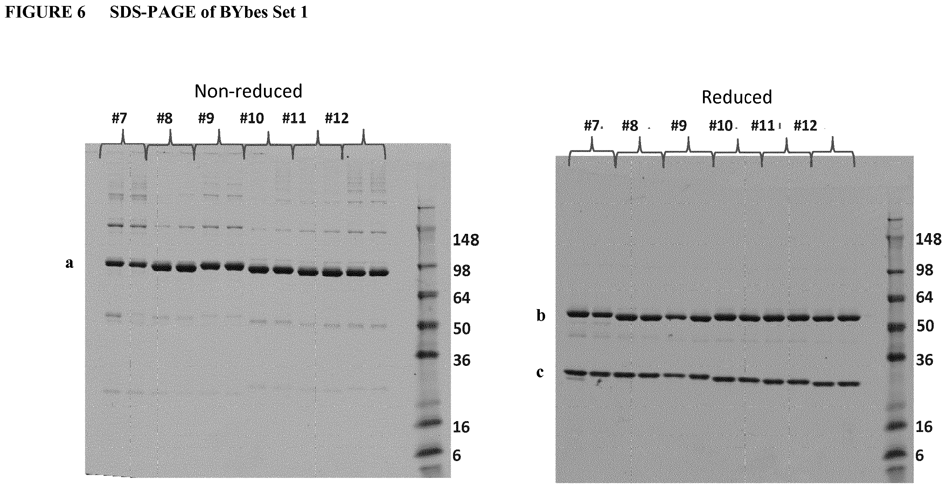

Figure 6 By non-reducing SDS-PAGE, full-length BYbe product (Fig. 6, a) was observed to migrate to - 89 kDa, higher than its predicted Mr (~79 kDa). Minor bands representing multimeric species and free light and heavy chain, can be observed above and below this band in all lanes, respectively. On the reducing gel, heavy (Fig. 6, b) and light chains (Fig. 6, c) migrated to their predicted molecular weights.

**Thermal Stability of Fabs and BYbes Table 7:**

| **Protein** | **Sample** | **Tm1** | **Tm2** |
|---|---|---|---|
| 645gL4gH5 Fab nh | #1 | 83.1 | ND |
| 645CDR -497FW/645CDR Fab nh | #2 | 80.2 | ND |
| 645CDR -2109FW Fab nh | #3 | 79.2 | ND |
| A26 Fab nh | #4 | 82.4 | ND |
| 497 Fab nh | #5 | 71.4 | ND |
| 2109 Fab nh | #6 | 70.8 | ND |
| A26 Fab-645 ds HL | #7 | 84.5 | 74.0 |
| A26 Fab-645CDR-497FW ds HL | #8 | 70.5 | ND |
| A26 Fab-645CDR-2109FW ds HL | #9 | 66.5 | ND |
| 645 Fab-A26 ds HL | #10 | 81.9 | ND |
| 645CDR-497FW Fab-645 ds HL | #11 | 73.5 | ND |
| 645CDR-2109FW Fab-645 ds HL | #12 | 83.4 | 69.5 |

The Tm of parent 645, 497 and 2109 Fabs were 83.1°C, 71.4°C and 70.8°C, respectively (Table 9, #1, #5-6). Grafting of 645 CDRs onto 497 or 2019 frameworks resulted in Tms of 80.2°C and 79.2°C, respectively confirming the importance of CDR residues in determining thermostability. However, as HC BYbes where the 645 variant was a di-sulphide stabilised scFv linked to A26 Fab heavy chain, the Tm of 645CDRs/497FW and 645CDRs/2109FW was significantly reduced by 15 and 18°C, respectively (Table 9, #7-9). When the 645 variant was placed in the Fab position of the HC BYbe, Tms were not as strongly affected where 645CDR/2109FW appears to be as stable as the parent 645 (Table 9, #10-12).

### Affinity to Human Serum Albumin (HSA)

**Table 8 -A Set 1 Affinity to HSA**

| | **Format** | **Analyte** | **ka (1/Ms)** | **kd (1/s)** | **KD (M)** |
|---|---|---|---|---|---|
| #1 | 645 Fab nh | HSA | 1.46 × 10⁵ | 1.96×10⁻⁴ | 1.34 ×10-9 |
| #2 | 645CDR-497FW Fab nh | HSA | 1.30 × 10⁵ | 6.01×10⁻⁴ | 4.63 ×10-9 |
| #3 | 645CDR-2109FW Fab nh | HSA | 1.50 × 10⁵ | 2.27×10⁻⁴ | 1.52 ×10-9 |
| #7 | A26 Fab -645gH5gL4 dsHL BYBe (wt) | HSA | 1.50 × 10⁵ | 1.92×10⁻⁴ | 1.28 ×10-9 |
| #8 | A26- Fab-645CDR-497FW ds HL BYbe | HSA | 1.35 × 10⁵ | 4.77×10⁻⁴ | 3.53 x10-9 |
| #9 | A26- Fab-645CDR-2109FW ds HL BYbe | HSA | 1.41 × 10⁵ | 6.79×10⁻⁴ | 4.81 ×10-9 |
| #10 | 645gL4gH5 Fab-A26 BYbe wt | HSA | 1.56 × 10⁵ | 2.50×10⁻⁴ | 1.61 ×10-9 |
| #11 | 645CDR-497FW Fab-A26 ds HL BYbe | HSA | 1.51 × 10⁵ | 1.86×10⁻⁴ | 1.23 ×10-9 |
| #12 | 645CDR-2109FW Fab-A26 ds HL BYbe | HSA | 1.37 × 10⁵ | 6.51×10⁻⁴ | 4.74 x10-9 |

In terms of order of magnitude, the on- and off- rates, and affinity of the formats to HSA in combinations tested is comparable to the parent formats.

**Summary Table 9**

| | **Protein** | **Expression yield (mg/L)** | **% monomer** | **Thermal stability** | | **Affinity to HSA (KD (M))** |
|---|---|---|---|---|---|---|
| | | | | **Tm1** | **Tm2** | |
| #1 | 645 Fab nh | 233.5 | 100.0 | 83.1 | ND | 1.34 ×10⁻⁹ |
| #2 | 497FW/645CDR Fab nh | 195.2 | 95.5 | 80.2 | ND | 4.63 ×10⁻⁹ |
| #3 | 2109FW/645CDR Fab nh | 199.8 | 95.0 | 79.2 | ND | 1.52 ×10⁻⁹ |
| #4 | A26 Fab nh | 106.5 | 96.1 | 82.4 | ND | nd |
| #5 | 497 Fab nh | 81.8 | 92.4 | 71.4 | ND | nd |
| #6 | 2109 Fab nh | 62.4 | 100.0 | 70.8 | ND | nd |
| #7 | A26 -645 BYBe wt | 53.6 | 60.6 | 84.5 | 74.0 | 1.28 ×10⁻⁹ |
| #8 | A26-497FW/645CDR BYbe | 135.5 | 92.1 | 70.5 | ND | 3.53 ×10⁻⁹ |
| #9 | A26-2109FW/645CDR BYbe | 156.5 | 79.9 | 66.5 | ND | 4.81 ×10⁻⁹ |
| #10 | 645-A26 BYbe wt | 160.9 | 98.9 | 81.9 | ND | 1.61 ×10⁻⁹ |
| #11 | 497FW/645CDR-A26 BYbe | 115.9 | 96.8 | 73.5 | ND | 1.23 ×10⁻⁹ |
| #12 | 2109FW/645CDR-A26 BYbe | 116.9 | 71.0 | 83.4 | 69.5 | 4.74 ×10⁻⁹ |

| | | | | | | |
|---|---|---|---|---|---|---|
| ND=not detected; nd=not done | | | | | | |

## Claims

1. A multispecific antibody molecule comprising at least two binding domains each with a variable heavy region VH and a variable light region VL, wherein each binding domain is specific for an antigen and comprises 6 CDRs, **characterised in that** at least one binding domain comprises:
a. a variable heavy region, VH_{A}, having a framework comprising SEQ ID NO: 1 for FR1, SEQ ID NO: 3 for FR2, SEQ ID NO: 4 for FR3 and SEQ ID NO: 5 for FR4, with the proviso that CDRH3 in VH_{A} is other than RYYSAMPFAY (SEQ ID NO: 129) and a variable light region, VL_{A}, having a framework comprising SEQ ID NO: 6 for FR1, SEQ ID NO: 7 for FR2, SEQ ID NO: 8 for FR3, and SEQ ID NO: 10 for FR4; or
b. a variable heavy region, VH_{A}, having a framework comprising SEQ ID NO:16 for FR1, SEQ ID NO: 18 for FR2, SEQ ID NO: 19 for FR3 and SEQ ID NO: 20 for FR4, with the proviso that CDRH3 in VH_{A} is other than RYYSAMPFAY (SEQ ID NO: 129) and a variable light region, VL_{A}, having a framework comprising SEQ ID NO: 11 for FR1, SEQ ID NO: 12 for FR2, SEQ ID NO: 13 for FR3, and SEQ ID NO: 15 for FR4;
and wherein the at least one variable region VH_{A} and the at least one variable region VL_{A} are present in a dsscFv, and wherein the multispecific antibody molecule is a Fab-dsscFv or Fab-(dsscFv)₂.

2. A multispecific antibody molecule according to claim 1, wherein VL_{A} comprises SEQ ID NO:99 and VH_{A} comprises SEQ ID NO:101.

3. A multispecific antibody molecule according to claim 2, wherein one binding domain comprises SEQ ID NO:106.

4. A multispecific antibody molecule according to claim 1, wherein VL_{A} comprises SEQ ID NO:103 and VH_{A} comprises SEQ ID NO:105.

5. A multispecific antibody molecule according to claim 4, wherein one binding domain comprises SEQ ID NO:111.

6. A multispecific antibody molecule according to any of claims 1 to 5, wherein the VH_{A} comprises 3 heavy chain CDRs having the sequence given in SEQ ID NO: 143 for CDRH1, SEQ ID NO: 144 for CDRH2 and SEQ ID NO: 145 for CDRH3.

7. A multispecific antibody molecule according to any of claims 1 to 6, wherein the VL_{A} comprises 3 light chain CDRs having the sequence given in SEQ ID NO:146 for CDRL1, SEQ ID NO:147 for CDRL2 and SEQ ID NO:148 for CDRL3.

8. A multispecific antibody molecule according to any preceding claim, wherein the CDRs have a non-human origin.

9. A multispecific antibody molecule according to any preceding claim, wherein the antibody molecule is bispecific or trispecific, such as bispecific.

10. An acceptor framework for minimising aggregation in a multispecific antibody comprising SEQ ID NO: 22 and 24, wherein XXX in the each of the CDR positions independently represent 1 to 35 amino acids, and wherein SEQ ID NO: 22 and 24 are present in a dsscFv, and wherein the multispecific antibody is a Fab-dsscFv or Fab-(dsscFv)₂.

11. A method of improving the levels of monomer expression of an antibody molecule having at least one binding domain with a variable heavy region and variable light region where each variable region has a framework and 3 CDRs, said method comprising the step of changing a framework of a variable region to a framework selected from the group comprising or consisting of:
i) a framework (such a VH region framework) comprising SEQ ID NO: 1 for FR1, SEQ ID NO: 3 for FR2, SEQ ID NO: 4 for FR3 and SEQ ID NO: 5 for FR4,
ii) a framework (such a VH region framework) comprising SEQ ID NO: 16 for FR1, SEQ ID NO: 18 for FR2, SEQ ID NO: 19 for FR3 and SEQ ID NO: 20 for FR4,
iii) a framework (such a VL region framework) comprising SEQ ID NO: 6 for FR1, SEQ ID NO: 7 for FR2, SEQ ID NO: 8 for FR3, and SEQ ID NO: 10 for FR4.
iv) a framework (such a VL region framework) comprising VL1, SEQ ID NO: 11 for FR1, SEQ ID NO: 12 for FR2, SEQ ID NO: 13 for FR3, and SEQ ID NO: 15 for FR4,
v) combinations thereof and,
wherein the framework i), ii), iii), iv), or combinations thereof are present in a dsscFv, and wherein the antibody molecule is a Fab-dsscFv or Fab-(dsscFv)₂.

## Patentansprüche

1. Multispezifisches Antikörpermolekül, umfassend wenigstens zwei Bindungsdomänen mit jeweils einer variablen schweren Region VH und einer variablen leichten Region VL, wobei jede Bindungsdomäne spezifisch für ein Antigen ist und 6 CDR umfasst, **dadurch gekennzeichnet, dass** wenigstens eine Bindungsdomäne Folgendes umfasst:
a. eine variable schwere Region, VH_{A}, mit einem Gerüst, das SEQ ID NO: 1 für FR1, SEQ ID NO: 3 für FR2, SEQ ID NO: 4 für FR3 und SEQ ID NO: 5 für FR4 umfasst, mit der Maßgabe, dass CDRH3 in VH_{A} von RYYSAMPFAY (SEQ ID NO: 129) verschieden ist, und eine variable leichte Region, VL_{A}, mit einem Gerüst, das SEQ ID NO: 6 für FR1, SEQ ID NO: 7 für FR2, SEQ ID NO: 8 für FR3 und SEQ ID NO: 10 für FR4 umfasst; oder
b. eine variable schwere Region, VH_{A}, mit einem Gerüst, das SEQ ID NO: 16 für FR1, SEQ ID NO: 18 für FR2, SEQ ID NO: 19 für FR3 und SEQ ID NO: 20 für FR4 umfasst, mit der Maßgabe, dass CDRH3 in VH_{A} von RYYSAMPFAY (SEQ ID NO: 129) verschieden ist, und eine variable leichte Region, VL_{A}, mit einem Gerüst, das SEQ ID NO: 11 für FR1, SEQ ID NO: 12 für FR2, SEQ ID NO: 13 für FR3 und SEQ ID NO: 15 für FR4 umfasst;
und wobei die wenigstens eine variable Region VH_{A} und die wenigstens eine variable Region VL_{A} in einem dsscFv vorliegen und wobei es sich bei dem multispezifischen Antikörpermolekül um ein Fab-dsscFv oder Fab-(dsscFv)₂ handelt.

2. Multispezifisches Antikörpermolekül nach Anspruch 1, wobei VL_{A} SEQ ID NO:99 und VH_{A} SEQ ID NO:101 umfasst.

3. Multispezifisches Antikörpermolekül nach Anspruch 2, wobei eine Bindungsdomäne SEQ ID NO:106 umfasst.

4. Multispezifisches Antikörpermolekül nach Anspruch 1, wobei VL_{A} SEQ ID NO:103 und VH_{A} SEQ ID NO:105 umfasst.

5. Multispezifisches Antikörpermolekül nach Anspruch 4, wobei eine Bindungsdomäne SEQ ID NO:111 umfasst.

6. Multispezifisches Antikörpermolekül nach einem der Ansprüche 1 bis 5, wobei die VH_{A} 3 Schwere-Kette-CDR mit der unter SEQ ID NO: 143 für CDRH1, SEQ ID NO: 144 für CDRH2 und SEQ ID NO: 145 für CDRH3 angegebenen Sequenz umfasst.

7. Multispezifisches Antikörpermolekül nach einem der Ansprüche 1 bis 6, wobei die VL_{A} 3 Leichte-Kette-CDR mit der unter SEQ ID NO: 146 für CDRL1, SEQ ID NO: 147 für CDRL2 und SEQ ID NO: 148 für CDRL3 angegebenen Sequenz umfasst.

8. Multispezifisches Antikörpermolekül nach einem vorhergehenden Anspruch, wobei die CDR nichtmenschlichen Ursprungs sind.

9. Multispezifisches Antikörpermolekül nach einem vorhergehenden Anspruch, wobei das Antikörpermolekül bispezifisch oder trispezifisch, beispielsweise bispezifisch ist.

10. Akzeptorgerüst zur Minimierung der Aggregation bei einem multispezifischen Antikörper, umfassend SEQ ID NO: 22 und 24, wobei XXX in den einzelnen CDR-Positionen jeweils unabhängig für 1 bis 35 Aminosäuren steht und wobei SEQ ID NO: 22 und 24 in einem dsscFv vorliegen und wobei es sich bei dem multispezifischen Antikörper um ein Fab-dsscFv oder Fab-(dsscFv)₂ handelt.

11. Verfahren zur Verbesserung der Monomerexpressionsniveaus eines Antikörpermoleküls mit wenigstens einer Bindungsdomäne mit einer variable schweren Region und variablen leichten Region, wobei jede variable Region ein Gerüst und 3 CDR aufweist, wobei das Verfahren den Schritt umfasst, bei dem ein Gerüst einer variablen Region zu einem Gerüst geändert wird, das ausgewählt ist aus der Gruppe umfassend bzw. bestehend aus:
i) ein bzw. einem Gerüst (so ein bzw. einem VH-Region-Gerüst), das SEQ ID NO: 1 für FR1, SEQ ID NO: 3 für FR2, SEQ ID NO: 4 für FR3 und SEQ ID NO: 5 für FR4 umfasst,
ii) ein bzw. einem Gerüst (so ein bzw. einem VH-Region-Gerüst), das SEQ ID NO: 16 für FR1, SEQ ID NO: 18 für FR2, SEQ ID NO: 19 für FR3 und SEQ ID NO: 20 für FR4 umfasst,
iii) ein bzw. einem Gerüst (so ein bzw. einem VL-Region-Gerüst), das SEQ ID NO: 6 für FR1, SEQ ID NO: 7 für FR2, SEQ ID NO: 8 für FR3 und SEQ ID NO: 10 für FR4 umfasst,
iv) ein bzw. einem Gerüst (so ein bzw. einem VL-Region-Gerüst), das SEQ ID NO: 11 für FR1, SEQ ID NO: 12 für FR2, SEQ ID NO: 13 für FR3 und SEQ ID NO: 15 für FR4 umfasst,
v) Kombinationen davon, und
wobei das Gerüst i), ii), iii), iv) oder Kombinationen davon in einem dsscFv vorliegen und wobei es sich bei dem Antikörpermolekül um ein Fab-dsscFv oder Fab-(dsscFv)₂ handelt.

## Revendications

1. Molécule d'anticorps multispécifique comprenant au moins deux domaines de liaison chacun comportant une région lourde variable VH et une région légère variable VL, chaque domaine de liaison étant spécifique pour un antigène et comprenant 6 CDR, **caractérisée en ce qu'**au moins un domaine de liaison comprend :
a. une région lourde variable, VH_{A}, possédant une structure comprenant la SEQ ID NO: 1 pour FR1, la SEQ ID NO: 3 pour FR2, la SEQ ID NO: 4 pour FR3 et la SEQ ID NO: 5 pour FR4, à la condition que CDRH3 dans VH_{A} soit différent de RYYSAMPFAY (SEQ ID NO: 129) et une région légère variable VL_{A}, possédant une structure comprenant la SEQ ID NO: 6 pour FR1, la SEQ ID NO: 7 pour FR2, la SEQ ID NO: 8 pour FR3 et la SEQ ID NO: 10 pour FR4 ; ou
b. une région lourde variable, VH_{A}, possédant une structure comprenant la SEQ ID NO: 16 pour FR1, la SEQ ID NO: 18 pour FR2, la SEQ ID NO: 19 pour FR3 et la SEQ ID NO: 20 pour FR4, à la condition que CDRH3 dans VH_{A} soit différent de RYYSAMPFAY (SEQ ID NO: 129) et une région légère variable VL_{A}, possédant une structure comprenant la SEQ ID NO: 11 pour FR1, la SEQ ID NO: 12 pour FR2, la SEQ ID NO: 13 pour FR3 et la SEQ ID NO: 15 pour FR4 ;
et l'au moins une région variable VH_{A} et l'au moins une région variable VL_{A} étant présentes dans un dsscFv, et la molécule d'anticorps multispécifique étant un Fab-dsscFv ou Fab-(dsscFv)₂.

2. Molécule d'anticorps multispécifique selon la revendication 1, VL_{A} comprenant la SEQ ID NO: 99 et VH_{A} comprenant la SEQ ID NO: 101.

3. Molécule d'anticorps multispécifique selon la revendication 2, un domaine de liaison comprenant la SEQ ID NO: 106.

4. Molécule d'anticorps multispécifique selon la revendication 1, VL_{A} comprenant la SEQ ID NO: 103 et VH_{A} comprenant la SEQ ID NO: 105.

5. Molécule d'anticorps multispécifique selon la revendication 4, un domaine de liaison comprenant la SEQ ID NO: 111.

6. Molécule d'anticorps multispécifique selon l'une quelconque des revendications 1 à 5, la VH_{A} comprenant 3 CDR de chaîne lourde possédant la séquence donnée dans la SEQ ID NO: 143 pour CDRH1, la SEQ ID NO: 144 pour CDRH2 et la SEQ ID NO: 145 pour CDRH3.

7. Molécule d'anticorps multispécifique selon l'une quelconque des revendications 1 à 6, la VL_{A} comprenant 3 CDR de chaîne légère possédant la séquence donnée dans la SEQ ID NO: 146 pour CDRL1, la SEQ ID NO: 147 pour CDRL2 et la SEQ ID NO: 148 pour CDRL3.

8. Molécule d'anticorps multispécifique selon une quelconque revendication précédente, les CDR possédant une origine non humaine.

9. Molécule d'anticorps multispécifique selon une quelconque revendication précédente, la molécule d'anticorps étant bispécifique ou trispécifique, telle que bispécifique.

10. Structure d'accepteur pour la minimisation de l'agrégation dans un anticorps multispécifique comprenant les SEQ ID NO: 22 et 24, XXX dans chacune des positions CDR représentant indépendamment 1 à 35 acides aminés, et les SEQ ID NO: 22 et 24 étant présentes dans un dsscFv, et l'anticorps multispécifique étant un Fab-dsscFv ou Fab-(dsscFv)₂.

11. Procédé d'amélioration des niveaux d'expression de monomère d'une molécule d'anticorps possédant au moins un domaine de liaison comportant une région lourde variable et une région légère variable où chaque région variable possède une structure et 3 CDR, ledit procédé comprenant l'étape de modification d'une structure d'une région variable en une structure choisie dans le groupe comprenant ou constitué par :
i) une structure (telle qu'une structure de région VH) comprenant la SEQ ID NO: 1 pour FR1, la SEQ ID NO: 3 pour FR2, la SEQ ID NO: 4 pour FR3 et la SEQ ID NO: 5 pour FR4,
ii) une structure (telle qu'une structure de région VH) comprenant la SEQ ID NO: 16 pour FR1, la SEQ ID NO: 18 pour FR2, la SEQ ID NO: 19 pour FR3 et la SEQ ID NO: 20 pour FR4,
iii) une structure (telle qu'une structure de région VL) comprenant la SEQ ID NO: 6 pour FR1, la SEQ ID NO: 7 pour FR2, la SEQ ID NO: 8 pour FR3 et la SEQ ID NO: 10 pour FR4,
iv) une structure (telle qu'une structure de région VL) comprenant VL1, la SEQ ID NO: 11 pour FR1, la SEQ ID NO: 12 pour FR2, la SEQ ID NO: 13 pour FR3 et la SEQ ID NO: 15 pour FR4,
v) des combinaisons correspondantes et,
la structure i), ii), iii), iv), ou des combinaisons correspondantes étant présente(s) dans un dsscFv, et la molécule d'anticorps étant un Fab-dsscFv ou Fab-(dsscFv)₂.


### SEQUENCE LISTING

<110> UCB BIOPHARMA SPRL
<120> Antibody frameworks for reducing aggregation of multispecific
   antibodies
<130> PF0109-WO
<160> 153
<170> PatentIn version 3.5
<210> 1
   <211> 25
   <212> PRT
   <213> Artificial Sequence
<220>
   <223> (497 VH)FR1
<400> 1
   Glu Val Gln Leu Val Glu Ser Gly Gly Gly Leu Val Lys Pro Gly Gly
   1               5                   10                  15
   Ser Leu Arg Leu Ser Cys Ala Ala Ser
               20                  25
<210> 2
   <211> 14
   <212> PRT
   <213> Artificial Sequence
<220>
   <223> (497 VH)FR2
<400> 2
   Trp Val Arg Gln Ala Pro Gly Lys Gly Leu Glu Trp Val Gly
   1               5                   10
<210> 3
   <211> 14
   <212> PRT
   <213> Artificial Sequence
<220>
   <223> (ds497 VH)FR2
<400> 3
   Trp Val Arg Gln Ala Pro Gly Lys Cys Leu Glu Trp Val Gly
   1               5                   10
<210> 4
   <211> 32
   <212> PRT
   <213> Artificial Sequence
<220>
   <223> (497 VH) FR3
<400> 4
   Arg Phe Thr Ile Ser Arg Asp Asp Ser Lys Asn Thr Leu Tyr Leu Gln
   1               5                   10                  15
   Met Asn Ser Leu Lys Thr Glu Asp Thr Ala Val Tyr Tyr Cys Thr Thr
               20                  25                  30
<210> 5
   <211> 11
   <212> PRT
   <213> Artificial Sequence
<220>
   <223> (497 VH) FR4
<400> 5
   Trp Gly Gln Gly Thr Leu Val Thr Val Ser Ser
   1               5                   10
<210> 6
   <211> 23
   <212> PRT
   <213> Artificial Sequence
<220>
   <223> (497 VL)FR1
<400> 6
   Ala Ile Gln Leu Thr Gln Ser Pro Ser Ser Leu Ser Ala Ser Val Gly
   1               5                   10                  15
   Asp Arg Val Thr Ile Thr Cys
               20
<210> 7
   <211> 15
   <212> PRT
   <213> Artificial Sequence
<220>
   <223> (497 VL)FR2
<400> 7
   Trp Tyr Gln Gln Lys Pro Gly Lys Ala Pro Lys Leu Leu Ile Tyr
   1               5                   10                  15
<210> 8
   <211> 32
   <212> PRT
   <213> Artificial Sequence
<220>
   <223> (497 VL)FR3
<400> 8
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 9
   <211> 10
   <212> PRT
   <213> Artificial Sequence
<220>
   <223> (497 VL)FR4
<400> 9
   Phe Gly Gln Gly Thr Lys Val Glu Ile Lys
   1               5                   10
<210> 10
   <211> 10
   <212> PRT
   <213> Artificial Sequence
<220>
   <223> (ds497 VL)FR4
<400> 10
   Phe Gly Cys Gly Thr Lys Val Glu Ile Lys
   1               5                   10
<210> 11
   <211> 23
   <212> PRT
   <213> Artificial Sequence
<220>
   <223> (2109 VL)FR1
<400> 11
   Asp Ile Gln Met Thr Gln Ser Pro Ser Ser Leu Ser Ala Ser Val Gly
   1               5                   10                  15
   Asp Arg Val Thr Ile Thr Cys
               20
<210> 12
   <211> 15
   <212> PRT
   <213> Artificial Sequence
<220>
   <223> (2109 VL)FR2
<400> 12
   Trp Tyr Gln Gln Lys Pro Gly Lys Val Pro Lys Leu Leu Ile Tyr
   1               5                   10                  15
<210> 13
   <211> 32
   <212> PRT
   <213> Artificial Sequence
<220>
   <223> (2109 VL)FR3
<400> 13
   Gly Val Pro Ser Arg Phe Ser Gly Thr Gly Ser Gly Thr Asp Tyr Thr
   1               5                   10                  15
   Leu Thr Ile Ser Ser Leu Gln Pro Glu Asp Val Ala Thr Tyr Phe Cys
               20                  25                  30
<210> 14
   <211> 10
   <212> PRT
   <213> Artificial Sequence
<220>
   <223> (2109 VL)FR4
<400> 14
   Phe Gly Gln Gly Thr Lys Leu Glu Ile Lys
   1               5                   10
<210> 15
   <211> 10
   <212> PRT
   <213> Artificial Sequence
<220>
   <223> (ds2109 VL)FR4
<400> 15
   Phe Gly Cys Gly Thr Lys Leu Glu Ile Lys
   1               5                   10
<210> 16
   <211> 25
   <212> PRT
   <213> Artificial Sequence
<220>
   <223> (2109 VH)FR1
<400> 16
   Glu Val Gln Leu Val Glu Ser Gly Gly Gly Leu Val Lys Pro Gly Gly
   1               5                   10                  15
   Ser Leu Arg Leu Ser Cys Ala Ala Ser
               20                  25
<210> 17
   <211> 14
   <212> PRT
   <213> Artificial Sequence
<220>
   <223> (2109 VH)FR2
<400> 17
   Trp Val Arg Gln Ala Pro Gly Lys Gly Leu Glu Trp Ile Gly
   1 5 10
<210> 18
   <211> 14
   <212> PRT
   <213> Artificial Sequence
<220>
   <223> (ds2109 VH)FR2
<400> 18
   Trp Val Arg Gln Ala Pro Gly Lys Cys Leu Glu Trp Ile Gly
   1 5 10
<210> 19
   <211> 32
   <212> PRT
   <213> Artificial Sequence
<220>
   <223> (2109 VH)FR3
<400> 19
   Arg Phe Thr Ile Ser Val Asp Lys Ala Lys Asn Ser Ala Tyr Leu Gln
   1 5 10 15
   Met Asn Ser Leu Arg Ala Glu Asp Thr Ala Val Tyr Tyr Cys Thr Arg
   20 25 30
<210> 20
   <211> 11
   <212> PRT
   <213> Artificial Sequence
<220>
   <223> (2109 VH)FR4
<400> 20
   Trp Gly Gln Gly Thr Leu Val Thr Val Ser Ser
   1 5 10
<210> 21
   <211> 85
   <212> PRT
   <213> Artificial Sequence
<220>
   <223> ds497 VH framework
<220>
   <221> MISC_FEATURE
   <222> (26)..(26)
   <223> Xaa represents a space for inserting a CDR
<220>
   <221> MISC_FEATURE
   <222> (41)..(41)
   <223> Xaa represents a space for inserting a CDR
<220>
   <221> MISC_FEATURE
   <222> (74)..(74)
   <223> Xaa represents a space for inserting a CDR
<400> 21
   Glu Val Gln Leu Val Glu Ser Gly Gly Gly Leu Val Lys Pro Gly Gly
   1               5                   10                  15
   Ser Leu Arg Leu Ser Cys Ala Ala Ser Xaa Trp Val Arg Gln Ala Pro
               20                  25                  30
   Gly Lys Gly Leu Glu Trp Val Gly Xaa Arg Phe Thr Ile Ser Arg Asp
           35                  40                  45
   Asp Ser Lys Asn Thr Leu Tyr Leu Gln Met Asn Ser Leu Lys Thr Glu
       50                  55                  60
   Asp Thr Ala Val Tyr Tyr Cys Thr Thr Xaa Trp Gly Gln Gly Thr Leu
   65                  70                  75                  80
   Val Thr Val Ser Ser
                   85
<210> 22
   <211> 85
   <212> **PRT**
   <213> Artificial Sequence
<220>
   <223> ds497 VH framework
<220>
   <221> MISC_FEATURE
   <222> (26)..(26)
   <223> Xaa represents a space for inserting a CDR
<220>
   <221> MISC_FEATURE
   <222> (41)..(41)
   <223> Xaa represents a space for inserting a CDR
<220>
   <221> MISC_FEATURE
   <222> (74)..(74)
   <223> Xaa represents a space for inserting a CDR
<400> 22
   Glu Val Gln Leu Val Glu Ser Gly Gly Gly Leu Val Lys Pro Gly Gly
   1               5                   10                  15
   Ser Leu Arg Leu Ser Cys Ala Ala Ser Xaa Trp Val Arg Gln Ala Pro
               20                  25                  30
   Gly Lys Cys Leu Glu Trp Val Gly Xaa Arg Phe Thr Ile Ser Arg Asp
           35                  40                  45
   Asp Ser Lys Asn Thr Leu Tyr Leu Gln Met Asn Ser Leu Lys Thr Glu
       50                  55                  60
   Asp Thr Ala Val Tyr Tyr Cys Thr Thr Xaa Trp Gly Gln Gly Thr Leu
   65                  70                  75                  80
   Val Thr Val Ser Ser
                   85
<210> 23
   <211> 83
   <212> PRT
   <213> Artificial Sequence
<220>
   <223> ds497 VL framework
<220>
   <221> MISC_FEATURE
   <222> (24)..(24)
   <223> Xaa represents a space for inserting a CDR
<220>
   <221> MISC_FEATURE
   <222> (40)..(40)
   <223> Xaa represents a space for inserting a CDR
<220>
   <221> MISC_FEATURE
   <222> (73)..(73)
   <223> Xaa represents a space for inserting a CDR
<400> 23
   Ala Ile Gln Leu Thr Gln Ser Pro Ser Ser Leu Ser Ala Ser Val Gly
   1 5 10 15
   Asp Arg Val Thr Ile Thr Cys Xaa Trp Tyr Gln Gln Lys Pro Gly Lys
   20 25 30
   Ala Pro Lys Leu Leu Ile Tyr Xaa Gly Val Pro Ser Arg Phe Ser Gly
   35 40 45
   Ser Gly Ser Gly Thr Asp Phe Thr Leu Thr Ile Ser Ser Leu Gln Pro
   50 55 60
   Glu Asp Phe Ala Thr Tyr Tyr Cys Xaa Phe Gly Gln Gly Thr Lys Val
   65 70 75 80
   Glu Ile Lys
<210> 24
   <211> 83
   <212> PRT
   <213> Artificial Sequence
<220>
   <223> ds497 VL framework
<220>
   <221> MISC_FEATURE
   <222> (24)..(24)
   <223> Xaa represents a space for inserting a CDR
<220>
   <221> MISC_FEATURE
   <222> (40)..(40)
   <223> Xaa represents a space for inserting a CDR
<220>
   <221> MISC_FEATURE
   <222> (73)..(73)
   <223> Xaa represents a space for inserting a CDR
<400> 24
   Ala Ile Gln Leu Thr Gln Ser Pro Ser Ser Leu Ser Ala Ser Val Gly
   1 5 10 15
   Asp Arg Val Thr Ile Thr Cys Xaa Trp Tyr Gln Gln Lys Pro Gly Lys
   20 25 30
   Ala Pro Lys Leu Leu Ile Tyr Xaa Gly Val Pro Ser Arg Phe Ser Gly
   35 40 45
   Ser Gly Ser Gly Thr Asp Phe Thr Leu Thr Ile Ser Ser Leu Gln Pro
   50 55 60
   Glu Asp Phe Ala Thr Tyr Tyr Cys Xaa Phe Gly Cys Gly Thr Lys Val
   65 70 75 80
   Glu Ile Lys
<210> 25
   <211> 83
   <212> PRT
   <213> Artificial Sequence
<220>
   <223> 2109 VL framework
<220>
   <221> MISC_FEATURE
   <222> (24)..(24)
   <223> Xaa represents a space for inserting a CDR
<220>
   <221> MISC_FEATURE
   <222> (40)..(40)
   <223> Xaa represents a space for inserting a CDR
<220>
   <221> MISC_FEATURE
   <222> (73)..(73)
   <223> Xaa represents a space for inserting a CDR
<400> 25
   Asp Ile Gln Met Thr Gln Ser Pro Ser Ser Leu Ser Ala Ser Val Gly
   1 5 10 15
   Asp Arg Val Thr Ile Thr Cys Xaa Trp Tyr Gln Gln Lys Pro Gly Lys
   20 25 30
   Val Pro Lys Leu Leu Ile Tyr Xaa Gly Val Pro Ser Arg Phe Ser Gly
   35 40 45
   Thr Gly Ser Gly Thr Asp Tyr Thr Leu Thr Ile Ser Ser Leu Gln Pro
   50 55 60
   Glu Asp Val Ala Thr Tyr Phe Cys Xaa Phe Gly Gln Gly Thr Lys Leu
   65 70 75 80
   Glu Ile Lys
<210> 26
   <211> 83
   <212> PRT
   <213> Artificial Sequence
<220>
   <223> ds2109 VL framework
<220>
   <221> MISC_FEATURE
   <222> (24)..(24)
   <223> Xaa represents a space for inserting a CDR
<220>
   <221> MISC_FEATURE
   <222> (40)..(40)
   <223> Xaa represents a space for inserting a CDR
<220>
   <221> MISC_FEATURE
   <222> (73)..(73)
   <223> Xaa represents a space for inserting a CDR
<400> 26
   Asp Ile Gln Met Thr Gln Ser Pro Ser Ser Leu Ser Ala Ser Val Gly
   1 5 10 15
   Asp Arg Val Thr Ile Thr Cys Xaa Trp Tyr Gln Gln Lys Pro Gly Lys
   20 25 30
   Val Pro Lys Leu Leu Ile Tyr Xaa Gly Val Pro Ser Arg Phe Ser Gly
   35 40 45
   Thr Gly Ser Gly Thr Asp Tyr Thr Leu Thr Ile Ser Ser Leu Gln Pro
   50 55 60
   Glu Asp Val Ala Thr Tyr Phe Cys Xaa Phe Gly Cys Gly Thr Lys Leu
   65 70 75 80
   Glu Ile Lys
<210> 27
   <211> 85
   <212> PRT
   <213> Artificial Sequence
<220>
   <223> 2109 VH framework
<220>
   <221> MISC_FEATURE
   <222> (26)..(26)
   <223> Xaa represents a space for inserting a CDR
<220>
   <221> MISC_FEATURE
   <222> (41)..(41)
   <223> Xaa represents a space for inserting a CDR
<220>
   <221> MISC_FEATURE
   <222> (74)..(74)
   <223> Xaa represents a space for inserting a CDR
<400> 27
   Glu Val Gln Leu Val Glu Ser Gly Gly Gly Leu Val Lys Pro Gly Gly
   1               5                   10                  15
   Ser Leu Arg Leu Ser Cys Ala Ala Ser Xaa Trp Val Arg Gln Ala Pro
               20                  25                  30
   Gly Lys Gly Leu Glu Trp Ile Gly Xaa Arg Phe Thr Ile Ser Val Asp
           35                  40                  45
   Lys Ala Lys Asn Ser Ala Tyr Leu Gln Met Asn Ser Leu Arg Ala Glu
       50                  55                  60
   Asp Thr Ala Val Tyr Tyr Cys Thr Arg Xaa Trp Gly Gln Gly Thr Leu
   65                  70                  75                  80
   Val Thr Val Ser Ser
                   85
<210> 28
   <211> 85
   <212> PRT
   <213> Artificial Sequence
<220>
   <223> ds2109 VH framework
<220>
   <221> MISC_FEATURE
   <222> (26)..(26)
   <223> Xaa represents a space for inserting a CDR
<220>
   <221> MISC_FEATURE
   <222> (41)..(41)
   <223> Xaa represents a space for inserting a CDR
<220>
   <221> MISC_FEATURE
   <222> (74)..(74)
   <223> Xaa represents a space for inserting a CDR
<400> 28
   Glu Val Gln Leu Val Glu Ser Gly Gly Gly Leu Val Lys Pro Gly Gly
   1               5                   10                  15
   Ser Leu Arg Leu Ser Cys Ala Ala Ser Xaa Trp Val Arg Gln Ala Pro
               20                  25                  30
   Gly Lys Cys Leu Glu Trp Ile Gly Xaa Arg Phe Thr Ile Ser Val Asp
           35                  40                  45
   Lys Ala Lys Asn Ser Ala Tyr Leu Gln Met Asn Ser Leu Arg Ala Glu
       50                  55                  60
   Asp Thr Ala Val Tyr Tyr Cys Thr Arg Xaa Trp Gly Gln Gly Thr Leu
   65                  70                  75                  80
   Val Thr Val Ser Ser
                   85
<210> 29
   <211> 11
   <212> PRT
   <213> Artificial Sequence
<220>
   <223> Linker
<400> 29
   Ser Gly Gly Gly Gly Thr Gly Gly Gly Gly Ser
   1               5                   10
<210> 30
   <211> 11
   <212> PRT
   <213> Artificial Sequence
<220>
   <223> Linker
<400> 30
   Ser Gly Gly Gly Gly Ser Gly Gly Gly Gly Ser
   1               5                   10
<210> 31
   <211> 8
   <212> PRT
   <213> Artificial Sequence
<220>
   <223> Hinge linker sequence
<400> 31
   Asp Lys Thr His Thr Cys Ala Ala
   1               5
<210> 32
   <211> 11
   <212> PRT
   <213> Artificial Sequence
<220>
   <223> Hinge linker sequence
<400> 32
   Asp Lys Thr His Thr Cys Pro Pro Cys Pro Ala
   1               5                   10
<210> 33
   <211> 18
   <212> PRT
   <213> Artificial Sequence
<220>
   <223> Hinge linker sequence
<400> 33
   Asp Lys Thr His Thr Cys Pro Pro Cys Pro Ala Thr Cys Pro Pro Cys
   1               5                   10                  15
   Pro Ala
<210> 34
   <211> 25
   <212> PRT
   <213> Artificial Sequence
<220>
   <223> Hinge linker sequence
<400> 34
   Asp Lys Thr His Thr Cys Pro Pro Cys Pro Ala Thr Cys Pro Pro Cys
   1               5                   10                  15
   Pro Ala Thr Cys Pro Pro Cys Pro Ala
               20                  25
<210> 35
   <211> 30
   <212> PRT
   <213> Artificial Sequence
<220>
   <223> Hinge linker sequence
<400> 35
   Asp Lys Thr His Thr Cys Pro Pro Cys Pro Ala Gly Lys Pro Thr Leu
   1               5                   10                  15
   Tyr Asn Ser Leu Val Met Ser Asp Thr Ala Gly Thr Cys Tyr
               20                  25                  30
<210> 36
   <211> 31
   <212> PRT
   <213> Artificial Sequence
<220>
   <223> Hinge linker sequence
<400> 36
   Asp Lys Thr His Thr Cys Pro Pro Cys Pro Ala Gly Lys Pro Thr His
   1               5                   10                  15
   Val Asn Val Ser Val Val Met Ala Glu Val Asp Gly Thr Cys Tyr
               20                  25                  30
<210> 37
   <211> 15
   <212> PRT
   <213> Artificial Sequence
<220>
   <223> Hinge linker sequence
<400> 37
   Asp Lys Thr His Thr Cys Cys Val Glu Cys Pro Pro Cys Pro Ala
   1               5                   10                  15
<210> 38
   <211> 26
   <212> PRT
   <213> Artificial Sequence
<220>
   <223> Hinge linker sequence
<400> 38
   Asp Lys Thr His Thr Cys Pro Arg Cys Pro Glu Pro Lys Ser Cys Asp
   1 5 10 15
   Thr Pro Pro Pro Cys Pro Arg Cys Pro Ala
   20 25
<210> 39
   <211> 11
   <212> PRT
   <213> Artificial Sequence
<220>
   <223> Hinge linker sequence
<400> 39
   Asp Lys Thr His Thr Cys Pro Ser Cys Pro Ala
   1 5 10
<210> 40
   <211> 7
   <212> PRT
   <213> Artificial Sequence
<220>
   <223> Flexible linker sequence
<400> 40
   Ser Gly Gly Gly Gly Ser Glu
   1 5
<210> 41
   <211> 6
   <212> **PRT**
   <213> **Artificial** Sequence
<220>
   <223> Flexible linker sequence
<400> **41**
   Asp Lys Thr His Thr Ser
   1 5
<210> 42
   <211> 6
   <212> **PRT**
   <213> **Artificial** Sequence
<220>
   <223> Flexible linker sequence
<220>
   <221> SITE
   <222> (1)..(1)
   <223> Ser is optional
<400> **42**
   Ser Gly Gly Gly Gly Ser
   1               5
<210> 43
   <211> 11
   <212> **PRT**
   <213> **Artificial** Sequence
<220>
   <223> Flexible linker sequence
<220>
   <221> SITE
   <222> (1)..(1)
   <223> Ser is optional
<400> 43
   Ser Gly Gly Gly Gly Ser Gly Gly Gly Gly Ser
   1               5                   10
<210> **44**
   <211> 16
   <212> **PRT**
   <213> **Artificial** Sequence
<220>
   <223> Flexible linker sequence
<220>
   <221> SITE
   <222> (1)..(1)
   <223> Ser is optional
<400> **44**
   Ser Gly Gly Gly Gly Ser Gly Gly Gly Gly Ser Gly Gly Gly Gly Ser
   1               5                   10                  15
<210> 45
   <211> 21
   <212> **PRT**
   <213> **Artificial** Sequence
<220>
   <223> Flexible linker sequence
<220>
   <221> SITE
   <222> (1)..(1)
   <223> Ser is optional
<400> **45**
   Ser Gly Gly Gly Gly Ser Gly Gly Gly Gly Ser Gly Gly Gly Gly Ser
   1               5                   10                  15
   Gly Gly Gly Gly Ser
        20
<210> 46
   <211> 16
   <212> **PRT**
   <213> **Artificial** Sequence
<220>
   <223> Flexible linker sequence
<400> **46**
   Ser Gly Gly Gly Gly Ser Gly Gly Gly Gly Thr Gly Gly Gly Gly Ser
   1               5                   10                  15
<210> 47
   <211> 26
   <212> PRT
   <213> Artificial Sequence
<220>
   <223> Flexible linker sequence
<220>
   <221> SITE
   <222> (1)..(1)
   <223> Ser is optional
<400> 47
   Ser Gly Gly Gly Gly Ser Gly Gly Gly Gly Ser Gly Gly Gly Gly Ser
   1               5                   10                  15
   Gly Gly Gly Gly Ser Gly Gly Gly Gly Ser
               20                  25
<210> 48
   <211> 11
   <212> **PRT**
   <213> **Artificial** Sequence
<220>
   <223> Flexible linker sequence
<400> **48**
   Ala Ala Ala Gly Ser Gly Gly Ala Ser Ala Ser
   1               5                   10
<210> 49
   <211> 16
   <212> **PRT**
   <213> **Artificial** Sequence
<220>
   <223> Flexible linker sequence
<220>
   <221> MISC_FEATURE
   <222> (7)..(7)
   <223> Xaa can be any naturally occurring amino acid
<400> 49
   Ala Ala Ala Gly Ser Gly Xaa Gly Gly Gly Ser Gly Ala Ser Ala Ser
   1               5                   10                  15
<210> 50
   <211> 21
   <212> PRT
   <213> Artificial Sequence
<220>
   <223> Flexible linker sequence
<220>
   <221> MISC_FEATURE
   <222> (7)..(7)
   <223> Xaa can be any naturally occurring amino acid
<220>
   <221> MISC_FEATURE
   <222> (12)..(12)
   <223> Xaa can be any naturally occurring amino acid
<400> 50
   Ala Ala Ala Gly Ser Gly Xaa Gly Gly Gly Ser Xaa Gly Gly Gly Ser
   1               5                   10                  15
   Gly Ala Ser Ala Ser
               20
<210> 51
   <211> 26
   <212> PRT
   <213> Artificial Sequence
<220>
   <223> Flexible linker sequence
<220>
   <221> MISC_FEATURE
   <222> (7)..(7)
   <223> Xaa can be any naturally occurring amino acid
<220>
   <221> MISC_FEATURE
   <222> (12)..(12)
   <223> Xaa can be any naturally occurring amino acid
<220>
   <221> MISC_FEATURE
   <222> (17)..(17)
   <223> Xaa can be any naturally occurring amino acid
<400> 51
   Ala Ala Ala Gly Ser Gly Xaa Gly Gly Gly Ser Xaa Gly Gly Gly Ser
   1               5                   10                  15
   Xaa Gly Gly Gly Ser Gly Ala Ser Ala Ser
               20                  25
<210> 52
   <211> 31
   <212> PRT
   <213> Artificial Sequence
<220>
   <223> Flexible linker sequence
<220>
   <221> MISC_FEATURE
   <222> (7)..(7)
   <223> Xaa can be any naturally occurring amino acid
<220>
   <221> MISC_FEATURE
   <222> (12)..(12)
   <223> Xaa can be any naturally occurring amino acid
<220>
   <221> MISC_FEATURE
   <222> (17)..(17)
   <223> Xaa can be any naturally occurring amino acid
<220>
   <221> MISC_FEATURE
   <222> (22)..(22)
   <223> Xaa can be any naturally occurring amino acid
<400> 52
   Ala Ala Ala Gly Ser Gly Xaa Gly Gly Gly Ser Xaa Gly Gly Gly Ser
   1               5                   10                  15
   Xaa Gly Gly Gly Ser Xaa Gly Gly Gly Ser Gly Ala Ser Ala Ser
               20                  25                  30
<210> 53
   <211> 13
   <212> PRT
   <213> Artificial Sequence
<220>
   <223> Flexible linker sequence
<220>
   <221> MISC_FEATURE
   <222> (7)..(7)
   <223> Xaa can be any naturally occurring amino acid
<400> 53
   Ala Ala Ala Gly Ser Gly Xaa Ser Gly Ala Ser Ala Ser
   1               5                   10
<210> 54
   <211> 28
   <212> PRT
   <213> Artificial Sequence
<220>
   <223> Flexible linker sequence
<400> 54
   Pro Gly Gly Asn Arg Gly Thr Thr Thr Thr Arg Arg Pro Ala Thr Thr
   1               5                   10                  15
   Thr Gly Ser Ser Pro Gly Pro Thr Gln Ser His Tyr
               20                  25
<210> 55
   <211> 11
   <212> **PRT**
   <213> **Artificial** Sequence
<220>
   <223> Flexible linker sequence
<400> 55
   Ala Thr Thr Thr Gly Ser Ser Pro Gly Pro Thr
   1               5                   10
<210> 56
   <211> 6
   <212> **PRT**
   <213> **Artificial** Sequence
<220>
   <223> Flexible linker sequence
<400> 56
   Ala Thr Thr Thr Gly Ser
   1               5
<210> 57
   <211> 21
   <212> **PRT**
   <213> **Artificial** Sequence
<220>
   <223> Flexible linker sequence
<400> 57
   Glu Pro Ser Gly Pro Ile Ser Thr Ile Asn Ser Pro Pro Ser Lys Glu
   1               5                   10                  15
   Ser His Lys Ser Pro
               20
<210> 58
   <211> 15
   <212> PRT
   <213> Artificial Sequence
<220>
   <223> Flexible linker sequence
<400> 58
   Gly Thr Val Ala Ala Pro Ser Val Phe Ile Phe Pro Pro Ser Asp
   1               5                   10                  15
<210> 59
   <211> 15
   <212> **PRT**
   <213> **Artificial** Sequence
<220>
   <223> Flexible linker sequence
<400> 59
   Gly Gly Gly Gly Ile Ala Pro Ser Met Val Gly Gly Gly Gly Ser
   1               5                   10                  15
<210> 60
   <211> 15
   <212> PRT
   <213> Artificial Sequence
<220>
   <223> Flexible linker sequence
<400> 60
   Gly Gly Gly Gly Lys Val Glu Gly Ala Gly Gly Gly Gly Gly Ser
   1               5                   10                  15
<210> 61
   <211> 15
   <212> **PRT**
   <213> **Artificial** Sequence
<220>
   <223> Flexible linker sequence
<400> 61
   Gly Gly Gly Gly Ser Met Lys Ser His Asp Gly Gly Gly Gly Ser
   1               5                   10                  15
<210> 62
   <211> 15
   <212> PRT
   <213> Artificial Sequence
<220>
   <223> Flexible linker sequence
<400> 62
   Gly Gly Gly Gly Asn Leu Ile Thr Ile Val Gly Gly Gly Gly Ser
   1               5                   10                  15
<210> 63
   <211> 15
   <212> PRT
   <213> Artificial Sequence
<220>
   <223> Flexible linker sequence
<400> 63
   Gly Gly Gly Gly Val Val Pro Ser Leu Pro Gly Gly Gly Gly Ser
   1               5                   10                  15
<210> 64
   <211> 12
   <212> **PRT**
   <213> **Artificial** Sequence
<220>
   <223> Flexible linker sequence
<400> **64**
   Gly Gly Glu Lys Ser Ile Pro Gly Gly Gly Gly Ser
   1               5                   10
<210> 65
   <211> 18
   <212> **PRT**
   <213> **Artificial** Sequence
<220>
   <223> Flexible linker sequence
<400> 65
   Arg Pro Leu Ser Tyr Arg Pro Pro Phe Pro Phe Gly Phe Pro Ser Val
   1               5                   10                  15
   Arg Pro
<210> 66
   <211> 18
   <212> PRT
   <213> Artificial Sequence
<220>
   <223> Flexible linker sequence
<400> 66
   Tyr Pro Arg Ser Ile Tyr Ile Arg Arg Arg His Pro Ser Pro Ser Leu
   1               5                   10                  15
   Thr Thr
<210> 67
   <211> 18
   <212> **PRT**
   <213> **Artificial** Sequence
<220>
   <223> Flexible linker sequence
<400> 67
   Thr Pro Ser His Leu Ser His Ile Leu Pro Ser Phe Gly Leu Pro Thr
   1               5                   10                  15
   Phe Asn
<210> 68
   <211> 18
   <212> PRT
   <213> Artificial Sequence
<220>
   <223> Flexible linker sequence
<400> 68
   Arg Pro Val Ser Pro Phe Thr Phe Pro Arg Leu Ser Asn Ser Trp Leu
   1               5                   10                  15
   Pro Ala
<210> 69
   <211> 18
   <212> PRT
   <213> Artificial Sequence
<220>
   <223> Flexible linker sequence
<400> 69
   Ser Pro Ala Ala His Phe Pro Arg Ser Ile Pro Arg Pro Gly Pro Ile
   1               5                   10                  15
   Arg Thr
<210> 70
   <211> 18
   <212> PRT
   <213> Artificial Sequence
<220>
   <223> Flexible linker sequence
<400> 70
   Ala Pro Gly Pro Ser Ala Pro Ser His Arg Ser Leu Pro Ser Arg Ala
   1               5                   10                  15
   Phe Gly
<210> 71
   <211> 18
   <212> **PRT**
   <213> **Artificial** Sequence
<220>
   <223> Flexible linker sequence
<400> 71
   Pro Arg Asn Ser Ile His Phe Leu His Pro Leu Leu Val Ala Pro Leu
   1               5                   10                  15
   Gly Ala
<210> 72
   <211> 18
   <212> PRT
   <213> Artificial Sequence
<220>
   <223> Flexible linker sequence
<400> 72
   Met Pro Ser Leu Ser Gly Val Leu Gln Val Arg Tyr Leu Ser Pro Pro
   1               5                   10                  15
   Asp Leu
<210> 73
   <211> 18
   <212> PRT
   <213> Artificial Sequence
<220>
   <223> Flexible linker sequence
<400> 73
   Ser Pro Gln Tyr Pro Ser Pro Leu Thr Leu Thr Leu Pro Pro His Pro
   1               5                   10                  15
   Ser Leu
<210> 74
   <211> 18
   <212> **PRT**
   <213> **Artificial** Sequence
<220>
   <223> Flexible linker sequence
<400> **74**
   Asn Pro Ser Leu Asn Pro Pro Ser Tyr Leu His Arg Ala Pro Ser Arg
   1               5                   10                  15
   Ile Ser
<210> 75
   <211> 17
   <212> **PRT**
   <213> **Artificial** Sequence
<220>
   <223> Flexible linker sequence
<400> 75
   Leu Pro Trp Arg Thr Ser Leu Leu Pro Ser Leu Pro Leu Arg Arg Arg
   1               5                   10                  15
   Pro
<210> 76
   <211> 18
   <212> **PRT**
   <213> **Artificial** Sequence
<220>
   <223> Flexible linker sequence
<400> 76
   Pro Pro Leu Phe Ala Lys Gly Pro Val Gly Leu Leu Ser Arg Ser Phe
   1               5                   10                  15
   Pro Pro
<210> 77
   <211> 18
   <212> **PRT**
   <213> **Artificial** Sequence
<220>
   <223> Flexible linker sequence
<400> 77
   Val Pro Pro Ala Pro Val Val Ser Leu Arg Ser Ala His Ala Arg Pro
   1               5                   10                  15
   Pro Tyr
<210> 78
   <211> 17
   <212> PRT
   <213> Artificial Sequence
<220>
   <223> Flexible linker sequence
<400> 78
   Leu Arg Pro Thr Pro Pro Arg Val Arg Ser Tyr Thr Cys Cys Pro Thr
   1               5                   10                  15
   Pro
<210> 79
   <211> 18
   <212> **PRT**
   <213> **Artificial** Sequence
<220>
   <223> Flexible linker sequence
<400> 79
   Pro Asn Val Ala His Val Leu Pro Leu Leu Thr Val Pro Trp Asp Asn
   1               5                   10                  15
   Leu Arg
<210> 80
   <211> 18
   <212> PRT
   <213> Artificial Sequence
<220>
   <223> Flexible linker sequence
<400> 80
   Cys Asn Pro Leu Leu Pro Leu Cys Ala Arg Ser Pro Ala Val Arg Thr
   1               5                   10                  15
   Phe Pro
<210> 81
   <211> 11
   <212> **PRT**
   <213> **Artificial** Sequence
<220>
   <223> rigid linker
<400> 81
   Gly Ala Pro Ala Pro Ala Ala Pro Ala Pro Ala
   1               5                   10
<210> 82
   <211> 4
   <212> PRT
   <213> Artificial Sequence
<220>
   <223> rigid linker
<400> 82
   Pro Pro Pro Pro
   1
<210> 83
   <211> 11
   <212> **PRT**
   <213> **Artificial** Sequence
<220>
   <223> albumin binding peptide
<400> 83
   Asp Leu Cys Leu Arg Asp Trp Gly Cys Leu Trp
   1               5                   10
<210> 84
   <211> 11
   <212> **PRT**
   <213> **Artificial** Sequence
<220>
   <223> albumin binding peptide
<400> **84**
   Asp Ile Cys Leu Pro Arg Trp Gly Cys Leu Trp
   1 5 10
<210> 85
   <211> 15
   <212> PRT
   <213> Artificial Sequence
<220>
   <223> albumin binding peptide
<400> 85
   Met Glu Asp Ile Cys Leu Pro Arg Trp Gly Cys Leu Trp Gly Asp
   1 5 10 15
<210> 86
   <211> 20
   <212> PRT
   <213> Artificial Sequence
<220>
   <223> albumin binding peptide
<400> 86
   Gln Arg Leu Met Glu Asp Ile Cys Leu Pro Arg Trp Gly Cys Leu Trp
   1 5 10 15
   Glu Asp Asp Glu
   20
<210> 87
   <211> 20
   <212> PRT
   <213> Artificial Sequence
<220>
   <223> albumin binding peptide
<400> 87
   Gln Gly Leu Ile Gly Asp Ile Cys Leu Pro Arg Trp Gly Cys Leu Trp
   1 5 10 15
   Gly Arg Ser Val
   20
<210> 88
   <211> 21
   <212> **PRT**
   <213> Artificial Sequence
<220>
   <223> albumin binding peptide
<400> 88
   Gln Gly Leu Ile Gly Asp Ile Cys Leu Pro Arg Trp Gly Cys Leu Trp
   1 5 10 15
   Gly Arg Ser Val Lys
   20
<210> 89
   <211> 15
   <212> PRT
   <213> Artificial Sequence
<220>
   <223> albumin binding peptide
<400> 89
   Glu Asp Ile Cys Leu Pro Arg Trp Gly Cys Leu Trp Glu Asp Asp
   1 5 10 15
<210> 90
   <211> 18
   <212> PRT
   <213> Artificial Sequence
<220>
   <223> albumin binding peptide
<400> 90
   Arg Leu Met Glu Asp Ile Cys Leu Pro Arg Trp Gly Cys Leu Trp Glu
   1 5 10 15
   Asp Asp
<210> 91
   <211> 16
   <212> **PRT**
   <213> **Artificial** Sequence
<220>
   <223> albumin binding peptide
<400> 91
   Met Glu Asp Ile Cys Leu Pro Arg Trp Gly Cys Leu Trp Glu Asp Asp
   1 5 10 15
<210> 92
   <211> 15
   <212> **PRT**
   <213> **Artificial** Sequence
<220>
   <223> albumin binding peptide
<400> 92
   Met Glu Asp Ile Cys Leu Pro Arg Trp Gly Cys Leu Trp Glu Asp
   1               5                   10                  15
<210> 93
   <211> 18
   <212> PRT
   <213> Artificial Sequence
<220>
   <223> albumin binding peptide
<400> 93
   Arg Leu Met Glu Asp Ile Cys Leu Ala Arg Trp Gly Cys Leu Trp Glu
   1               5                   10                  15
   Asp Asp
<210> 94
   <211> 20
   <212> PRT
   <213> Artificial Sequence
<220>
   <223> albumin binding peptide
<400> 94
   Glu Val Arg Ser Phe Cys Thr Arg Trp Pro Ala Glu Lys Ser Cys Lys
   1               5                   10                  15
   Pro Leu Arg Gly
               20
<210> 95
   <211> 20
   <212> PRT
   <213> Artificial Sequence
<220>
   <223> albumin binding peptide
<400> 95
   Arg Ala Pro Glu Ser Phe Val Cys Tyr Trp Glu Thr Ile Cys Phe Glu
   1 5 10 15
   Arg Ser Glu Gln
   20
<210> 96
   <211> 11
   <212> PRT
   <213> Artificial Sequence
<220>
   <223> albumin binding peptide
<400> 96
   Glu Met Cys Tyr Phe Pro Gly Ile Cys Trp Met
   1 5 10
<210> 97
   <211> 20
   <212> PRT
   <213> Artificial Sequence
<220>
   <223> Linker
<400> 97
   Gly Gly Gly Gly Ser Gly Gly Gly Gly Ser Gly Gly Gly Gly Ser Gly
   1 5 10 15
   Gly Gly Gly Ser
   20
<210> 98
   <211> 110
   <212> PRT
   <213> Artificial Sequence
<220>
   <223> 645 L CDRs with 497 VL framework comprising SEQ ID NO: 6, 7, 8 and 9
<400> 98
   Ala Ile Gln Leu Thr Gln Ser Pro Ser Ser Leu Ser Ala Ser Val Gly
   1 5 10 15
   Asp Arg Val Thr Ile Thr Cys Gln Ser Ser Pro Ser Val Trp Ser Asn
   20 25 30
   Phe Leu Ser Trp Tyr Gln Gln Lys Pro Gly Lys Ala Pro Lys Leu Leu
   35 40 45
   Ile Tyr Glu Ala Ser Lys Leu Thr Ser Gly Val Pro Ser Arg Phe Ser
   50 55 60
   Gly Ser Gly Ser Gly Thr Asp Phe Thr Leu Thr Ile Ser Ser Leu Gln
   65 70 75 80
   Pro Glu Asp Phe Ala Thr Tyr Tyr Cys Gly Gly Gly Tyr Ser Ser Ile
   85 90 95
   Ser Asp Thr Thr Phe Gly Gln Gly Thr Lys Val Glu Ile Lys
   100 105 110
<210> 99
   <211> 110
   <212> PRT
   <213> Artificial Sequence
<220>
   <223> 645 L CDRs with ds497 VL framework comprising SEQ ID NO: 6, 7, 8 and 10
<400> 99
   Ala Ile Gln Leu Thr Gln Ser Pro Ser Ser Leu Ser Ala Ser Val Gly
   1               5                   10                  15
   Asp Arg Val Thr Ile Thr Cys Gln Ser Ser Pro Ser Val Trp Ser Asn
               20                  25                  30
   Phe Leu Ser Trp Tyr Gln Gln Lys Pro Gly Lys Ala Pro Lys Leu Leu
           35                  40                  45
   Ile Tyr Glu Ala Ser Lys Leu Thr Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Phe Ala Thr Tyr Tyr Cys Gly Gly Gly Tyr Ser Ser Ile
                   85                  90                  95
   Ser Asp Thr Thr Phe Gly Cys Gly Thr Lys Val Glu Ile Lys
               100                 105                 110
<210> 100
   <211> 121
   <212> PRT
   <213> Artificial Sequence
<220>
   <223> 645 H CDRs with 497 VH framework comprising SEQ ID NO: 1, 2, 4 and 5
<400> 100
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Ile Asp Leu Ser Asn Tyr
   20 25 30
   Ala Ile Asn Trp Val Arg Gln Ala Pro Gly Lys Gly Leu Glu Trp Val
   35 40 45
   Gly Ile Ile Trp Ala Ser Gly Thr Thr Phe Tyr Ala Thr Trp Ala Lys
   50 55 60
   Gly Arg Phe Thr Ile Ser Arg Asp Asp Ser Lys Asn Thr Leu Tyr Leu
   65 70 75 80
   Gln Met Asn Ser Leu Lys Thr Glu Asp Thr Ala Val Tyr Tyr Cys Thr
   85 90 95
   Thr Thr Val Pro Gly Tyr Ser Thr Ala Pro Tyr Phe Asp Leu Trp Gly
   100 105 110
   Gln Gly Thr Leu Val Thr Val Ser Ser
   115 120
<210> 101
   <211> 121
   <212> **PRT**
   <213> **Artificial** Sequence
<220>
   <223> 645 H CDRs with ds497 VH framework comprising SEQ ID NO: 1, 3, 4 and 5
<400> 101
   Glu Val Gln Leu Val Glu Ser Gly Gly Gly Leu Val Lys Pro Gly Gly
   1 5 10 15
   Ser Leu Arg Leu Ser Cys Ala Ala Ser Gly Ile Asp Leu Ser Asn Tyr
   20 25 30
   Ala Ile Asn Trp Val Arg Gln Ala Pro Gly Lys Cys Leu Glu Trp Val
   35 40 45
   Gly Ile Ile Trp Ala Ser Gly Thr Thr Phe Tyr Ala Thr Trp Ala Lys
   50 55 60
   Gly Arg Phe Thr Ile Ser Arg Asp Asp Ser Lys Asn Thr Leu Tyr Leu
   65 70 75 80
   Gln Met Asn Ser Leu Lys Thr Glu Asp Thr Ala Val Tyr Tyr Cys Thr
   85 90 95
   Thr Thr Val Pro Gly Tyr Ser Thr Ala Pro Tyr Phe Asp Leu Trp Gly
   100 105 110
   Gln Gly Thr Leu Val Thr Val Ser Ser
   115 120
<210> 102
   <211> 110
   <212> PRT
   <213> Artificial Sequence
<220>
   <223> 645 L CDRs with 2109 VL framework comprising SEQ ID NO: 11, 12, 13 and 14
<400> 102
   Asp Ile Gln Met Thr Gln Ser Pro Ser Ser Leu Ser Ala Ser Val Gly
   1 5 10 15
   Asp Arg Val Thr Ile Thr Cys Gln Ser Ser Pro Ser Val Trp Ser Asn
   20 25 30
   Phe Leu Ser Trp Tyr Gln Gln Lys Pro Gly Lys Val Pro Lys Leu Leu
   35 40 45
   Ile Tyr Glu Ala Ser Lys Leu Thr Ser Gly Val Pro Ser Arg Phe Ser
   50 55 60
   Gly Thr Gly Ser Gly Thr Asp Tyr Thr Leu Thr Ile Ser Ser Leu Gln
   65 70 75 80
   Pro Glu Asp Val Ala Thr Tyr Phe Cys Gly Gly Gly Tyr Ser Ser Ile
   85 90 95
   Ser Asp Thr Thr Phe Gly Gln Gly Thr Lys Leu Glu Ile Lys
   100 105 110
<210> 103
   <211> 110
   <212> PRT
   <213> Artificial Sequence
<220>
   <223> 645 L CDRs with ds2109 VL framework comprising SEQ ID NO: 11, 12, 13 and 15
<400> 103
   Asp Ile Gln Met Thr Gln Ser Pro Ser Ser Leu Ser Ala Ser Val Gly
   1 5 10 15
   Asp Arg Val Thr Ile Thr Cys Gln Ser Ser Pro Ser Val Trp Ser Asn
               20                  25                  30
   Phe Leu Ser Trp Tyr Gln Gln Lys Pro Gly Lys Val Pro Lys Leu Leu
           35                  40                  45
   Ile Tyr Glu Ala Ser Lys Leu Thr Ser Gly Val Pro Ser Arg Phe Ser
       50                  55                  60
   Gly Thr Gly Ser Gly Thr Asp Tyr Thr Leu Thr Ile Ser Ser Leu Gln
   65                  70                  75                  80
   Pro Glu Asp Val Ala Thr Tyr Phe Cys Gly Gly Gly Tyr Ser Ser Ile
                   85                  90                  95
   Ser Asp Thr Thr Phe Gly Cys Gly Thr Lys Leu Glu Ile Lys
               100                 105                 110
<210> 104
   <211> 121
   <212> PRT
   <213> Artificial Sequence
<220>
   <223> 645 H CDRs with 2109 VH framework comprising SEQ ID NO: 16, 17, 19 and 20
<400> 104
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Ile Asp Leu Ser Asn Tyr
               20                  25                  30
   Ala Ile Asn Trp Val Arg Gln Ala Pro Gly Lys Gly Leu Glu Trp Ile
           35                  40                  45
   Gly Ile Ile Trp Ala Ser Gly Thr Thr Phe Tyr Ala Thr Trp Ala Lys
       50                  55                  60
   Gly Arg Phe Thr Ile Ser Val Asp Lys Ala Lys Asn Ser Ala Tyr Leu
   65                  70                  75                  80
   Gln Met Asn Ser Leu Arg Ala Glu Asp Thr Ala Val Tyr Tyr Cys Thr
                   85                  90                  95
   Arg Thr Val Pro Gly Tyr Ser Thr Ala Pro Tyr Phe Asp Leu Trp Gly
               100                 105                 110
   Gln Gly Thr Leu Val Thr Val Ser Ser
   115 120
<210> 105
   <211> 121
   <212> PRT
   <213> Artificial Sequence
<220>
   <223> 645 H CDRs with ds2109 VH framework comprising SEQ ID NO: 16, 18, 19 and 20
<400> 105
   Glu Val Gln Leu Val Glu Ser Gly Gly Gly Leu Val Lys Pro Gly Gly
   1 5 10 15
   Ser Leu Arg Leu Ser Cys Ala Ala Ser Gly Ile Asp Leu Ser Asn Tyr
   20 25 30
   Ala Ile Asn Trp Val Arg Gln Ala Pro Gly Lys Cys Leu Glu Trp Ile
   35 40 45
   Gly Ile Ile Trp Ala Ser Gly Thr Thr Phe Tyr Ala Thr Trp Ala Lys
   50 55 60
   Gly Arg Phe Thr Ile Ser Val Asp Lys Ala Lys Asn Ser Ala Tyr Leu
   65 70 75 80
   Gln Met Asn Ser Leu Arg Ala Glu Asp Thr Ala Val Tyr Tyr Cys Thr
   85 90 95
   Arg Thr Val Pro Gly Tyr Ser Thr Ala Pro Tyr Phe Asp Leu Trp Gly
   100 105 110
   Gln Gly Thr Leu Val Thr Val Ser Ser
   115 120
<210> 106
   <211> 251
   <212> **PRT**
   <213> **Artificial** Sequence
<220>
   <223> scFv 645CDR/497FW ds HL
<400> 106
   Glu Val Gln Leu Val Glu Ser Gly Gly Gly Leu Val Lys Pro Gly Gly
   1 5 10 15
   Ser Leu Arg Leu Ser Cys Ala Ala Ser Gly Ile Asp Leu Ser Asn Tyr
                   20                  25                  30
   Ala Ile Asn Trp Val Arg Gln Ala Pro Gly Lys Cys Leu Glu Trp Val
           35                  40                  45
   Gly Ile Ile Trp Ala Ser Gly Thr Thr Phe Tyr Ala Thr Trp Ala Lys
       50                  55                  60
   Gly Arg Phe Thr Ile Ser Arg Asp Asp Ser Lys Asn Thr Leu Tyr Leu
   65                  70                  75                  80
   Gln Met Asn Ser Leu Lys Thr Glu Asp Thr Ala Val Tyr Tyr Cys Thr
                   85                  90                  95
   Thr Thr Val Pro Gly Tyr Ser Thr Ala Pro Tyr Phe Asp Leu Trp Gly
               100                 105                 110
   Gln Gly Thr Leu Val Thr Val Ser Ser Gly Gly Gly Gly Ser Gly Gly
           115                 120                 125
   Gly Gly Ser Gly Gly Gly Gly Ser Gly Gly Gly Gly Ser Ala Ile Gln
       130                 135                 140
   Leu Thr Gln Ser Pro Ser Ser Leu Ser Ala Ser Val Gly Asp Arg Val
   145                 150                 155                 160
   Thr Ile Thr Cys Gln Ser Ser Pro Ser Val Trp Ser Asn Phe Leu Ser
                   165                 170                 175
   Trp Tyr Gln Gln Lys Pro Gly Lys Ala Pro Lys Leu Leu Ile Tyr Glu
               180                 185                 190
   Ala Ser Lys Leu Thr Ser Gly Val Pro Ser Arg Phe Ser Gly Ser Gly
           195                 200                 205
   Ser Gly Thr Asp Phe Thr Leu Thr Ile Ser Ser Leu Gln Pro Glu Asp
       210                 215                 220
   Phe Ala Thr Tyr Tyr Cys Gly Gly Gly Tyr Ser Ser Ile Ser Asp Thr
   225                 230                 235                 240
   Thr Phe Gly Cys Gly Thr Lys Val Glu Ile Lys
                   245                 250
<210> 107
   <211> 262
   <212> PRT
   <213> Artificial Sequence
<220>
   <223> Linker-scFv 645CDR/497FW ds HL
<400> 107
   Ser Gly Gly Gly Gly Ser Gly Gly Gly Gly Ser Glu Val Gln Leu Val
   1               5                   10                  15
   Glu Ser Gly Gly Gly Leu Val Lys Pro Gly Gly Ser Leu Arg Leu Ser
               20                  25                  30
   Cys Ala Ala Ser Gly Ile Asp Leu Ser Asn Tyr Ala Ile Asn Trp Val
           35                  40                  45
   Arg Gln Ala Pro Gly Lys Cys Leu Glu Trp Val Gly Ile Ile Trp Ala
       50                  55                  60
   Ser Gly Thr Thr Phe Tyr Ala Thr Trp Ala Lys Gly Arg Phe Thr Ile
   65                  70                  75                  80
   Ser Arg Asp Asp Ser Lys Asn Thr Leu Tyr Leu Gln Met Asn Ser Leu
                   85                  90                  95
   Lys Thr Glu Asp Thr Ala Val Tyr Tyr Cys Thr Thr Thr Val Pro Gly
               100                 105                 110
   Tyr Ser Thr Ala Pro Tyr Phe Asp Leu Trp Gly Gln Gly Thr Leu Val
           115                 120                 125
   Thr Val Ser Ser Gly Gly Gly Gly Ser Gly Gly Gly Gly Ser Gly Gly
       130                 135                 140
   Gly Gly Ser Gly Gly Gly Gly Ser Ala Ile Gln Leu Thr Gln Ser Pro
   145                 150                 155                 160
   Ser Ser Leu Ser Ala Ser Val Gly Asp Arg Val Thr Ile Thr Cys Gln
                   165                 170                 175
   Ser Ser Pro Ser Val Trp Ser Asn Phe Leu Ser Trp Tyr Gln Gln Lys
               180                 185                 190
   Pro Gly Lys Ala Pro Lys Leu Leu Ile Tyr Glu Ala Ser Lys Leu Thr
           195                 200                 205
   Ser Gly Val Pro Ser Arg Phe Ser Gly Ser Gly Ser Gly Thr Asp Phe
       210                 215                 220
   Thr Leu Thr Ile Ser Ser Leu Gln Pro Glu Asp Phe Ala Thr Tyr Tyr
   225 230 235 240
   Cys Gly Gly Gly Tyr Ser Ser Ile Ser Asp Thr Thr Phe Gly Cys Gly
   245 250 255
   Thr Lys Val Glu Ile Lys
   260
<210> 108
   <211> 98
   <212> PRT
   <213> Artificial Sequence
<220>
   <223> IgG1 wild type CH1
<220>
   <221> SITE
   <222> (1)..(1)
   <223> Ala is optional
<400> 108
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val
<210> 109
   <211> 98
   <212> PRT
   <213> Artificial Sequence
<220>
   <223> IgG4 wild type CH1
<220>
   <221> SITE
   <222> (1)..(1)
   <223> Ala is optional
<400> 109
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val
<210> 110
   <211> 106
   <212> PRT
   <213> Artificial Sequence
<220>
   <223> Ig wild type kappa constant light chain
<400> 110
   Thr Val Ala Ala Pro Ser Val Phe Ile Phe Pro Pro Ser Asp Glu Gln
   1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Cys
               100                 105
<210> 111
   <211> 251
   <212> **PRT**
   <213> **Artificial** Sequence
<220>
   <223> scFv 645CDR/2109 FW ds HL
<400> 111
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Ile Asp Leu Ser Asn Tyr
               20                  25                  30
   Ala Ile Asn Trp Val Arg Gln Ala Pro Gly Lys Cys Leu Glu Trp Ile
           35                  40                  45
   Gly Ile Ile Trp Ala Ser Gly Thr Thr Phe Tyr Ala Thr Trp Ala Lys
       50                  55                  60
   Gly Arg Phe Thr Ile Ser Val Asp Lys Ala Lys Asn Ser Ala Tyr Leu
   65                  70                  75                  80
   Gln Met Asn Ser Leu Arg Ala Glu Asp Thr Ala Val Tyr Tyr Cys Thr
                   85                  90                  95
   Arg Thr Val Pro Gly Tyr Ser Thr Ala Pro Tyr Phe Asp Leu Trp Gly
               100                 105                 110
   Gln Gly Thr Leu Val Thr Val Ser Ser Gly Gly Gly Gly Ser Gly Gly
           115                 120                 125
   Gly Gly Ser Gly Gly Gly Gly Ser Gly Gly Gly Gly Ser Asp Ile Gln
       130                 135                 140
   Met Thr Gln Ser Pro Ser Ser Leu Ser Ala Ser Val Gly Asp Arg Val
   145                 150                 155                 160
   Thr Ile Thr Cys Gln Ser Ser Pro Ser Val Trp Ser Asn Phe Leu Ser
                   165                 170                 175
   Trp Tyr Gln Gln Lys Pro Gly Lys Val Pro Lys Leu Leu Ile Tyr Glu
               180                 185                 190
   Ala Ser Lys Leu Thr Ser Gly Val Pro Ser Arg Phe Ser Gly Thr Gly
           195                 200                 205
   Ser Gly Thr Asp Tyr Thr Leu Thr Ile Ser Ser Leu Gln Pro Glu Asp
       210                 215                 220
   Val Ala Thr Tyr Phe Cys Gly Gly Gly Tyr Ser Ser Ile Ser Asp Thr
   225                 230                 235                 240
   Thr Phe Gly Cys Gly Thr Lys Leu Glu Ile Lys
                   245                 250
<210> 112
   <211> 262
   <212> PRT
   <213> Artificial Sequence
<220>
   <223> Linker-scFv 645CDR/2109 FW ds HL
<400> 112
   Ser Gly Gly Gly Gly Ser Gly Gly Gly Gly Ser Glu Val Gln Leu Val
   1               5                   10                  15
   Glu Ser Gly Gly Gly Leu Val Lys Pro Gly Gly Ser Leu Arg Leu Ser
               20                  25                  30
   Cys Ala Ala Ser Gly Ile Asp Leu Ser Asn Tyr Ala Ile Asn Trp Val
           35                  40                  45
   Arg Gln Ala Pro Gly Lys Cys Leu Glu Trp Ile Gly Ile Ile Trp Ala
       50                  55                  60
   Ser Gly Thr Thr Phe Tyr Ala Thr Trp Ala Lys Gly Arg Phe Thr Ile
   65                  70                  75                  80
   Ser Val Asp Lys Ala Lys Asn Ser Ala Tyr Leu Gln Met Asn Ser Leu
                   85                  90                  95
   Arg Ala Glu Asp Thr Ala Val Tyr Tyr Cys Thr Arg Thr Val Pro Gly
               100                 105                 110
   Tyr Ser Thr Ala Pro Tyr Phe Asp Leu Trp Gly Gln Gly Thr Leu Val
           115                 120                 125
   Thr Val Ser Ser Gly Gly Gly Gly Ser Gly Gly Gly Gly Ser Gly Gly
       130                 135                 140
   Gly Gly Ser Gly Gly Gly Gly Ser Asp Ile Gln Met Thr Gln Ser Pro
   145                 150                 155                 160
   Ser Ser Leu Ser Ala Ser Val Gly Asp Arg Val Thr Ile Thr Cys Gln
                   165                 170                 175
   Ser Ser Pro Ser Val Trp Ser Asn Phe Leu Ser Trp Tyr Gln Gln Lys
               180                 185                 190
   Pro Gly Lys Val Pro Lys Leu Leu Ile Tyr Glu Ala Ser Lys Leu Thr
           195                 200                 205
   Ser Gly Val Pro Ser Arg Phe Ser Gly Thr Gly Ser Gly Thr Asp Tyr
       210                 215                 220
   Thr Leu Thr Ile Ser Ser Leu Gln Pro Glu Asp Val Ala Thr Tyr Phe
   225                 230                 235                 240
   Cys Gly Gly Gly Tyr Ser Ser Ile Ser Asp Thr Thr Phe Gly Cys Gly
                   245                 250                 255
   Thr Lys Leu Glu Ile Lys
               260
<210> 113
   <211> 110
   <212> PRT
   <213> Artificial Sequence
<220>
   <223> 645gL4
<400> 113
   Asp Ile Gln Met Thr Gln Ser Pro Ser Ser Val Ser Ala Ser Val Gly
   1               5                   10                  15
   Asp Arg Val Thr Ile Thr Cys Gln Ser Ser Pro Ser Val Trp Ser Asn
               20                  25                  30
   Phe Leu Ser Trp Tyr Gln Gln Lys Pro Gly Lys Ala Pro Lys Leu Leu
           35                  40                  45
   Ile Tyr Glu Ala Ser Lys Leu Thr Ser Gly Val Pro Ser Arg Phe Ser
       50                  55                  60
   Gly Ser Gly Ser Gly Thr Asp Phe Thr Leu Thr Ile Ser Ser Leu Gln
   65 70 75 80
   Pro Glu Asp Phe Ala Thr Tyr Tyr Cys Gly Gly Gly Tyr Ser Ser Ile
   85 90 95
   Ser Asp Thr Thr Phe Gly Gly Gly Thr Lys Val Glu Ile Lys
   100 105 110
<210> 114
   <211> 121
   <212> **PRT**
   <213> **Artificial** Sequence
<220>
   <223> 645gH5
<400> **114**
   Glu Val Gln Leu Leu Glu Ser Gly Gly Gly Leu Val Gln Pro Gly Gly
   1 5 10 15
   Ser Leu Arg Leu Ser Cys Ala Val Ser Gly Ile Asp Leu Ser Asn Tyr
   20 25 30
   Ala Ile Asn Trp Val Arg Gln Ala Pro Gly Lys Gln Leu Glu Trp Ile
   35 40 45
   Gly Ile Ile Trp Ala Ser Gly Thr Thr Phe Tyr Ala Thr Trp Ala Lys
   50 55 60
   Gly Arg Phe Thr Ile Ser Arg Asp Asn Ser Lys Asn Thr Val Tyr Leu
   65 70 75 80
   Gln Met Asn Ser Leu Arg Ala Glu Asp Thr Ala Val Tyr Tyr Cys Ala
   85 90 95
   Arg Thr Val Pro Gly Tyr Ser Thr Ala Pro Tyr Phe Asp Leu Trp Gly
   100 105 110
   Gln Gly Thr Leu Val Thr Val Ser Ser
   115 120
<210> 115
   <211> 10
   <212> PRT
   <213> Artificial Sequence
<220>
   <223> CDRH1
<400> 115
   Gly Phe Thr Phe Ser Asp Tyr Asn Met Ala
   1               5                   10
<210> 116
   <211> 17
   <212> PRT
   <213> Artificial Sequence
<220>
   <223> CDRH2
<400> 116
   Thr Ile Thr Tyr Glu Gly Arg Asn Thr Tyr Tyr Arg Asp Ser Val Lys
   1               5                   10                  15
   Gly
<210> 117
   <211> 16
   <212> PRT
   <213> Artificial Sequence
<220>
   <223> CDRH3
<400> 117
   Pro Pro Gln Tyr Tyr Glu Gly Ser Ile Tyr Arg Leu Trp Phe Ala His
   1               5                   10                  15
<210> 118
   <211> 11
   <212> PRT
   <213> Artificial Sequence
<220>
   <223> CDRL1
<400> 118
   Arg Ala Asp Glu Ser Val Arg Thr Leu Met His
   1               5                   10
<210> 119
   <211> 7
   <212> PRT
   <213> Artificial Sequence
<220>
   <223> CDRL2
<400> 119
   Leu Val Ser Asn Ser Glu Ile
   1 5
<210> 120
   <211> 9
   <212> PRT
   <213> Artificial Sequence
<220>
   <223> CDRL3
<400> 120
   Gln Gln Thr Trp Ser Asp Pro Trp Thr
   1 5
<210> 121
   <211> 10
   <212> **PRT**
   <213> **Artificial** Sequence
<220>
   <223> CDRH1
<400> 121
   Gly Val Ile Phe Ser Asp Tyr Tyr Met Ala
   1 5 10
<210> 122
   <211> 17
   <212> PRT
   <213> Artificial Sequence
<220>
   <223> CDRH2
<400> 122
   Ser Ile Asn Phe Asn Ala Asp Ile Ser Tyr Tyr Arg Glu Ser Val Lys
   1 5 10 15
   Gly
<210> 123
   <211> 12
   <212> PRT
   <213> Artificial Sequence
<220>
   <223> CDRH3
<400> 123
   Asp Ala Asn Arg Gln Asn Tyr Asp Trp Phe Ala Tyr
   1 5 10
<210> 124
   <211> 15
   <212> **PRT**
   <213> **Artificial** Sequence
<220>
   <223> CDRL1
<400> **124**
   Lys Ala Ser Glu Ser Val Ser Ser Ser Met Tyr Ser Tyr Met His
   1               5                   10                  15
<210> 125
   <211> 7
   <212> PRT
   <213> Artificial Sequence
<220>
   <223> CDRL2
<400> 125
   Arg Ala Ser Asn Leu Glu Ser
   1               5
<210> 126
   <211> 9
   <212> PRT
   <213> Artificial Sequence
<220>
   <223> CDRL3
<400> 126
   Gln Gln Ser Trp Thr Ala Pro Arg Thr
   1               5
<210> 127
   <211> 10
   <212> **PRT**
   <213> **Artificial** Sequence
<220>
   <223> CDRH1
<400> 127
   Gly Tyr Thr Phe Thr Asp Asn Tyr Ile His
   1               5                   10
<210> 128
   <211> 17
   <212> PRT
   <213> Artificial Sequence
<220>
   <223> CDRH2
<400> 128
   Tyr Ile Asn Pro Ser Ser Ala Tyr Ala His Tyr Asn Glu Lys Phe Lys
   1               5                   10                  15
   Thr
<210> 129
   <211> 10
   <212> PRT
   <213> Artificial Sequence
<220>
   <223> CDRH3
<400> 129
   Arg Tyr Tyr Ser Ala Met Pro Phe Ala Tyr
   1               5                   10
<210> 130
   <211> 11
   <212> PRT
   <213> Artificial Sequence
<220>
   <223> CDRL1
<400> 130
   Arg Ala Ser Glu Asp Ile Tyr Ser Gly Leu Ala
   1               5                   10
<210> 131
   <211> 11
   <212> **PRT**
   <213> **Artificial** Sequence
<220>
   <223> CDRL1
<400> 131
   Arg Ala Ser Glu Asp Ile Tyr Asn Gly Leu Ala
   1               5                   10
<210> 132
   <211> 7
   <212> PRT
   <213> Artificial Sequence
<220>
   <223> CDRL2
<400> 132
   Asp Ser Ser Thr Leu His Thr
   1 5
<210> 133
   <211> 7
   <212> PRT
   <213> Artificial Sequence
<220>
   <223> CDRL2
<400> 133
   Asn Ser Asn Thr Leu His Thr
   1 5
<210> 134
   <211> 7
   <212> PRT
   <213> Artificial Sequence
<220>
   <223> CDRL2
<400> 134
   Asn Ser Ser Thr Leu His Thr
   1 5
<210> 135
   <211> 7
   <212> PRT
   <213> Artificial Sequence
<220>
   <223> CDRL2
<400> 135
   Asp Ser Asn Thr Leu His Thr
   1 5
<210> 136
   <211> 9
   <212> PRT
   <213> Artificial Sequence
<220>
   <223> CDRL3
<400> 136
   Gln Gln Asn Tyr Asp Phe Pro Leu Thr
   1 5
<210> 137
   <211> 23
   <212> PRT
   <213> Artificial Sequence
<220>
   <223> VL FR1
<220>
   <221> MISC_FEATURE
   <222> (1)..(1)
   <223> Xaa is A or D
<220>
   <221> MISC_FEATURE
   <222> (4)..(4)
   <223> Xaa is L or M
<400> 137
   Xaa Ile Gln Xaa Thr Gln Ser Pro Ser Ser Leu Ser Ala Ser Val Gly
   1               5                   10                  15
   Asp Arg Val Thr Ile Thr Cys
               20
<210> 138
   <211> 15
   <212> PRT
   <213> Artificial Sequence
<220>
   <223> VL FR2
<220>
   <221> MISC_FEATURE
   <222> (9)..(9)
   <223> Xaa is A or V
<400> 138
   Trp Tyr Gln Gln Lys Pro Gly Lys Xaa Pro Lys Leu Leu Ile Tyr
   1               5                   10                  15
<210> 139
   <211> 32
   <212> PRT
   <213> Artificial Sequence
<220>
   <223> VL FR3
<220>
   <221> MISC_FEATURE
   <222> (9)..(9)
   <223> Xaa is S or T
<220>
   <221> MISC_FEATURE
   <222> (15)..(15)
   <223> Xaa is F or Y
<220>
   <221> MISC_FEATURE
   <222> (27)..(27)
   <223> Xaa is F or V
<220>
   <221> MISC_FEATURE
   <222> (31)..(31)
   <223> Xaa is Y or F
<400> 139
   Gly Val Pro Ser Arg Phe Ser Gly Xaa Gly Ser Gly Thr Asp Xaa Thr
   1               5                   10                  15
   Leu Thr Ile Ser Ser Leu Gln Pro Glu Asp Xaa Ala Thr Tyr Xaa Cys
               20                  25                  30
<210> 140
   <211> 10
   <212> PRT
   <213> Artificial Sequence
<220>
   <223> VL FR4
<220>
   <221> MISC_FEATURE
   <222> (7)..(7)
   <223> Xaa is V or L
<400> 140
   Phe Gly Gln Gly Thr Lys Xaa Glu Ile Lys
   1               5                   10
<210> 141
   <211> 14
   <212> PRT
   <213> Artificial Sequence
<220>
   <223> VH FR2
<220>
   <221> MISC_FEATURE
   <222> (13)..(13)
   <223> Xaa is V or I
<400> 141
   Trp Val Arg Gln Ala Pro Gly Lys Gly Leu Glu Trp Xaa Gly
   1 5 10
<210> 142
   <211> 32
   <212> PRT
   <213> Artificial Sequence
<220>
   <223> VH FR3
<220>
   <221> MISC_FEATURE
   <222> (6)..(6)
   <223> Xaa is R or V
<220>
   <221> MISC_FEATURE
   <222> (8)..(8)
   <223> Xaa is D or K
<220>
   <221> MISC_FEATURE
   <222> (9)..(9)
   <223> Xaa is S or A
<220>
   <221> MISC_FEATURE
   <222> (12)..(12)
   <223> Xaa is T or S
<220>
   <221> MISC_FEATURE
   <222> (13)..(13)
   <223> Xaa is L or A
<220>
   <221> MISC_FEATURE
   <222> (21)..(21)
   <223> Xaa is K or R
<220>
   <221> MISC_FEATURE
   <222> (22)..(22)
   <223> Xaa is T or A
<220>
   <221> MISC_FEATURE
   <222> (32)..(32)
   <223> Xaa is T or R
<400> 142
   Arg Phe Thr Ile Ser Xaa Asp Xaa Xaa Lys Asn Xaa Xaa Tyr Leu Gln
   1 5 10 15
   Met Asn Ser Leu Xaa Xaa Glu Asp Thr Ala Val Tyr Tyr Cys Thr Xaa
   20 25 30
<210> 143
   <211> 10
   <212> **PRT**
   <213> **Artificial** Sequence
<220>
   <223> CDRH1
<400> 143
   Gly Ile Asp Leu Ser Asn Tyr Ala Ile Asn
   1 5 10
<210> 144
   <211> 16
   <212> PRT
   <213> Artificial Sequence
<220>
   <223> CDRH2
<400> 144
   Ile Ile Trp Ala Ser Gly Thr Thr Phe Tyr Ala Thr Trp Ala Lys Gly
   1 5 10 15
<210> 145
   <211> 13
   <212> PRT
   <213> Artificial Sequence
<220>
   <223> CDRH3
<400> 145
   Thr Val Pro Gly Tyr Ser Thr Ala Pro Tyr Phe Asp Leu
   1 5 10
<210> 146
   <211> 12
   <212> **PRT**
   <213> **Artificial** Sequence
<220>
   <223> CDRL1
<400> **146**
   Gln Ser Ser Pro Ser Val Trp Ser Asn Phe Leu Ser
   1 5 10
<210> 147
   <211> 7
   <212> PRT
   <213> Artificial Sequence
<220>
   <223> CDRL2
<400> 147
   Glu Ala Ser Lys Leu Thr Ser
   1 5
<210> 148
   <211> 11
   <212> PRT
   <213> Artificial Sequence
<220>
   <223> CDRL3
<400> 148
   Gly Gly Gly Tyr Ser Ser Ile Ser Asp Thr Thr
   1 5 10
<210> 149
   <211> 123
   <212> PRT
   <213> Artificial Sequence
<220>
   <223> Variable heavy chain sequence
<400> 149
   Gln Val Gln Leu Val Glu Ser Gly Gly Gly Leu Ile Lys Pro Gly Gly
   1               5                   10                  15
   Ser Leu Arg Ser Cys Ala Ala Ser Gly Asp Thr Val Ser Asp Glu Ser
               20                  25                  30
   Met Thr Trp Val Arg Gln Ala Pro Gly Lys Gly Leu Glu Trp Val Ser
           35                  40                  45
   Ala Ile Ser Ser Ser Gly Gly Ser Thr Tyr Tyr Ala Asp Ser Val Lys
       50                  55                  60
   Gly Arg Phe Thr Ile Ser Arg Asp Asn Ser Lys Asn Thr Val Tyr Leu
   65                  70                  75                  80
   Gln Met Asn Ser Leu Arg Ala Glu Asp Thr Ala Val Tyr Tyr Cys Val
                   85                  90                  95
   Thr Asp Asn Arg Ser Cys Gln Thr Ser Leu Cys Thr Ser Thr Thr Arg
               100                 105                 110
   Ser Trp Gly Gln Gly Thr Met Thr Val Ser Ser
           115                 120
<210> 150
   <211> 119
   <212> PRT
   <213> Artificial Sequence
<220>
   <223> Variable heavy chain sequence
<400> 150
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Asn Ala
               20                  25                  30
   Trp Met Thr Trp Val Thr Gln Ala Pro Gly Lys Gly Leu Glu Trp Val
           35                  40                  45
   Cys Arg Ile Lys Thr Lys Thr Asp Gly Gly Thr Thr Asp Tyr Ala Ala
       50                  55                  60
   Pro Val Lys Gly Phe Arg Thr Cys Ser Arg Asp Asp Ser Lys Asn Thr
   65                  70                  75                  80
   Leu Tyr Leu Gln Met Asn Ser Leu Lys Thr Glu Asp Thr Ala Val Tyr
                   85                  90                  95
   Tyr Cys Thr Thr Asp Arg Asp His Ser Ser Gly Ser Trp Gly Gln Gly
               100                 105                 110
   Thr Leu Val Thr Val Ser Ser
           115
<210> 151
   <211> 119
   <212> PRT
   <213> Artificial Sequence
<220>
   <223> Variable heavy chain sequence
<400> 151
   Asp Gln Val Leu Val Gln Ser Gly Gly Gly Leu Val Leu Pro Gly Gly
   1               5                   10                  15
   Ser Leu Arg Leu Ser Cys Thr Ala Ser Gly Phe Pro Phe Ser Asn Ala
               20                  25                  30
   Trp Met Ser Trp Val Arg Gln Ala Pro Gly Lys Gly Leu Glu Trp Val
           35                  40                  45
   Cys Arg Ile Thr Ser Lys Thr Asp Gly Gly Thr Thr Asp Tyr Val Ala
       50                  55                  60
   Pro Val Lys Gly Arg Phe Thr Cys Ser Arg Asp Asp Ser Lys Asn Thr
   65 70 75 80
   Leu Tyr Leu Gln Met Asn Ser Leu Lys Thr Glu Asp Thr Ala Val Tyr
   85 90 95
   Tyr Cys Thr Thr Asp Gln Ala Asn Ala Phe Asp Ile Trp Gly Gln Gly
   100 105 110
   Thr Met Val Thr Val Ser Ser
   115
<210> 152
   <211> 108
   <212> PRT
   <213> Artificial Sequence
<220>
   <223> Variable light chain sequence
<400> 152
   Ala Ile Gln Leu Thr Gln Pro Ser Ser Leu Ser Ala Ser Val Gly Asp
   1 5 10 15
   Arg Val Thr Ile Thr Cys Lys Ala Ser Glu Ser Val Ser Ser Met Tyr
   20 25 30
   Ser Tyr Met His Trp Tyr Gln Gln Lys Pro Gly Ala Pro Lys Leu Leu
   35 40 45
   Ile Tyr Arg Ala Ser Asn Leu Glu Ser Gly Val Pro Ser Arg Phe Ser
   50 55 60
   Gly Ser Gly Ser Thr Asp Phe Thr Leu Thr Ile Ser Ser Leu Gln Pro
   65 70 75 80
   Glu Asp Phe Ala Thr Tyr Tyr Cys Gln Gln Ser Trp Thr Ala Pro Arg
   85 90 95
   Thr Phe Gly Gln Gly Thr Lys Val Glu Ile Lys Arg
   100 105
<210> 153
   <211> 123
   <212> PRT
   <213> Artificial Sequence
<220>
   <223> Variable heavy chain sequence
<400> 153
   Glu Val Gln Leu Val Glu Ser Gly Gly Gly Leu Val Lys Pro Gly Gly
   1               5                   10                  15
   Ser Leu Arg Ser Cys Ala Ala Ser Gly Val Ile Phe Ser Asp Tyr Tyr
               20                  25                  30
   Met Ala Trp Val Arg Arg Arg Arg Gln Ala Pro Gly Lys Gly Leu Glu
           35                  40                  45
   Trp Val Ala Ser Ile Asn Phe Asn Ala Asp Ile Ser Tyr Tyr Arg Glu
       50                  55                  60
   Ser Val Lys Gly Arg Phe Thr Ile Ser Arg Asp Asp Ser Lys Asn Thr
   65                  70                  75                  80
   Leu Tyr Leu Gln Met Asn Ser Leu Lys Thr Glu Asp Thr Ala Val Tyr
                   85                  90                  95
   Tyr Cys Thr Thr Asp Ala Asn Arg Gln Asn Tyr Asp Trp Phe Ala Tyr
               100                 105                 110
   Trp Gly Gln Gly Thr Leu Val Thr Val Ser Ser
           115                 120